(19) Europäisches Patentamt
European Patent Office
Office européen des brevets

(11) EP 4 253 367 A1

(12) EUROPEAN PATENT APPLICATION
published in accordance with Art. 153(4) EPC

(43) Date of publication:
**04.10.2023 Bulletin 2023/40**

(21) Application number: **21897081.2**

(22) Date of filing: **25.11.2021**

(51) International Patent Classification (IPC):
***C07D 223/16*** (2006.01) ***C07D 405/02*** (2006.01)
***C07D 487/10*** (2006.01) ***A61K 31/55*** (2006.01)
***A61P 9/12*** (2006.01) ***A61P 9/04*** (2006.01)
***A61P 5/38*** (2006.01) ***A61P 25/24*** (2006.01)
***A61P 1/16*** (2006.01) ***A61P 15/06*** (2006.01)
***A61P 13/12*** (2006.01)

(52) Cooperative Patent Classification (CPC):
**A61K 31/55; A61P 1/16; A61P 5/38; A61P 9/04; A61P 9/12; A61P 13/12; A61P 15/06; A61P 25/24; C07D 223/16; C07D 405/02; C07D 487/10**

(86) International application number:
**PCT/CN2021/133158**

(87) International publication number:
**WO 2022/111581 (02.06.2022 Gazette 2022/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.11.2020 CN 202011353057**
**09.11.2021 CN 202111322711**

(71) Applicants:
- **Shanghai Jemincare Pharmaceuticals Co., Ltd.**
**Pudong New Area, Shanghai 201203 (CN)**
- **Jiangxi Jemincare Group Co., Ltd.**
**Nanchang, Jiangxi 330000 (CN)**

(72) Inventors:
- **LU, Hongfu**
**Shanghai 201203 (CN)**
- **LV, Yongcong**
**Shanghai 201203 (CN)**
- **YE, Yan**
**Shanghai 201203 (CN)**
- **PENG, Jianbiao**
**Shanghai 201203 (CN)**
- **GUO, Haibing**
**Shanghai 201203 (CN)**

(74) Representative: **Henderson, Helen Lee**
**Withers & Rogers LLP**
**2 London Bridge**
**London SE1 9RA (GB)**

(54) **NOVEL BENZAZEPINE SPIRO DERIVATIVE**

(57) A benzazepine spiro derivative as represented by formula (I) and a pharmaceutically acceptable salt thereof, and the use of a compound in the diagnosis, prevention and/or treatment of diseases related to vasopressin receptors.

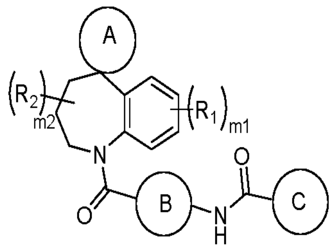

(I)

EP 4 253 367 A1

## Description

The present invention claims the following priorities:

application number: CN202011353057.1, filing date: November 26, 2020;
application number: CN202111322711.7, filing date: November 09, 2021.

### TECHNICAL FIELD

The present disclosure relates to novel benzazepine spiro derivatives and salts thereof. The present disclosure also relates to a medicament comprising a benzazepine derivative and a salt thereof as an active ingredient, useful for diagnosis, prevention, and/or treatment of a vasopressin receptor-related disease.

### BACKGROUND

Hormones play an important role in the regulation of environmental homeostasis in human body, and arginine vasopressin (AVP) is closely related to the regulation of water and sodium metabolism in human body. Metabolic disorders of arginine vasopressin (AVP) can cause hyponatremia and syndrome of inappropriate antidiuretic hormone secretion, congestive heart failure, liver cirrhosis, kidney disease, hypertension, and edema. Arginine vasopressin (AVP) receptor antagonists can inhibit the binding of AVP to the receptor, thereby playing a therapeutic role in the above-mentioned diseases. Arginine vasopressin V2 receptor antagonists, represented by tolvaptan, can increase free water excretion without affecting the metabolism of electrolytes, thus becoming ideal drugs for the treatment of the above-mentioned diseases. However, the marketed AVP V2 receptor antagonists, such as Tolvaptan, are metabolized by hepatic metabolic enzymes, which produce a large amount of metabolic products in the body and cause severe drug-induced hepatotoxicity. FDA gives a black box warning on the drug trade label, limiting its application. Therefore, it is important to develop novel V2 receptor antagonists with high efficacy and low side effects.

### CONTENT OF THE PRESENT INVENTION

It is an object of the present disclosure to provide a novel benzazepine spiro compound or a salt thereof having vasopressin V2 receptor antagonism, and favorable metabolic stability and/or metabolic products with reduced drug-induced hepatotoxicity properties, and a pharmaceutical use of the compound.

In one aspect of the present disclosure, the present disclosure provides a compound of Formula (X), an optical isomer thereof, and a pharmaceutically acceptable salt thereof,

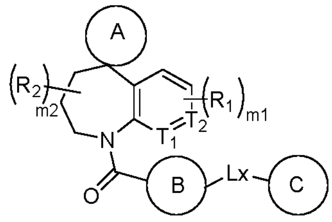

(X)

,

wherein

ring A is selected from heterocycloalkyl and cycloalkyl, and the heterocycloalkyl and cycloalkyl are optionally substituted with 1, 2, 3, or 4 $R_A$ groups;
ring B is selected from aryl, heteroaryl, heterocycloalkyl, and cycloalkyl, and the aryl, heteroaryl, heterocycloalkyl, or cycloalkyl is optionally substituted with 1, 2, or 3 $R_3$ groups;
ring C is selected from aryl, heteroaryl, heterocycloalkyl, and cycloalkyl, and the aryl, heteroaryl, heterocycloalkyl, or cycloalkyl is optionally substituted with 1, 2, or 3 $R_4$ groups;
$T_1$ and $T_2$ are each independently selected from N and CH;
$R_1$, $R_2$, $R_3$, and $R_4$ are each independently selected from H, F, Cl, Br, I, CN, OH, $NH_2$, alkyl, heteroalkyl, aryl, heteroaryl, heterocycloalkyl, and cycloalkyl, and the alkyl, heteroalkyl, aryl, heteroaryl, heterocycloalkyl, or cycloalkyl is optionally substituted with 1, 2, 3, or 4 R groups;
R and $R_A$ are each independently selected from H, F, Cl, Br, I, CN, OH, $NH_2$, alkyl, heteroalkyl, aryl, heteroaryl,

heterocycloalkyl, and cycloalkyl, and the alkyl, heteroalkyl, aryl, heteroaryl, heterocycloalkyl, or cycloalkyl is optionally substituted with 1, 2, 3, or 4 R' groups;

R' is selected from H, F, Cl, Br, I, CN, OH, $NH_2$, alkyl, and heteroalkyl;

m1 and m2 are each independently selected from 1, 2, 3, or 4;

Lx is selected from -NH (C=O)-, -alkyl-NH (C=O)-, -NH(C=O)-alkyl-, alkyl, alkenyl, and alkynyl, and the -alkyl-NH(C=O)-, -NH(C=O)-alkyl-, alkyl, alkenyl, or alkynyl is optionally substituted with 1, 2, 3, or 4 R groups; and

when ring A is selected from heterocycloalkyl, the compound of Formula (I) is not selected from

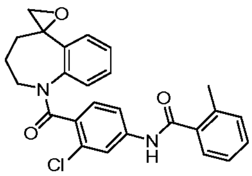, 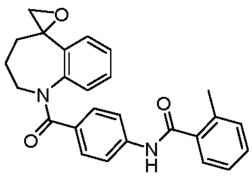, and 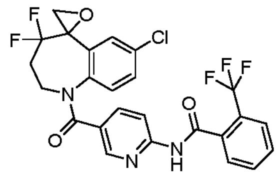,

the heterocycloalkyl or heteroaryl comprises 1, 2, 3, or 4 heteroatoms or heteroatomic groups independently selected from -O-, -NH-, -N=, -S-, -C(=O)-, -C(=O)O-, - S(=O)-, $-S(=O)_2-$, and N.

In another aspect of the present disclosure, the present disclosure provides a compound of Formula (I), an optical isomer thereof, and a pharmaceutically acceptable salt thereof,

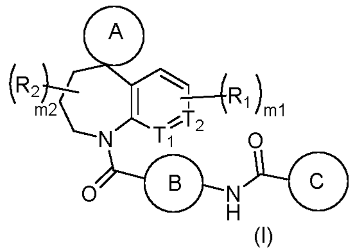

(I) ,

wherein

ring A is selected from 3- to 6-membered heterocycloalkyl and $C_{3-6}$ cycloalkyl, and the 3- to 6-membered heterocycloalkyl and $C_{3-6}$ cycloalkyl are optionally substituted with 1 or 2 $R_A$ groups;

ring B is selected from phenyl and 5- to 6-membered heteroaryl, and the phenyl or 5-to 6-membered heteroaryl is optionally substituted with 1, 2, or 3 $R_3$ groups;

ring C is selected from phenyl and 5- to 6-membered heteroaryl, and the phenyl or 5-to 6-membered heteroaryl is optionally substituted with 1, 2, or 3 $R_4$ groups;

$T_1$ and $T_2$ are each independently selected from N and CH;

$R_1$ is each independently selected from H, F, Cl, Br, I, CN, OH, $NH_2$, and $C_{1-6}$ alkyl, and the $C_{1-6}$ alkyl is optionally substituted with 1, 2, or 3 R groups;

$R_2$ is each independently selected from H, F, Cl, Br, I, CN, OH, $NH_2$, $C_{1-6}$ alkyl, and $C_{3-6}$ cycloalkyl, and the $C_{1-6}$ alkyl or $C_{3-6}$ cycloalkyl is optionally substituted with 1, 2, or 3 R groups;

$R_3$ is each independently selected from H, F, Cl, Br, I, CN, OH, $NH_2$, and $C_{1-6}$ alkyl, and the $C_{1-6}$ alkyl is optionally substituted with 1, 2, or 3 R groups;

$R_4$ is each independently selected from H, F, Cl, Br, I, CN, OH, $NH_2$, $C_{1-6}$ alkyl, $C_{1-6}$ alkoxy, $C_{1-6}$ alkylamino, $C_{3-6}$ cycloalkyl, phenyl, and 5- to 6-membered heteroaryl, and the $C_{1-6}$ alkyl, $C_{1-6}$ alkoxy, $C_{1-6}$ alkylamino, $C_{3-6}$ cycloalkyl, phenyl, or 5- to 6-membered heteroaryl is optionally substituted with 1, 2, or 3 R groups;

R and $R_A$ are each independently selected from H, F, Cl, Br, I, CN, OH, $NH_2$, $C_{1-6}$ alkyl, $C_{1-6}$ alkoxy, and $C_{1-6}$ alkylamino, and the $C_{1-6}$ alkyl, $C_{1-6}$ alkoxy, and $C_{1-6}$ alkylamino is optionally substituted with 1, 2, or 3 R' groups;

R' is selected from H, F, Cl, Br, I, CN, OH, $NH_2$, and $C_{1-6}$ alkyl;

m1 and m2 are each independently selected from 1, 2, or 3; and

when ring A is selected from 3- to 6-membered heterocycloalkyl, the compound of Formula (I) is not selected from

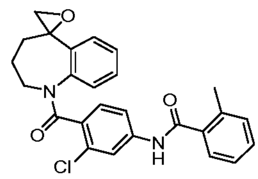, 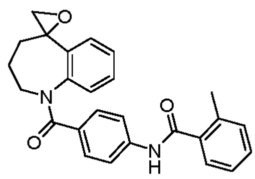,

and

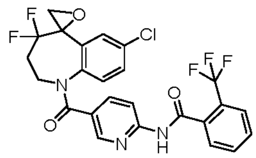,

the 3- to 6-membered heterocycloalkyl or 5- to 6-membered heteroaryl comprises1, 2, or 3 heteroatoms or heteroatom groups independently selected from -O-, -NH-, -N=, -S-, - C(=O)-, -C(=O)O-, -S(=O)-, $-S(=O)_2-$, and N.

In another aspect of the present disclosure, the present disclosure provides a compound of Formula (II), an optical isomer thereof, and a pharmaceutically acceptable salt thereof,

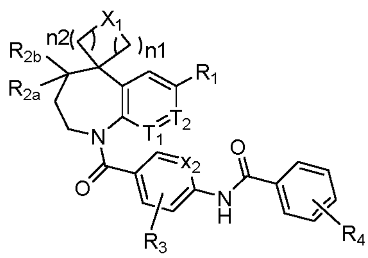

(II) ,

wherein

$X_1$ is selected from $C(R_A)_2$, NH, and O;
$X_2$ is selected from CH and N;
$T_1$ and $T_2$ are each independently selected from N and CH;
$R_1$ is each independently selected from H, F, Cl, Br, I, CN, OH, $NH_2$, and $C_{1-6}$ alkyl, and the $C_{1-6}$ alkyl is optionally substituted with 1, 2, or 3 R groups;
$R_{2a}$ and $R_{2b}$ are each independently selected from H, F, Cl, Br, I, CN, OH, $NH_2$, $C_{1-6}$ alkyl, and $C_{3-6}$ cycloalkyl, and the $C_{1-6}$ alkyl or $C_{3-6}$ cycloalkyl is optionally substituted with 1, 2, or 3 R groups;
$R_3$ is selected from H, F, Cl, Br, I, CN, OH, $NH_2$, and $C_{1-6}$ alkyl, and the $C_{1-6}$ alkyl is optionally substituted with 1, 2, or 3 R groups;
$R_4$ is each independently selected from H, F, Cl, Br, I, CN, OH, $NH_2$, $C_{1-6}$ alkyl, $C_{1-6}$ alkoxy, $C_{1-6}$ alkylamino, $C_{3-6}$ cycloalkyl, phenyl, and 5- to 6-membered heteroaryl, and the $C_{1-6}$ alkyl, $C_{1-6}$ alkoxy, $C_{1-6}$ alkylamino, $C_{3-6}$ cycloalkyl, phenyl, or 5- to 6-membered heteroaryl is optionally substituted with 1, 2, or 3 R groups;
R is selected from H, F, Cl, Br, I, CN, OH, $NH_2$, $C_{1-6}$ alkyl, $C_{1-6}$ alkoxy, and $C_{1-6}$ alkylamino, and the $C_{1-6}$ alkyl, $C_{1-6}$ alkoxy, and $C_{1-6}$ alkylamino are optionally substituted with 1, 2, or 3 R' groups;
R' is selected from H, F, Cl, Br, I, CN, OH, $NH_2$, and $C_{1-6}$ alkyl;
n1 is selected from 0, 1, or 2;
n2 is selected from 1, 2, or 3; and
when $X_1$ is selected from O, the compound of Formula (II) is not selected from

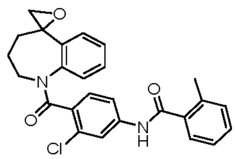, 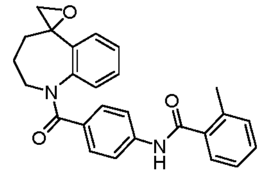, and 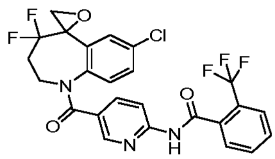.

In another aspect of the present disclosure, the present disclosure further provides a compound of Formula (III), an optical isomer thereof, and a pharmaceutically acceptable salt thereof,

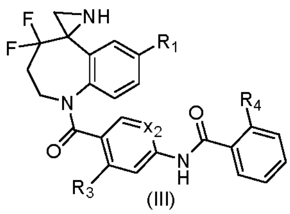

(III) ,

wherein

$R_1$ is each independently selected from H, F, Cl, Br, I, CN, OH, $NH_2$, and $C_{1-6}$ alkyl, and the $C_{1-6}$ alkyl is optionally substituted with 1, 2, or 3 R groups;

$R_3$ is selected from H, F, Cl, Br, I, CN, OH, $NH_2$, and $C_{1-6}$ alkyl, and the $C_{1-6}$ alkyl is optionally substituted with 1, 2, or 3 R groups;

$R_4$ is each independently selected from H, F, Cl, Br, I, CN, OH, $NH_2$, $C_{1-6}$ alkyl, $C_{1-6}$ alkoxy, $C_{1-6}$ alkylamino, $C_{3-6}$ cycloalkyl, phenyl, and 5- to 6-membered heteroaryl, and the $C_{1-6}$ alkyl, $C_{1-6}$ alkoxy, $C_{1-6}$ alkylamino, $C_{3-6}$ cycloalkyl, phenyl, or 5- to 6-membered heteroaryl is optionally substituted with 1, 2, or 3 R groups;

R is selected from H, F, Cl, Br, I, CN, OH, $NH_2$, $C_{1-6}$ alkyl, $C_{1-6}$ alkoxy, and $C_{1-6}$ alkylamino, and the $C_{1-6}$ alkyl, $C_{1-6}$ alkoxy, and $C_{1-6}$ alkylamino are optionally substituted with 1, 2, or 3 R' groups;

R' is selected from H, F, Cl, Br, I, CN, OH, $NH_2$, and $C_{1-6}$ alkyl;

$X_2$ is selected from CH and N.

In some embodiments of the present disclosure, R is selected from H, F, Cl, Br, I, CN, OH, $NH_2$, $CH_3$, $CF_3$,

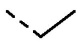, 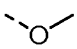, 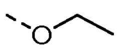, 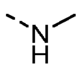, and 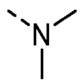,

and the other variables are as defined herein.

In some embodiments of the present disclosure, $R_A$ is selected from H, OH, and $NH_2$, and the other variables are as defined herein.

In some embodiments of the present disclosure, $R_4$ is selected from H, F, Cl, Br, I, CN, OH, $NH_2$, $CH_3$, $CF_3$,

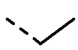, 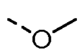, 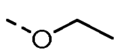, 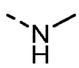, 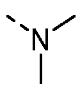,

cyclopropyl, cyclobutyl, cyclopentyl, phenyl, pyridyl, pyrimidinyl, thienyl, and thiazolyl, and the other variables are as defined herein.

In some embodiments of the present disclosure, ring A is selected from cyclopropyl, cyclobutyl, cyclopentyl, aziridinyl, oxiranyl, azetidinyl, oxetanyl, pyrrolidinyl, and tetrahydrofuranyl, and the cyclopropyl, cyclobutyl, cyclopentyl, aziridinyl, oxiranyl, azetidinyl, oxetanyl, pyrrolidinyl, or tetrahydrofuranyl is optionally substituted with 1 or 2 $R_A$ groups, and the other variables are as defined herein.

In some embodiments of the present disclosure, ring A is selected from

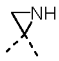, 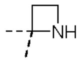,

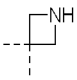, 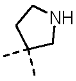, 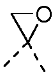, 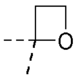, 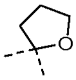, 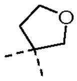, 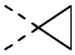, 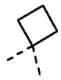, 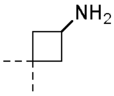, and 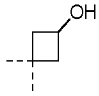,

and the other variables are as defined herein.

In some embodiments of the present disclosure, the structural moiety

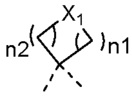

is selected from

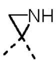, 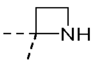, 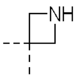, 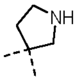, 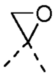, 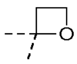, 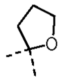, 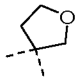, 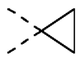, 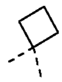,

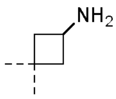, and 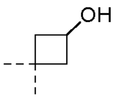,

and the other variables are as defined herein.

In some embodiments of the present disclosure, ring B is selected from phenyl and pyridyl, and the phenyl or pyridyl is optionally substituted with 1, 2, or 3 $R_3$ groups, and the other variables are as defined herein.

In some embodiments of the present disclosure, ring C is selected from

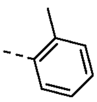,

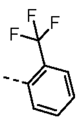, 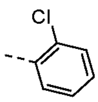, and 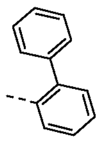,

and the other variables are as defined herein.

In another aspect of the present disclosure, the present disclosure further provides a compound of the following formula, an optical isomer thereof, and a pharmaceutically acceptable salt thereof, selected from

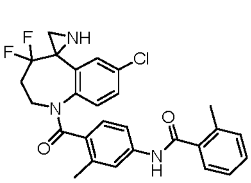 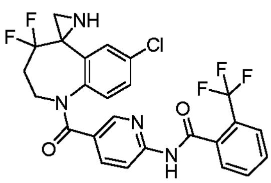 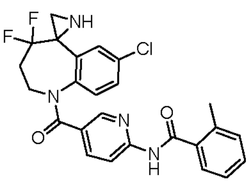 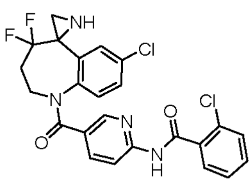

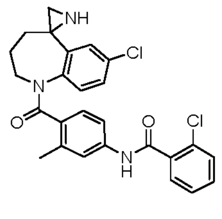
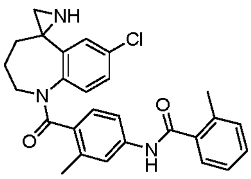
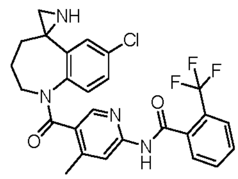
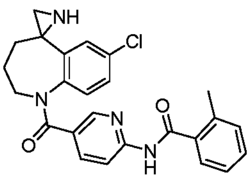
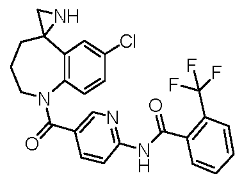

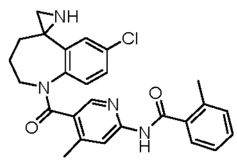
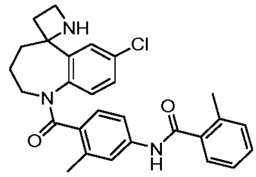
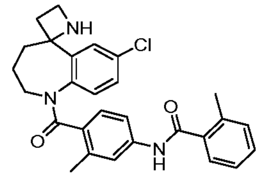
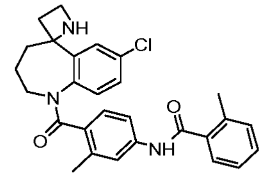

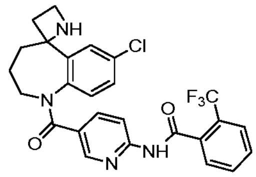
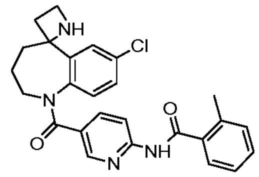
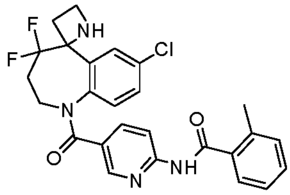
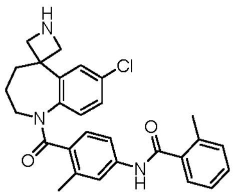

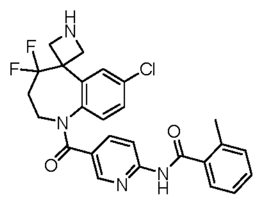
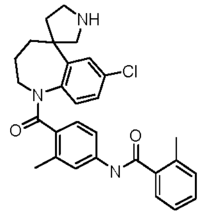
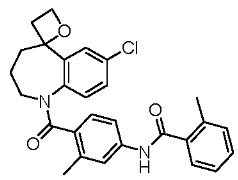
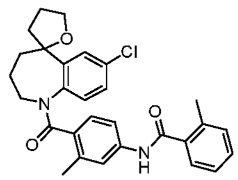
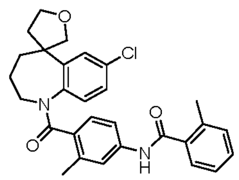

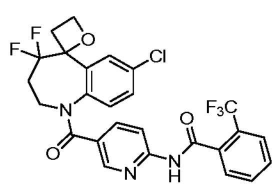
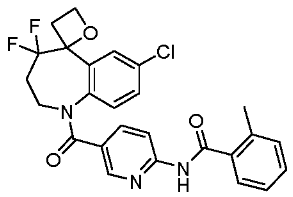
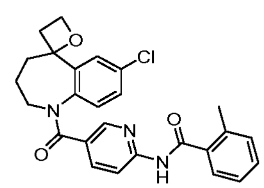
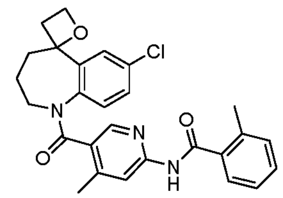

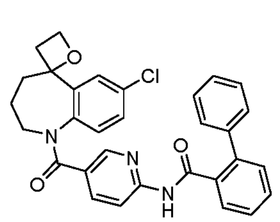
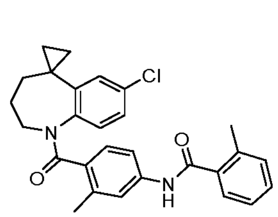
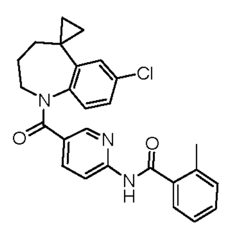
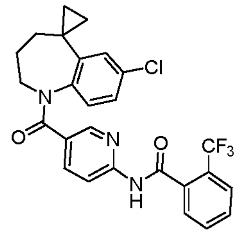

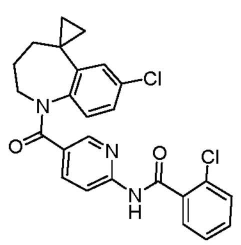
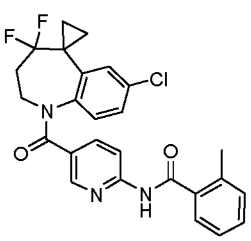
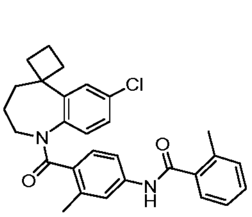
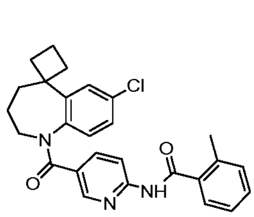
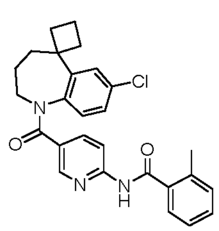

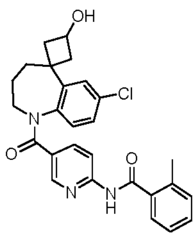
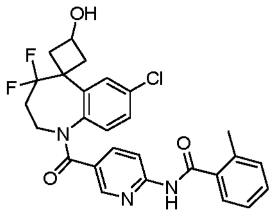
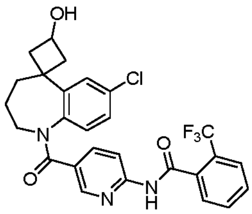
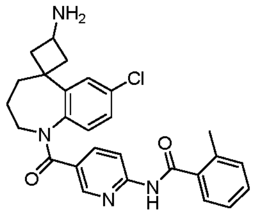

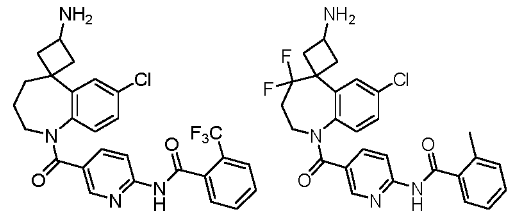
.

In yet another aspect of the present disclosure, the present disclosure also proposes a use of the aforementioned compound, the optical isomer thereof, and the pharmaceutically acceptable salt thereof in the manufacture of a medicament for prevention or treatment of a disease associated with arginine vasopressin V1a receptor, arginine vasopressin V1b receptor, arginine vasopressin V2 receptor, sympathetic nervous system or renin angiotensin aldosterone system.

In some embodiments of the disclosure, the disease associated with arginine vasopressin V1a receptor, arginine vasopressin V1b receptor, arginine vasopressin V2 receptor, sympathetic nervous system, or renin angiotensin aldosterone system comprises: hypertension, Raynaud's syndrome, dysmenorrhea, premature labor, corticotropin releasing hormone secretion disorder, adrenal hyperplasia, depression, chronic congestive heart failure, cirrhosis, syndrome of inappropriate antidiuretic hormone secretion, hyponatremia due to chronic heart failure/cirrhosis/inappropriate antidiuretic hormone secretion, or polycystic kidney disease.

**Definitions and Illustrations**

As used herein, the following terms and phrases are intended to have the following meanings, unless otherwise indicated. A particular term or phrase should not be considered indefinite or unclear without a specific definition and should be interpreted in a generic sense. When a trade name appears herein, it is intended to refer to its corresponding commercial product or its active ingredient.

As used herein, the phrase "at least one", when referring to a list of one or more elements, is understood to mean at least one element selected from any one or more of the elements in the list of elements, but does not necessarily include at least one of each of the elements specifically listed within the list of elements and does not preclude any combination of the elements in the list of elements. This definition also allows that elements may optionally be present other than the elements specifically identified within the list of elements to which the phrase "at least one" refers, whether related or unrelated to those specifically identified elements.

The term "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The term "pharmaceutically acceptable salts" refers to salts of the compounds of the present disclosure which are prepared from compounds of the present disclosure which have been found to have particular substituents with relatively nontoxic acids or bases. When compounds of the present disclosure contain relatively acidic functionalities, base addition salts can be obtained by contacting the neutral form of such compounds with a sufficient amount of the base, either in a pure solution or in a suitable inert solvent. Pharmaceutically acceptable base addition salts include sodium, potassium, calcium, ammonium, organic amine or magnesium salts, or similar salts. When compounds of the present disclosure contain relatively basic functionalities, acid addition salts can be obtained by contacting the neutral form of such compounds with a sufficient amount of the acid in a solution or in a suitable inert solvent. Examples of pharmaceutically acceptable acid addition salts include salts of inorganic acids such as hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid and the like; and salts of organic acids such as acetic acid, propionic acid, isobutyric acid, trifluoroacetic acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, methanesulfonic acid and the like; also included are salts of amino acids such as arginine and the like, and salts

of organic acids such as glucuronic acid and the like. Certain specific compounds of the present disclosure contain both basic and acidic functionalities that can be converted into either base or acid addition salts.

The pharmaceutically acceptable salts of the present disclosure can be synthesized from the parent compound which contains an acid or base group by conventional chemical methods. Generally, such salts are prepared by reacting the free acid or base forms of these compounds with a stoichiometric amount of the appropriate base or acid in water or in an organic solvent, or in a mixture of the two.

The compounds of the present disclosure may exist in particular geometric or stereoisomeric forms. The present disclosure contemplates all such compounds, including cis-and trans-isomers, (-)-and (+)-enantiomers, (R)-and (S)-enantiomers, diastereomers, (D)-isomers, (L)-isomers, and racemic and other mixtures thereof, such as enantiomerically or diastereomerically enriched mixtures, all of which are within the scope of the present disclosure. Additional asymmetric carbon atoms may be present in substituents such as alkyl groups. All such isomers, as well as mixtures thereof, are included within the scope of the claimed disclosure.

The compounds of the present disclosure may have particular. The terms "tautomer" or "tautomeric form" mean, unless otherwise stated, that at room temperature, different functional group isomers are in dynamic equilibrium and are capable of undergoing rapid interconversion. Tautomers are possible (e.g. in a solution), and a chemical equilibrium of the tautomers can be reached. For example, proton tautomers (also known as prototropic tautomers) include interconversions by migration of a proton, such as keto-enol isomerization and imine-enamine isomerization. Valence tautomers include interconversions by recombination of some of the bonding electrons. A specific example of keto-enol tautomerization is an interconversion between two tautomers of pentane-2,4-dione and 4-hydroxy-pene-3-ene-2-ketone.

The compounds of the present disclosure may contain unnatural proportions of atomic isotopes at one or more of the atoms that constitute such compounds. For example, the compounds may be labeled with radioactive isotopes, such as tritium ($^3H$), iodine -125 ($^{125}I$), or C-14 ($^{14}C$). For another example, a deuterated drug can be formed by replacing hydrogen with deuterium, wherein the bond formed between deuterium and carbon is stronger than that formed between ordinary hydrogen and carbon, and the deuterated drug has the advantages of reducing toxic side effects, increasing drug stability, enhancing therapeutic efficacy, and the biological half-life of the drug, compared with the undeuterated drug. All isotopic variations of the compounds of the present disclosure, whether radioactive or not, are intended to be encompassed within the scope of the present disclosure, "optional" or "optionally" means that the subsequently described event or circumstance may but need not occur and that the description includes instances where the event or circumstance occurs and instances in which it does not.

When a group bears a dashed line "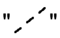" on a bond, as in

" 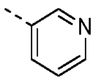 ",

the dashed line indicates the point of attachment of the group to the rest of the molecule. When a single bond bears "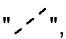", such as in "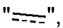", the dotted line represents a single bond or is absent, also meaning that "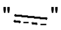" represents a single bond "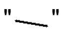" or a double bond "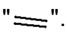".

The terms "substituted" or "substituted with" mean that any one or more hydrogen atoms on the specified atom are replaced with a substituent, and may include deuterium and hydrogen variants, provided that the valency of the specified atom is normal and that the substituted compound is stable. The term "optionally substituted" or "optionally substituted with" means that the substituents may or may not be substituted and, unless otherwise specified, where the nature and number of substituents may be any on a chemically feasible basis.

When any variable (e.g., R) occurs more than one time in a constituent or structure of a compound, its definition on each occurrence is independent. Thus, for example, if a group is substituted with 1, 2, or 3 R' groups, then the group may optionally be substituted with 1 or 2 or 3 R' groups, and R' in each case has independent options. Also, combinations of substituents and/or variables thereof are permissible only if such combinations result in stable compounds.

When one of the variables is selected from a single bond, it indicates that the two groups to which it is attached are directly connected, e.g. Li in

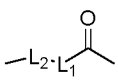

representing a single bond indicates that the structure is actually

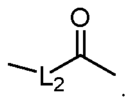

When the recited substituent does not indicate through which atom it is attached to the substituted group, such substituent may be bonded through any of its atoms. For example, a pyridyl group as a substituent may be attached to the substituted group through any of the carbon atoms on the pyridine ring.

When the linking group listed does not indicate the linking direction thereof, the linking direction is arbitrary. For example, when the linking group L in

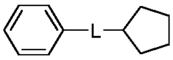

is - $CH_2O$-, the -CHzO- may link the phenyl group and the cyclopentyl group in the same direction as reading from left to right to constitute

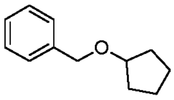

,

or may link the phenyl group and the cyclopentyl group in the opposite direction as reading from left to right to constitute

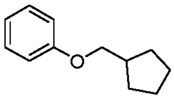

.

Combinations of such linking groups, substituents, and/or variations thereof are permissible only if such combinations result in stable compounds.

Unless otherwise specified, the number of ring atoms is typically defined as the number of ring members, e.g. "3- to 6-membered ring" means a "ring" having 3-6 atoms arranged therearound.

Unless otherwise specified, the term "alkyl" refers to a saturated aliphatic hydrocarbon group which is a straight or branched chain group containing from 1 to 20 carbon atoms, preferably an alkyl group containing from 1 to 12 carbon atoms, more preferably an alkyl group containing from 1 to 6 carbon atoms, more preferably an alkyl group containing from 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, and the like. More preferred are lower alkyl groups containing 1 to 6 carbon atoms, non-limiting examples including methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. Alkyl groups may be substituted or unsubstituted, and when substituted, substituents may be substituted at any available point of attachment, preferably one or more substituents independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio and oxo.

Unless otherwise specified, the term "$C_{1-6}$ alkyl" is used to denote a straight or branched chain saturated hydrocarbon group consisting of 1 to 6 carbon atoms. The $C_{1-6}$ alkyl includes $C_{1-5}$, $C_{1-4}$, $C_{1-3}$, $C_{1-2}$, $C_{2-6}$, $C_{2-4}$, $C_6$, and $C_5$ alkyl groups and the like; it can be monovalent (e.g. $CH_3$), divalent (-$CH_2$-), or polyvalent (e.g. hypo

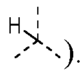

Examples of $C_{1-6}$ alkyl include, but are not limited to, $CH_3$,

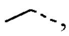, 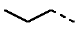, 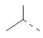, 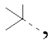, 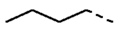, 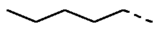,

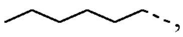, 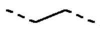, 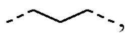, 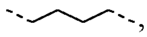, 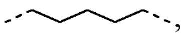, 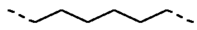,

and the like.

Unless otherwise specified, the term "$C_{1-4}$ alkyl" is used to denote a straight or branched chain saturated hydrocarbon group consisting of 1 to 4 carbon atoms. The $C_{1-4}$ alkyl includes $C_{1-2}$, $C_{1-3}$, $C_{3-4}$, and $C_{2-3}$ alkyl groups and the like; it can be monovalent (e.g. $CH_3$), divalent ($-CH_2-$), or polyvalent (e.g. hypo

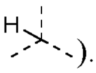

Examples of $C_{1-4}$ alkyl include, but are not limited to, $CH_3$,

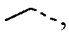, 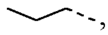, 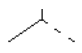, 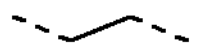, 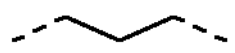,

and the like.

Unless otherwise specified, "$C_{2-3}$ alkenyl" is used to denote a straight or branched hydrocarbon group consisting of 2 to 3 carbon atoms containing at least one carbon-carbon double bond which may be located at any position of the group. The $C_{2-3}$ alkenyl includes $C_3$ and $C_2$ alkenyls; the $C_{2-3}$ alkenyl group may be monovalent, divalent, or polyvalent. Examples of $C_{2-3}$ alkenyl include, but are not limited to,

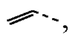, 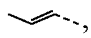, 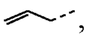, 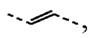, 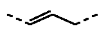

, and the like.

Unless otherwise specified, "$C_{2-3}$ alkynyl" is used to denote a straight or branched hydrocarbon group consisting of 2 to 3 carbon atoms containing at least one carbon-carbon triple bond which may be located at any position of the group. It may be monovalent, divalent or polyvalent. The $C_{2-3}$ alkynyl group includes $C_3$ and $C_2$ alkynyl groups. Examples of $C_{2-3}$ alkynyl groups include, but are not limited to,

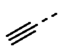, 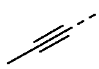, 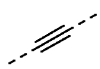, 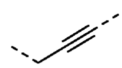,

and the like.

The term "heteroalkyl", by itself or in combination with another term, means, unless otherwise stated, a stable straight or branched chain alkyl group, or a combination thereof, consisting of a number of carbon atoms and at least one heteroatom or heteroatomic group. In some embodiments, the heteroatom is selected from B, O, N, and S, wherein the nitrogen and sulfur atoms are optionally oxidized and the nitrogen heteroatom is optionally quaternized. In other embodiments, the heteroatom group is selected from -C(=O)O-, -C(=O)-, -C(=S)-, - S(=O), $-S(=O)_2-$, -C(=O)N(H)-, -N(H)-, -C(=NH)-, $-S(=O)_2N(H)-$, and -S(=O)N(H)-. In some embodiments, the heteroalkyl is $C_{1-6}$ heteroalkyl; in other embodiments, the heteroalkyl is $C_{1-3}$ heteroalkyl. The heteroatom or heteroatomic group may be placed at any interior position of the heteroalkyl group, including the point of attachment of the alkyl group to the rest of the molecule, but the term "alkoxy" is art-recognized and refers to those alkyl groups attached to the rest of the molecule through an oxygen atom. Examples of heteroalkyl groups include, but are not limited to, $-OCH_3$, $-OCH_2CH_3$, $-OCH_2CH_2CH_3$, $-OCH_2(CH_3)_2$, $-CH_2-CH_2-O-CH_3$, $-NHCH_3$, $-N(CH_3)_2$, $-NHCH_2CH_3$, $-N(CH_3)(CH_2CH_3)$, $-CH_2-CH_2-NH-CH_3$, $-CH_2-CH_2-N(CH_3)-CH_3$, $-SCH_3$, $-SCH_2CH_3$, $-SCH_2CH_2CH_3$, $-SCH_2(CH_3)_2$, $-CH_2-S-CH_2-CH_3$, $- CH_2-CH_2$, $-S(=O)-CH_3$, $-CH_2-CH_2-S(=O)_2-CH_3$,

and up to two heteroatoms may be consecutive, such as $-CH_2-NH-OCH_3$.

Unless otherwise specified, the term "$C_{1-6}$ alkoxy" denotes those alkyl groups containing from 1 to 6 carbon atoms which are attached to the rest of the molecule through one oxygen atom. The $C_{1-6}$ alkoxy group includes $C_{1-4}$, $C_{1-3}$, $C_{1-2}$, $C_{2-6}$, $C_{2-4}$, $C_6$, $C_5$, $C_4$, and $C_3$ alkoxy groups and the like. Examples of $C_{1-6}$ alkoxy include, but are not limited to, methoxy, ethoxy, propoxy (including n-propoxy and isopropoxy), butoxy (including n-butoxy, isobutoxy, s-butoxy, and t-butoxy), pentoxy (including n-pentoxy, isopentoxy, and neopentoxy), hexoxy and the like.

Unless otherwise specified, the term "$C_{1-3}$ alkoxy" denotes those alkyl groups containing from 1 to 3 carbon atoms which are attached to the rest of the molecule through one oxygen atom. The $C_{1-3}$ alkoxy group includes $C_{1-3}$, $C_{1-2}$, $C_{2-3}$, $C_1$, $C_2$, and $C_3$ alkoxy groups and the like. Examples of $C_{1-3}$ alkoxy include, but are not limited to, methoxy, ethoxy, propoxy (including n-propoxy and isopropoxy), and the like.

Unless otherwise specified, the term "$C_{1-6}$ alkylamino" denotes those alkyl groups containing from 1 to 6 carbon atoms which are attached to the rest of the molecule through an amino group. The $C_{1-6}$ alkylamino group includes $C_{1-4}$, $C_{1-3}$, $C_{1-2}$, $C_{2-6}$, $C_{2-4}$, $C_6$, $C_5$, $C_4$, $C_3$, and $C_2$ alkylamino groups and the like. Examples of $C_{1-6}$ alkylamino include, but are not limited to, $-NHCH_3$, $-N(CH_3)_2$, $-NHCH_2CH_3$, $-N(CH_3)CH_2CH_3$, $-N(CH_2CH_3)(CH_2CH_3)$, - $NHCH_2CH_2CH_3$, $-NHCH_2(CH_3)_2$, $-NHCH_2CH_2CH_2CH_3$, and the like.

Unless otherwise specified, the term "$C_{1-3}$ alkylamino" denotes those alkyl groups containing from 1 to 3 carbon atoms which are attached to the rest of the molecule through an amino group. The $C_{1-3}$ alkylamino group includes $C_{1-3}$, $C_{1-2}$, $C_{2-3}$, $C_1$, $C_2$, and $C_3$ alkylamino groups and the like. Examples of $C_{1-3}$ alkylamino include, but are not limited to, $-NHCH_3$, - $N(CH_3)_2$, $-NHCH_2CH_3$, $-N(CH_3)CH_2CH_3$, $-NHCH_2CH_2CH_3$, $-NHCH_2(CH_3)_2$, and the like.

Unless otherwise specified, the term "$C_{1-6}$ alkylthio" denotes those alkyl groups containing from 1 to 6 carbon atoms which are attached to the rest of the molecule through a sulfur atom. The $C_{1-6}$ alkylthio group includes $C_{1-4}$, $C_{1-3}$, $C_{1-2}$, $C_{2-6}$, $C_{2-4}$, $C_6$, $C_5$, $C_4$, $C_3$, and $C_2$ alkylthio groups and the like. Examples of $C_{1-6}$ alkylthio include, but are not limited to, - $SCH_3$, $-SCH_2CH_3$, $-SCH_2CH_2CH_3$, $-SCH_2(CH_3)_2$, and the like.

Unless otherwise specified, the term "$C_{1-3}$ alkylthio" denotes those alkyl groups containing from 1 to 3 carbon atoms which are attached to the rest of the molecule through a sulfur atom. The $C_{1-3}$alkylthio group includes $C_{1-3}$, $C_{1-2}$, $C_{2-3}$, $C_1$, $C_2$, and $C_3$ alkylthio groups and the like. Examples of $C_{1-3}$ alkylthio include, but are not limited to, $-SCH_3$, $-SCH_2CH_3$, - $SCH_2CH_2CH_3$, $-SCH_2(CH_3)_2$, and the like.

Unless otherwise specified, the term "cycloalkyl" refers to a saturated or partially unsaturated monocyclic or polycyclic cyclic hydrocarbon substituent, the cycloalkyl ring containing from 3 to 20 carbon atoms, preferably from 3 to 12 carbon atoms (either at a particular point or an interval of optional two points, e.g. 3, 4, 5, 6 ring atoms, 4 to 11 ring atoms, 6 to 12 ring atoms, etc.), more preferably from 3 to 8 carbon atoms, and most preferably from 3 to 6 (e.g. 3, 4, 5, or 6) carbon atoms. Non-limiting examples of monocyclic cycloalkyl groups include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like, preferably cycloalkyl; polycyclic cycloalkyl groups include spiro, fused and bridged cycloalkyl groups.

The term "spirocycloalkyl" refers to a 5- to 20-membered polycyclic group in which one carbon atom (referred to as a spiro atom) is shared between monocyclic rings, which may contain one or more double bonds, but none of the rings have a completely conjugated π-electron system. It is preferably 6- to 14-membered, more preferably 7- to 10-membered. Spirocycloalkyl is classified as mono-, di-or poly-spirocycloalkyl depending on the number of spiro atoms shared between the rings, preferably mono-and di-spirocycloalkyl. More preferred are 4-membered/4-membered, 4-membered/5-membered, 4-membered/6-membered, 5-membered/5-membered or 5-membered/6-membered mono-spirocycloalkyl. Non-limiting examples of spirocycloalkyl groups include:

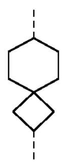 , 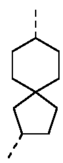 , 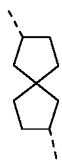 , 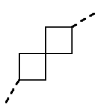 , and 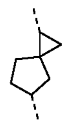 ,

etc.

The term "fused cycloalkyl" refers to a 5- to 20-membered all-carbon polycyclic group in which each ring in the system shares an adjacent pair of carbon atoms with the other rings in the system, wherein one or more of the rings may contain one or more double bonds, but none of the rings has a completely conjugated π-electron system. It is preferably 6- to 14-membered, more preferably 7- to 10-membered. Bicyclic, tricyclic, tetracyclic, or polycyclic fused cycloalkyl groups may be classified according to the number of constituent rings, preferably bicyclic or tricyclic, more preferably 5-membered/5-membered or 5-membered/6-membered bicycloalkyl groups. Non-limiting examples of fused cycloalkyl groups include:

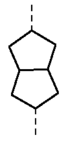, 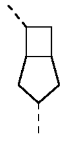, 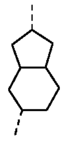, 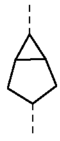, 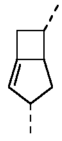, 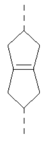, and 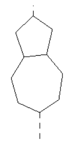,

etc.

The term "bridged cycloalkyl" refers to a 5- to 20-membered all-carbon polycyclic group in which any two rings share two carbon atoms that are not directly connected, which may contain one or more double bonds, but none of the rings have a completely conjugated π-electron system. It is preferably 5- to 14-membered, more preferably 7- to 10-membered. It can be divided into bicyclic, tricyclic, tetracyclic or polycyclic bridged cycloalkyl groups according to the number of constituent rings, preferably bicyclic, tricyclic or tetracyclic, more preferably bicyclic or tricyclic. Non-limiting examples of bridged cycloalkyl groups include:

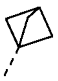, 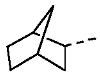, 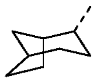, 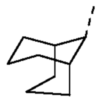, 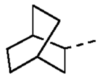, 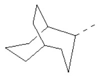, 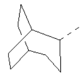, 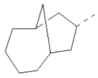, and 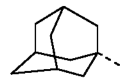,

etc.

Such cycloalkyl groups include the aforementioned cycloalkyl groups (e.g. monocyclic, fused, spiro, and bridged cycloalkyl groups) fused to an aryl, heteroaryl, or heterocycloalkyl ring wherein the ring attached to the parent structure is a cycloalkyl group, non-limiting examples include indanyl, tetrahydronaphthyl, benzocycloheptanyl, and the like; preferred are phenylcyclopentyl, tetrahydronaphthyl.

Cycloalkyl groups may be optionally substituted or unsubstituted. When substituted, the substituents are preferably one or more substituents independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio and oxo.

The term "heterocyclyl" refers to a saturated or partially unsaturated monocyclic or polycyclic cyclic hydrocarbon substituent containing from 3 to 20 ring atoms in which one or more ring atoms is a heteroatom selected from nitrogen, oxygen, or $S(O)_m$ (where m is an integer from 0 to 2), but excluding the ring portion of -O-O-, -O-S-, or-S-S-, and the remaining ring atoms are carbon. Preferably it contains from 3 to 12 ring atoms (which may be a specific point or may be an interval of optionally two points, for example, 3, 4, 5, 6 ring atoms, from 4 to 11 ring atoms, from 6 to 12 ring atoms, etc.), of which from 1 to 4 are heteroatoms; preferably it contains from 3 to 8 ring atoms, of which from 1 to 3 are heteroatoms; more preferably it contains from 3 to 6 ring atoms, of which from 1 to 3 are heteroatoms. Non-limiting examples of monocyclic heterocyclyl groups include azetidinyl, pyrrolidinyl, imidazolidinyl, tetrahydrofuranyl, tetrahydropyranyl, tetrahydrothiophenyl, dihydroimidazolyl, dihydrofuranyl, dihydropyrazolyl, dihydropyrrolyl, piperidinyl, piperazinyl, morpholinyl, thiomorpholinyl, homopiperazinyl, and the like, preferably tetrahydropyranyl, piperidinyl, pyrrolidinyl. Polycyclic heterocyclyl groups include spiro, fused and bridged heterocyclyl groups.

The term "spiroheterocyclyl" refers to a 5- to 20-membered polycyclic heterocyclic group in which one atom (referred to as a spiro atom) is shared between monocyclic rings, wherein one or more of the ring atoms is a heteroatom selected from nitrogen, oxygen, or $S(O)_m$ (where m is an integer from 0 to 2), and the remaining ring atoms are carbon. It may contain one or more double bonds, but no ring has a fully conjugated π-electron system. It is preferably 6- to 14-membered, more preferably 7- to 11-membered. Spiroheterocyclyl groups are classified as mono-, bi-or poly-spiroheterocyclyl, preferably mono-and bi-spiroheterocyclyl, depending on the number of spiro atoms shared between the rings. More preferred are 4-membered/4-membered, 4-membered/5-membered, 4-membered/6-membered, 5-membered/5-membered or 5-membered/6-membered mono-spiro heterocyclyl groups. Non-limiting examples of spiroheterocyclyl groups include:

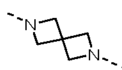, 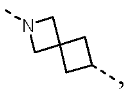, 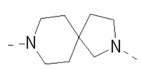,

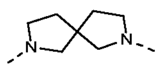, 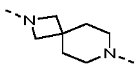, 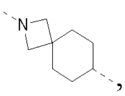, and 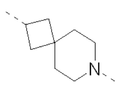,

etc.

The term "fused heterocyclyl" refers to a 5- to 20-membered polycyclic heterocyclic group in which each ring in the system shares an adjacent pair of atoms with other rings in the system. One or more of the rings may contain one or more double bonds, but none of the rings has a completely conjugated $\pi$-electron system, wherein one or more of the ring atoms is a heteroatom selected from nitrogen, oxygen, or $S(O)_m$ (where m is an integer from 0 to 2), and the remaining ring atoms are carbon. It is preferably 6- to 14-membered, more preferably 7-to 11-membered. Bicyclic, tricyclic, tetracyclic, or polycyclic fused heterocyclyl groups can be classified according to the number of constituent rings, preferably bicyclic or tricyclic, more preferably 5-membered/5-membered or 5-membered/6-membered bicyclic fused heterocyclyl groups. Non-limiting examples of fused heterocyclyl groups include:

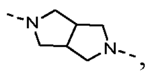, 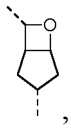,

and

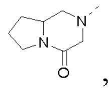,

etc.

The term "bridged heterocyclyl" refers to a 5- to 14-membered polycyclic heterocyclic group in which any two rings share two atoms that are not directly joined, which may contain one or more double bonds, but none of the rings have a completely conjugated $\pi$-electron system, wherein one or more of the ring atoms is a heteroatom selected from nitrogen, oxygen, or $S(O)_m$ (wherein m is an integer from 0 to 2), and the remaining ring atoms are carbon. It is preferably 6- to 14-membered, more preferably 7- to 11-membered. They can be divided into bicyclic, tricyclic, tetracyclic, or polycyclic bridged heterocyclic groups according to the number of constituent rings, preferably a bicyclic, tricyclic, or tetracyclic group, more preferably a bicyclic or tricyclic group. Non-limiting examples of bridged heterocyclyl groups include:

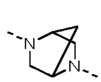, 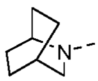, 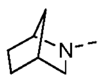, and 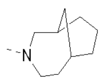,

etc.

The heterocyclic groups include the heterocyclic groups described above (e.g. monocyclic, fused, spiro, and bridged heterocyclic groups) fused to an aryl, heteroaryl, or cycloalkyl ring, wherein the ring attached to the parent structure is a heterocyclic group, non-limiting examples of which include:

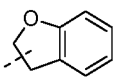, 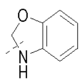, 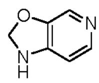, and 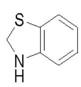,

etc.

Heterocyclyl groups may be optionally substituted or unsubstituted. When substituted, the substituents are preferably one or more substituents independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio and oxo.

The term "aryl" refers to a 6- to 20-membered, preferably 6- to 10-membered, more preferably 6-membered, all-carbon monocyclic or fused polycyclic (i.e. rings which share an adjacent pair of carbon atoms) group having a conjugated $\pi$-electron system, such as phenyl and naphthyl. Such aryl groups include the aforementioned aryl fused to a heteroaryl, heterocyclyl or cycloalkyl ring wherein the ring attached to the parent structure is an aryl ring, non-limiting examples of which include:

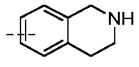, 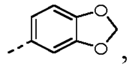, 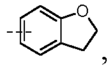, 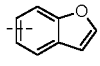, 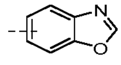,

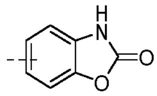, and 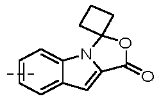.

The aryl group may be substituted or unsubstituted. When substituted, the substituents are preferably one or more substituents independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, and heterocycloalkylthio.

The term "heteroaryl" refers to a heteroaromatic system comprising 1 to 4 heteroatoms and 5 to 20 ring atoms, wherein the heteroatoms are selected from oxygen, sulfur, and nitrogen. Heteroaryl is preferably 5- to 10-membered, containing 1 to 3 heteroatoms; more preferably 5- or 6-membered, containing 1 to 3 heteroatoms; non-limiting examples are pyrazolyl, imidazolyl, furanyl, thienyl, thiazolyl, oxazolyl, pyrrolyl, triazolyl, tetrazolyl, pyridinyl, pyrimidinyl, thiadiazole, pyrazinyl, and the like. The heteroaryl ring can be fused to an aryl, heterocyclyl, or cycloalkyl ring, where the ring attached to the parent structure is a heteroaryl ring, non-limiting examples of which include:

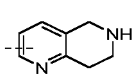, 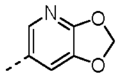, 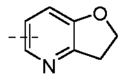, 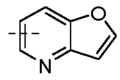,

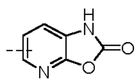, 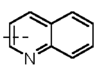, and 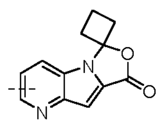.

The heteroaryl group may be optionally substituted or unsubstituted. When substituted, the substituents are preferably one or more substituents independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, and heterocycloalkylthio.

Unless otherwise specified, "$C_{3-6}$ cycloalkyl" denotes a saturated cyclic hydrocarbon group consisting of 3 to 6 carbon atoms, which is a mono- and bicyclic-ring system, including $C_{3-5}$, $C_{4-5}$, and $C_{5-6}$ cycloalkyl groups and the like; it may be monovalent, divalent, or polyvalent. Examples of $C_{3-6}$ cycloalkyl include, but are not limited to, cyclopropyl, cyclobutyl, cyclopentyl, cyclohexyl, and the like.

Unless otherwise specified, the term "3- to 6-membered heterocycloalkyl" by itself or in combination with other terms means, unless otherwise stated, a saturated cyclic group consisting of 3 to 6 ring atoms, 1, 2, 3, or 4 ring atoms of which are heteroatoms independently selected from O, S, and N, and the remaining are carbon atoms, wherein the nitrogen atom is optionally quaternized and the nitrogen and sulfur heteroatoms may optionally be oxidized (i.e., NO and $S(O)_p$, and p is 1 or 2). It includes monocyclic and bicyclic ring systems, wherein bicyclic ring systems include spirocyclic rings and bridged rings. In addition, with respect to the "3- to 6-membered heterocycloalkyl", a heteroatom can occupy the position of attachment of the heterocycloalkyl to the rest of the molecule. The 3- to 6-membered heterocycloalkyl includes 4- to 6-membered, 5- to 6-membered, 4-, 5- and 6-membered heterocycloalkyl, and the like. Examples of 3- to 6-membered heterocycloalkyl include, but are not limited to, azetidinyl, oxetanyl, thietanyl, pyrrolidinyl, pyrazolidinyl, imidazolidinyl, tetrahydrothiophenyl (including tetrahydrothiophen-2-yl and tetrahydrothiophen-3-yl, etc.), tetrahydrofuranyl (including tetrahydrofuran-2-yl, etc.), tetrahydropyranyl, piperidinyl (including 1-piperidinyl, 2-piperidinyl, and 3-piperidinyl, etc.), piperazinyl (including 1-piperazinyl and 2-piperazinyl, etc.), morpholinyl (including 3-morpholinyl and 4-morpholinyl, etc.), dioxanyl, dithianyl, isoxazolidinyl, isothiazolidinyl, 1,2-oxazinyl, 1,2-thiazinyl, hexahydropyridazinyl, homopiperazinyl, or homopiperidinyl, and the like.

Unless otherwise specified, the terms "5- to 6-membered heteroaromatic ring" and "5-to 6-membered heteroaryl" are used interchangeably herein, and the term "5- to 6-membered heteroaryl" denotes a monocyclic group consisting of 5 to 6 ring atoms having a conjugated $\pi$ electron system, and 1, 2, 3, or 4 of the ring atoms are heteroatoms independently selected from O, S, and N, and the remainder is carbon atoms. The nitrogen atoms thereof are optionally quaternized, and the nitrogen and sulfur heteroatoms may optionally be oxidized (i.e., NO and $S(O)_p$, and p is 1 or 2). The 5- to 6-membered heteroaryl may be attached to the remainder of the molecule through a heteroatom or a carbon atom. The 5- to 6-membered heteroaryl group includes 5- and 6-membered heteroaryl groups. Examples of the 5- to 6-membered heteroaryl include, but are not limited to, pyrrolyl (including N-pyrrolyl, 2-pyrrolyl, and 3-pyrrolyl, etc.), pyrazolyl (including 2-pyrazolyl and 3-pyrazolyl, etc.), imidazolyl (including N-imidazolyl, 2-imidazolyl, 4-imidazolyl, and 5-imidazolyl, etc.), oxazolyl (including 2-oxazolyl, 4-oxazolyl, and 5-oxazolyl, etc.), triazolyl (1H-1,2,3-triazolyl, 2H-1,2,3-triazolyl, 1H-1,2,4-triazolyl, and 4H-1,2,4-triazolyl, etc.), tetrazolyl, isoxazolyl (3-isoxazolyl, 4-isoxazolyl, and 5-isoxazolyl, etc.), thiazolyl (including 2-thiazolyl, 4-thiazolyl, and 5-thiazolyl, etc.), furanyl (including 2-furanyl and 3-furanyl, etc.), thienyl (including 2-thienyl and 3-thienyl, etc.), pyridinyl (including 2-pyridinyl, 3-pyridinyl, and 4-pyridinyl, etc.), pyrazinyl, or pyrimidinyl (including 2-pyrimidinyl and 4-pyrimidinyl, etc.).

Unless otherwise specified, $C_{n-n+m}$ or $C_n$-$C_{n+m}$ includes any one particular instance of n to n+m carbons, e.g. $C_{1-12}$ includes $C_1$, $C_2$, $C_3$, $C_4$, $C_5$, $C_6$, $C_7$, $C_8$, $C_9$, $C_{10}$, $C_{11}$, and $C_{12}$, as well as any range of n to n+m, e.g. $C_{1-12}$ includes $C_{1-3}$, $C_{1-6}$, $C_{1-9}$, $C_{3-6}$, $C_{3-9}$, $C_{3-12}$, $C_{6-9}$, $C_{6-12}$, and $C_{9-12}$, etc.; similarly, n-membered to n+m-membered means that the number of atoms in the ring is n to n+m, e.g., 3- to 12-membered rings including 3-membered rings, 4-membered rings, 5-membered rings, 6-membered rings, 7-membered rings, 8-membered rings, 9-membered rings, 10-membered rings, 11-membered rings, and 12-membered rings, as well as any range of n to n+m, e.g., 3- to 12-membered rings including 3- to 6-membered rings, 3- to 9-membered rings, 5- to 6-membered rings, 5- to 7-membered rings, 5- to 10- membered rings, 6- to 7-membered rings, 6- to 8-membered rings, 6- to 9-membered rings, and 6- to 10-membered rings, etc.

The term "leaving group" refers to a functional group or atom that may be substituted with another functional group or atom by a substitution reaction (e.g. an affinity substitution reaction). For example, representative leaving groups include triflate; chlorine, bromine, iodine; sulfonate groups such as mesylate, tosylate, brosylate, tosylate, and the like; acyloxy, such as acetoxy, trifluoroacetoxy, and the like.

The term "protecting group" includes, but is not limited to, an "amino protecting group", a "hydroxy protecting group" or a "mercapto protecting group". The term "amino protecting group" refers to a protecting group suitable for preventing side reactions at the amino nitrogen. Representative amino protecting groups include, but are not limited to: formyl groups; acyl groups, for example alkanoyl (such as acetyl, trichloroacetyl or trifluoroacetyl); alkoxycarbonyl groups such as tert-butoxycarbonyl (Boc); arylmethoxycarbonyl groups such as carbobenzoxy (Cbz) and 9-fluorene methoxycarbonyl (Fmoc); arylmethyl groups, such as benzyl (Bn), trityl (Tr), 1,1-di-(4'-methoxyphenyl) methyl; silyl groups such as trimethylsilicyl (TMS) and t-butyldimethylsilyl (TBS) and the like. The term "hydroxyl protecting group" refers to a protecting group suitable for preventing hydroxyl side reactions. Representative hydroxyl protecting groups include, but are not limited to: alkyl groups such as methyl, ethyl, and t-butyl; acyl groups, such as alkanoyl (such as acetyl); arylmethyl groups, such as benzyl (Bn), p-methoxybenzyl (PMB), 9-fluorenylmethyl (Fm) and diphenylmethyl (benzhydryl, DPM); silyl groups such as trimethylsilicyl (TMS) and t-butyldimethylsilyl (TBS) and the like.

The compounds of the present disclosure may be prepared by a variety of synthetic methods well known to those skilled in the art, including the specific embodiments set forth below, embodiments thereof formed in combination with other chemical synthetic methods, and equivalents thereof known to those skilled in the art. Preferred embodiments include, but are not limited to, the examples of the present disclosure.

The solvents used in the present disclosure are commercially available.

The compounds are named according to conventional naming principles in the art or using ChemDraw® software, and the commercially available compounds adopt the name of the supplier catalogue.

BRIEF DESCRIPTION OF THE DRAWINGS

FIG. 1 is a graph showing the results of an LLC-PK1 cell proliferation inhibition experiment according to an embodiment of the present disclosure.

DETAILED DESCRIPTION OF THE PREFERRED EMBODIMENT

The present application is described in detail below by way of examples, without implying that there are any disadvantageous limitations to the present application. While the present application has been described in detail and specific embodiments thereof have been disclosed, it will be apparent to those skilled in the art that various changes and modifications can be made in the specific embodiments of the present application without departing from the spirit and scope of the disclosure.

The experimental materials and reagents used in the following embodiments are commercially available unless otherwise specified.

**Preparation of intermediates**

Reference Example 1: Preparation of intermediate I-1

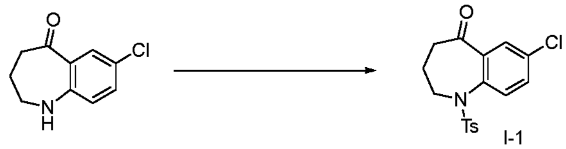

I-1

P-Toluenesulfonyl chloride (21.9 g, 115 mmol) was added to a solution of 7-chloro-1,2,3,4-tetrahydrobenzo[B] azepin -5-one (15 g, 76.7 mmol) in pyridine (150 mL) at room temperature. The reaction solution was allowed to react at room temperature for 16 hours. Concentration was performed under reduced pressure, the reagent was poured into water (200 mL), and extracted with ethyl acetate (100 mL×3), and the organic phases were combined. The organic phases were washed with a saturated sodium chloride solution (100 mL), dried over anhydrous sodium sulfate, and filtered. The filtrate was concentrated under reduced pressure to remove the organic solvent and the residue was purified by silica gel chromatography to afford intermediate I-1.

LC-MS (ESI) $[M+H]^+$ 349.9.

$^1H$ NMR (400 MHz, $CDCl_3$) $\delta$ 7.66 (d, $J$ = 2.4 Hz, 1H), 7.58 (d, $J$ = 8.3 Hz, 2H), 7.47 (dd, $J$ = 8.6, 2.5 Hz, 1H), 7.43 (d, $J$ = 8.5 Hz, 1H), 7.28 (d, $J$ = 8.0 Hz, 2H), 3.83 (t, $J$ = 6.5 Hz, 2H), 2.43 (s, 3H), 2.40 - 2.35 (m, 2H), 2.00 - 1.91 (m, 2H).

Reference Example 2: Preparation of intermediate I-2

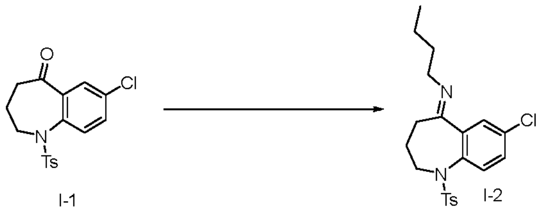

I-1 I-2

Intermediate I-1 (20.0 g, 57.2 mmol) was added to cyclohexane (250 mL), while n-butylamine (8.49 mL, 85.8 mmol) and trifluoroacetic acid (1.00 mL) were added at room temperature. The reaction mixture was refluxed under argon for 48 hours, and concentrated under reduced pressure to obtain a residue. A mixed solution of ethyl acetate and petroleum ether (volume ratio 1: 15, 64 mL) was added to the residue with stirring. After stirring for 10 minutes, the mixture was filtered with suction, and the resulting solid was dried under reduced pressure to obtain intermediate I-2.

$^1H$ NMR (400 MHz, $CDCl_3$) $\delta$ 7.56 - 7.51 (m, 2H), 7.45 (d, $J$ = 2.2 Hz, 1H), 7.41 - 7.38 (m, 1H), 7.37 - 7.34 (m, 1H), 7.22 (d, $J$ = 8.0 Hz, 2H), 3.76 (t, $J$ = 6.2 Hz, 2H), 2.93 (t, $J$ = 7.2 Hz, 2H), 2.40 (s, 3H), 2.10 - 2.06 (m, 2H), 1.76 - 1.69 (m, 2H), 1.65 - 1.58 (m, 2H), 1.41 - 1.35 (m, 2H), 0.96 (t, $J$ = 7.4 Hz, 3H).

Reference Example 3: Preparation of intermediate I-3

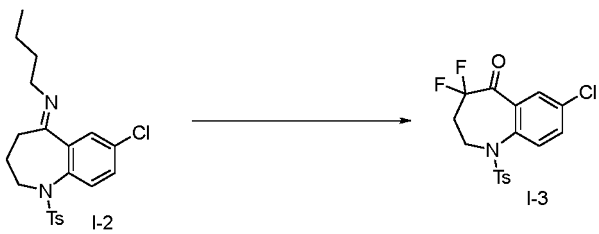

I-2 I-3

Selectfluor (26.2 g, 74.0 mmol) was added to acetonitrile (200 mL) at room temperature. Intermediate I-2 (15.0 g, 37.0 mmol) was added in 5 portions, with one portion every 40 minutes. Upon completion of the addition, the reaction mixture was stirred at room temperature for 72 hours. Ice water (200 mL) was added, and concentrated hydrochloric acid (15.0 mL) was slowly added dropwise with stirring. After stirring for five minutes, the mixture was filtered with suction, and the resulting solid was dried with suction under reduced pressure to give intermediate I-3.

$^1H$ NMR (400 MHz, DMSO-$d_6$) $\delta$ 7.79 - 7.63 (m, 4H), 7.44 (d, $J$ = 8.0 Hz, 2H), 7.37 - 7.32 (m, 1H), 4.07 - 3.98 (m, 2H), 2.67 - 2.53 (m, 2H), 2.41 (s, 3H).

Reference Example 4: Preparation of intermediate I-4

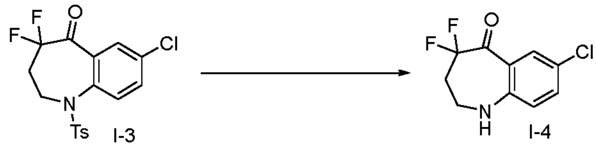

I-3 I-4

Intermediate I-3 (12.0 g, 31.1 mmol) was added to concentrated sulfuric acid (15.0 mL) at 0°C and the reaction mixture was stirred at room temperature for 4 hours. After being poured into ice water, the pH was adjusted to 11 with 50% aqueous sodium hydroxide solution, extraction was performed with ethyl acetate (100 mL× 3), the organic phases were combined and washed with saturated brine (200 mL), dried over anhydrous sodium sulfate and filtered, and then the filtrate was concentrated under reduced pressure to obtain the crude product. The crude product was purified by silica gel chromatography to afford intermediate I-4.

LCMS (ESI) $[M+H]^+$ 232.1.

$^1H$ NMR (400 MHz, DMSO) $\delta$ 7.45 (dd, *J* = 8.4, 3.6 Hz, 2H), 7.34 (dd, *J* = 8.9, 2.6 Hz, 1H), 6.91 (d, *J* = 8.9 Hz, 1H), 3.29 - 3.21 (m, 2H), 2.70 - 2.56 (m, 2H).

Reference Example 5: Preparation of intermediate I-5

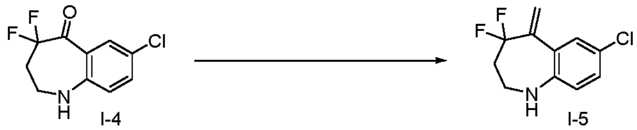

I-4 I-5

Methyl triphenylphosphonium bromide (18.5 g, 51.8 mmol) was added to tetrahydrofuran (120 mL) at room temperature and cooled to 0°C. Potassium tert-butoxide (5.81 g, 51.8 mmol) was added. The reaction mixture was stirred at 0°C for 1 h and then a solution of intermediate I-4 (6.00 g, 25.9 mmol) in tetrahydrofuran (20 mL) was added at 0°C. After the addition, the reaction mixture was stirred at room temperature for 16 hours. The reaction solution was poured into ice water (200 mL), and extracted with ethyl acetate (100 mL×3), and the organic phases were combined and washed with saturated brine (200 mL), dried over anhydrous sodium sulfate, and filtered, while the filtrate was concentrated under reduced pressure to obtain the crude product. The crude product was purified by silica gel chromatography to afford intermediate I-5.

LCMS (ESI) $[M+H]^+$ 230.1.

Reference Example 6: Preparation of intermediate I-6

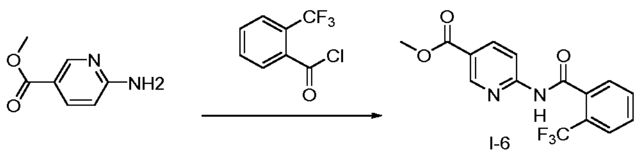

I-6

Methyl 6-aminonicotinate (1.0 g, 6.57 mmol) was dissolved in pyridine (20 mL) and 2-trifluoromethyl benzoyl chloride (1.51 g, 7.25 mmol) was added at room temperature. After the addition was complete, the reaction mixture was stirred at room temperature for 1 h. The reaction mixture was poured into ice water (100 mL), and extracted with ethyl acetate (50 mL×3), and the organic phases were combined and washed with water (50 mL×5), dried over anhydrous sodium sulfate, and filtered, while the filtrate was concentrated under reduced pressure, and the residue was purified by silica gel chromatography to obtain the intermediate I-6.

LC-MS (ESI) $[M+H]^+$ 325.0.

Reference Example 7: Preparation of intermediate I-7

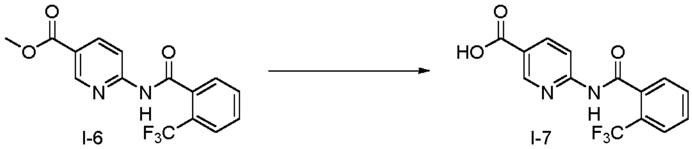


Intermediate I-6 (1.35 g, 4.16 mmol) was dissolved in tetrahydrofuran (10 mL) at room temperature, and a solution of sodium hydroxide (499 mg, 12.5 mmol) in water (2 mL) was added. After the completion of the addition, the reaction mixture was stirred at 70°C for 1 hour. After the completion of the reaction, the reaction solution was adjusted to pH=5 to 6 with 1 N hydrochloric acid. Filtration and drying of the solid afforded intermediate I-7.

LC-MS (ESI) $[M+H]^+$ 311.0.

Reference Example 8: Preparation of intermediate I-8

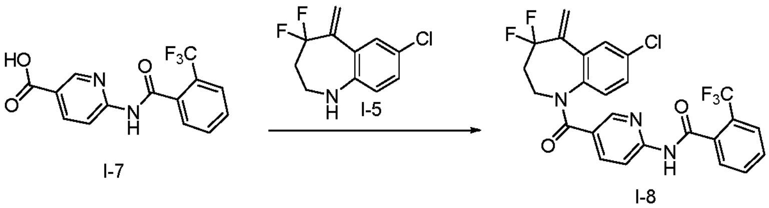


Intermediate I-7 (244 mg, 0.785 mmol) was dissolved in N,N-dimethylacetamide (6.00 mL) at room temperature, cooled to 0°C and thionyl chloride (125 mg, 1.05 mmol) was added. After the reaction mixture was stirred at room temperature for 3 hours, a solution of intermediate I-5 (120 mg, 0.523 mmol) in N,N-dimethylacetamide (3 mL) was added and the reaction mixture was further stirred at room temperature for 16 hours. The reaction solution was added into water (30 mL), and extracted with ethyl acetate (20 mL× 3), and the organic phases were combined and respectively washed with a saturated aqueous sodium bicarbonate solution (50 mL) and saturated brine (50 mL), dried over anhydrous sodium sulfate and filtered, while the filtrate was concentrated under reduced pressure to obtain the crude product. The crude product was purified by silica gel chromatography to afford intermediate I-8.

LCMS (ESI) $[M+H]^+$ 522.2.

Reference Example 9: Preparation of intermediate I-9

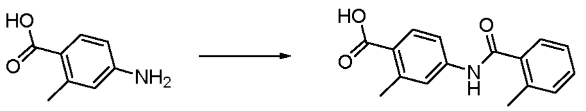


Methylbenzoyl chloride (5.00 g, 32.3 mmol) was dissolved in chloroform (80 mL) and 4-amino-2-methylbenzoic acid (4.88 g, 32.3 mmol), and triethylamine (9.81 g, 96.9 mmol) were added at room temperature. The reaction mixture was stirred at room temperature for 3 hours. The reaction system was poured into water (200 mL), extracted with ethyl acetate (100 mL×3), and the organic phases were combined and washed with saturated brine (100 mL), dried over anhydrous sodium sulfate, and filtered, while the filtrate was concentrated under reduced pressure to give the crude product. The crude product was purified by silica gel chromatography to afford intermediate I-9.

$^1H$ NMR (400 MHz, DMSO-$d_6$) $\delta$ 10.53 (s, 1H), 7.87 (d, $J$ = 8.4 Hz, 1H), 7.73 - 7.62 (m, 2H), 7.51 - 7.45 (m, 1H), 7.44 - 7.37 (m, 1H), 7.36 - 7.25 (m, 2H), 2.53 (s, 3H), 2.39 (s, 3H).

Reference Example 10: Preparation of intermediate I-10

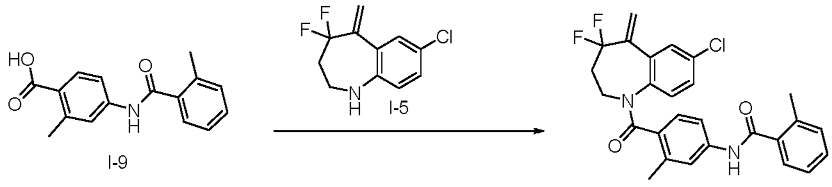

I-9 I-5 I-10

Intermediate I-9 (211 mg, 0.785 mmol) was dissolved in *N,N*-dimethylacetamide (6.00 mL) at room temperature, cooled to 0°C and thionyl chloride (125 mg, 1.05 mmol) was added under argon. After the reaction mixture was stirred at room temperature for 3 hours, a solution of intermediate I-5 (120 mg, 0.523 mmol) in N,N-dimethylacetamide (3.00 mL) was added. The reaction mixture was continued to stir at room temperature for 16 hours. The reaction solution was added into water (30 mL), and extracted with ethyl acetate (20 mL× 3), and the organic phases were combined and sequentially washed with a saturated aqueous sodium bicarbonate solution (50 mL) and saturated brine (50 mL), dried over anhydrous sodium sulfate and filtered, while the filtrate was concentrated under reduced pressure to obtain the crude product. The crude product was purified by silica gel chromatography to afford intermediate I-10.

LCMS (ESI) $[M+H]^+$ 481.1.

Reference Example 11: Preparation of intermediate I-11

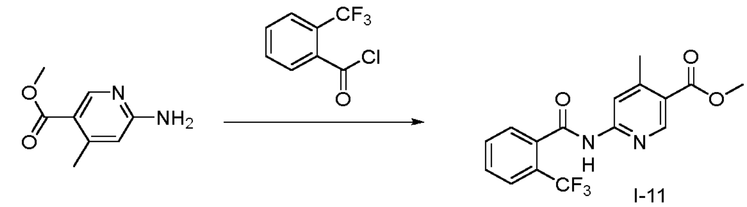

I-11

Methyl 6-amino-4-methylnicotinate (500 mg, 3.01 mmol) was dissolved in pyridine (20 mL) and 2-trifluoromethyl benzoyl chloride (628 mg, 3.01 mmol) was added at room temperature. After the addition was completed, the reaction mixture was stirred at room temperature for 1 hour. The reaction mixture was poured into ice water (100 mL), and extracted with ethyl acetate (50 mL×3), and the organic phases were combined and washed with water (50 mL×5), dried over anhydrous sodium sulfate, and filtered, while the filtrate was concentrated under reduced pressure, and the residue was purified by silica gel chromatography to obtain the intermediate 1-11.

LC-MS (ESI) $[M+H]^+$ 339.1.

Reference Example 12: Preparation of intermediate I-12

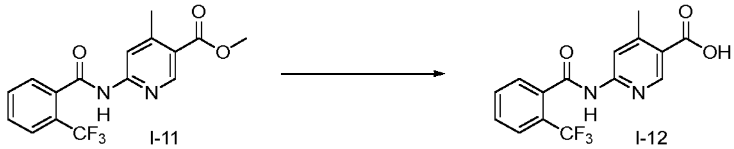

I-11 I-12

Intermediate I-11 (700 mg, 2.07 mmol) was dissolved in tetrahydrofuran (10 mL) and a solution of sodium hydroxide (414 mg, 10.35 mmol) in water (5 mL) was added at room temperature. After the addition was completed, the reaction mixture was stirred at 70°C for 1 hour. The pH of the reaction solution was adjusted to pH=5 to 6 with 1 N hydrochloric acid, the solution was filtered and the solid was dried to give intermediate I-12.

Reference Example 13: Preparation of intermediate I-13

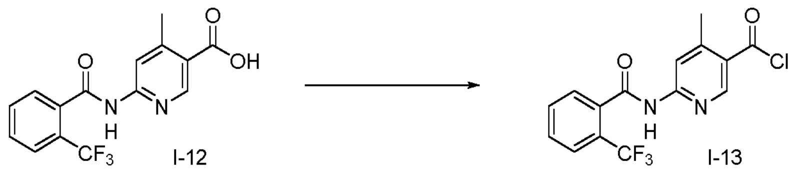

I-12 I-13

Intermediate I-12 (200 mg, 0.617 mmol) was dissolved in dichloromethane (10 mL) and oxalyl chloride (165 mg, 1.30 mmol) and N,N-dimethylformamide (1 drop) were added at 0°C. After the addition was completed, the reaction mixture was stirred at 0°C for 1 hour. The reaction solution was concentrated under reduced pressure to give crude intermediate I-13. The crude was used in the next reaction without further purification.

Reference Example 14: Preparation of intermediate I-14

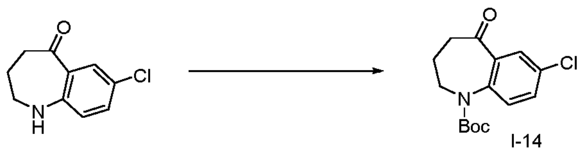

I-14

7-Chloro-1,2,3,4-tetrahydrobenzo[B]azepin-5-one (26.0 g, 0.133 mol) and di-tert-butyl dicarbonate (200 g) were heated to 100°C for 16 hours. After cooling to room temperature, most of the di-tert-butyl dicarbonate was removed under reduced pressure with an oil pump and the residue was purified by silica gel chromatography to give intermediate I-14.

LC-MS (ESI) $[M+H-56]^+$ 239.9.

$^1H$ NMR (400 MHz, DMSO-$d_6$) δ 7.66 - 7.58 (m, 2H), 7.48 (d, *J* = 8.4 Hz, 1H), 3.66 (s, 2H), 2.64 (t, *J* = 6.6 Hz, 2H), 2.07 - 1.96 (m, 2H), 1.40 (d, *J* = 13.8 Hz, 9H).

Reference Example 15: Preparation of intermediate I-15

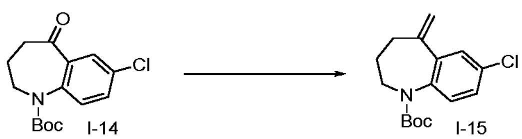

I-14 I-15

Methyl triphenylphosphonium bromide (47.9 g, 0.134 mol) was added to a solution of potassium tert-butoxide (18.9 g, 0.168 mol) in tetrahydrofuran (500 mL) at 0°C under argon and the reaction solution was stirred at 0°C for 30 minutes. Intermediate I-14 (33.0 g, 0.112 mol) was then added to the reaction. The reaction solution was stirred at room temperature for 16 hours. The reaction solution was diluted into water (500 mL) and extracted with ethyl acetate (500 mL×2). The organic phases were combined and washed with water (500 mL) and brine (500 mL), dried over anhydrous sodium sulfate, and filtered, and the filtrate was concentrated under reduced pressure to remove the organic solvent to give the crude product, which was purified by silica gel chromatography to give intermediate 1-15.

LC-MS (ESI) $[M+H-56]^+$ 238.0.

$^1H$ NMR (400 MHz, DMSO-$d_6$) δ 7.36 (s, 1H), 7.30 (dd, *J* = 8.5, 2.5 Hz, 1H), 7.22 (d, *J* = 8.5 Hz, 1H), 5.20 (d, *J* = 11.7 Hz, 2H), 3.51 (s, 2H), 2.37 (t, *J* = 6.1 Hz, 2H), 1.81 (s, 2H), 1.45 - 1.28 (m, 9H).

Reference Example 16: Preparation of intermediate I-16

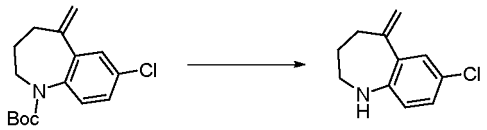

I-15 I-16

Intermediate I-15 (6.10 g, 20.8 mmol) was dissolved in a mixed solution of trifluoroacetic acid/dichloromethane (20.0 mL/40.0 mL) at room temperature, and the reaction mixture was stirred at room temperature for 3 hours. The system was poured into saturated sodium bicarbonate solution (200 mL), and extracted with dichloromethane (50 mL× 4), and the organic phases were combined and washed with saturated brine (50 mL), dried over anhydrous sodium sulfate and filtered. The filtrate was concentrated under reduced pressure to obtain the crude product, which was purified by silica gel chromatography to obtain the intermediate I-16.
$^1$H NMR (400 MHz, DMSO-$d_6$) $\delta$ 7.11 (d, J = 2.5 Hz, 1H), 7.00 (dd, J = 8.6, 2.5 Hz, 1H), 6.69 (d, J = 8.6 Hz, 1H), 5.17 (d, J = 1.6 Hz, 1H), 4.99 - 4.93 (m, 1H), 3.18 - 3.07 (m, 2H), 2.50 - 2.45 (m, 2H), 1.88 - 1.76 (m, 2H).

Reference Example 17: Preparation of intermediate I-17

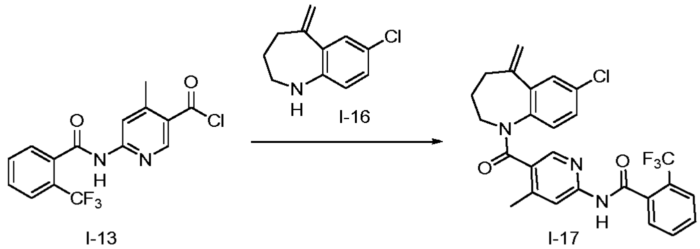

I-16

I-13 I-17

Intermediate I-13 (150 mg) was dissolved in pyridine (10 mL) and intermediate I-16 (60 mg, 0.310 mmol) was added at room temperature. After complete addition, the reaction mixture was stirred at room temperature for 16 hours. The reaction solution was diluted with water (10 mL), and extracted with ethyl acetate (10 mL×3), and the organic phases were combined and washed with water (10 mL×3), dried over anhydrous sodium sulfate, and filtered, while the filtrate was concentrated under reduced pressure, and the residue was purified by silica gel chromatography to obtain the intermediate I-17.
LC-MS (ESI) [M+H]$^+$ 500.1.

Reference Example 18: Preparation of intermediate I-18

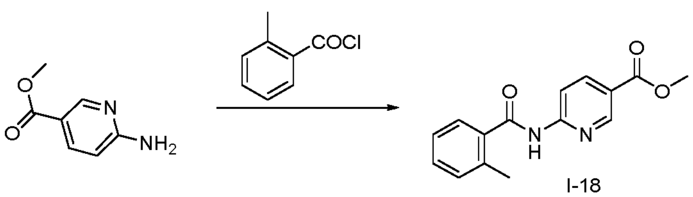

I-18

Methylbenzoyl chloride (6.11 g, 39.5 mmol) was added to a solution of methyl 6-aminonicotinate (5.00 g, 32.9 mmol) in pyridine (40.0 mL) at room temperature. After the reaction mixture was stirred at room temperature for 2 hours, it was poured into water (300 mL), filtered by suction, and the resulting solid was dried to give intermediate I-18.
LC-MS (ESI) [M+H]$^+$ 271.0.

Reference Example 19: Preparation of intermediate I-19

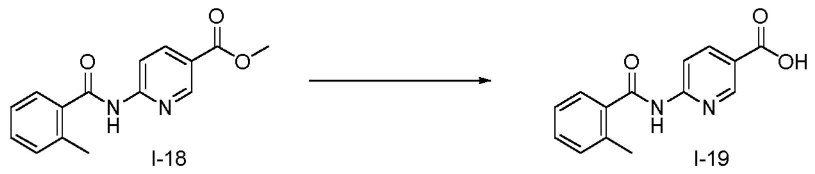

Sodium hydroxide (2.66 g, 66.6 mmol) was added to a solution of intermediate I-18 (6.00 g, 22.2 mmol) in methanol/water (60 mL/30 mL) at room temperature. The reaction mixture was stirred at 70°C for 30 minutes. The reaction mixture was cooled to room temperature, adjusted to pH=5 to 6 with 3 N dilute hydrochloric acid in an ice-water bath, extracted with ethyl acetate (50 mL×6). Th organic phases were combined and washed with brine (100 mL), dried over anhydrous sodium sulfate, and filtered, and the filtrate was concentrated under reduced pressure to remove the organic solvent to afford intermediate 1-19.

LC-MS (ESI) $[M+H]^+$ 257.1.

Reference Example 20: Preparation of intermediate I-20

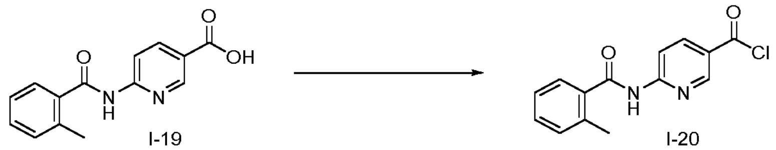

Intermediate I-19 (1.50 g, 5.85 mmol) and N,N-dimethylformamide (0.10 mL) were added to dichloromethane (20.0 mL) at room temperature, the reaction system was cooled to 0°C and oxalyl chloride (1.49 g, 11.7 mmol) was added. The reaction solution was stirred at 0°C for 1 h and concentrated to dryness to obtain the crude intermediate I-20. The crude was used in the next reaction without further purification.

Reference Example 21: Preparation of intermediate I-21

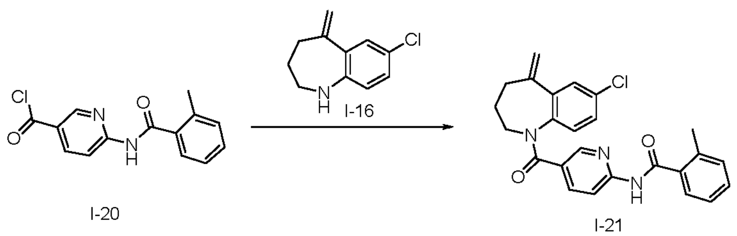

Intermediate I-16(378 mg, 1.95 mmol) was added to pyridine (5.00 mL), and intermediate I-20 (500 mg) was added in one portion at room temperature. After the reaction solution was stirred at room temperature for 16 hours, it was poured into water (30 mL) and extracted with ethyl acetate (20 mL× 3), and the organic phases were combined and washed with saturated brine (50 mL), dried over anhydrous sodium sulfate, and filtered and the filtrate was concentrated under reduced pressure to obtain the crude product. The crude product was purified by silica gel chromatography to afford intermediate I-21.

LC-MS (ESI) $[M+H]^+$ 432.1.

Reference Example 22: Preparation of intermediate I-22

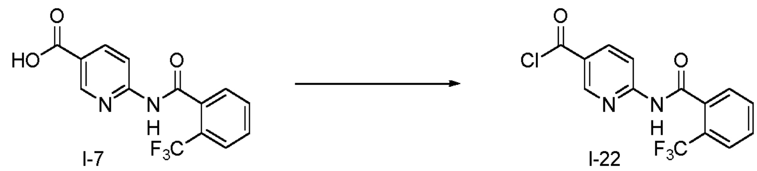

Intermediate I-7 (200 mg, 0.65 mmol) was dissolved in dichloromethane (10 mL), and then oxalyl chloride (165 mg, 1.30 mmol) and N,N-dimethylformamide (1 drop) were added at 0°C. After the addition was completed, the reaction mixture was stirred at 0°C for 1 hour. The reaction solution was concentrated under reduced pressure to afford crude intermediate I-22. The crude was used in the next reaction without further purification.

Reference Example 23: Preparation of intermediate I-23

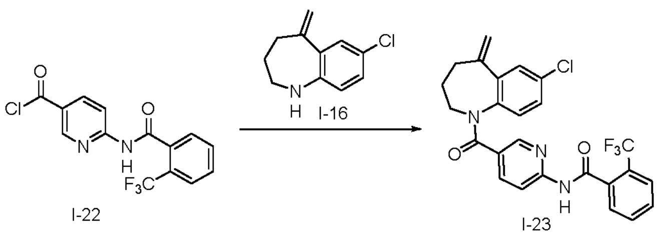

Intermediate I-22 (214 mg, 0.65 mmol) was dissolved in pyridine (10 mL) and intermediate I-16(97 mg, 0.501 mmol) was added at room temperature. After the addition was completed, the reaction mixture was stirred at room temperature for 16 hours. The reaction solution was diluted with water (10 mL), and extracted with ethyl acetate (10 mL×3), and the organic phases were combined and washed with water (10 mL×3), dried over anhydrous sodium sulfate, and filtered, while the filtrate was concentrated under reduced pressure, and the residue was purified by silica gel chromatography to obtain the intermediate I-23.

LC-MS (ESI) $[M+H]^+$ 486.2.

Reference Example 24: Preparation of intermediate I-24

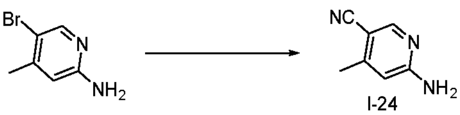

Cuprous cyanide (24.0 g, 0.268 mol) was added to a solution of 2-amino-5-bromo-4-methylpyridine (25.0 g, 0.134 mol) in N,N-dimethylacetamide (230 mL) at room temperature. The reaction mixture was stirred at 170°C for 16 hours under argon. After cooling to 0°C, ethylenediamine (50 mL) and water (500 mL) were added to the reaction solution and stirred for 15 min to quench the reaction. The mixture was extracted with ethyl acetate (300 mL×3), the organic phases were combined and washed with water (300 mL×3) and brine (500 mL), dried over anhydrous sodium sulfate, and filtered, and the filtrate was concentrated under reduced pressure to remove the organic solvent to obtain the crude product. The crude product was purified by silica gel chromatography to afford intermediate I-24.

LC-MS (ESI) $[M+H]^+$ 134.1.

$^1H$ NMR (400 MHz, DMSO-$d_6$) δ 8.23 (s, 1H), 6.89 (s, 2H), 6.35 (s, 1H), 2.25 (s, 3H).

Reference Example 25: Preparation of intermediate I-25

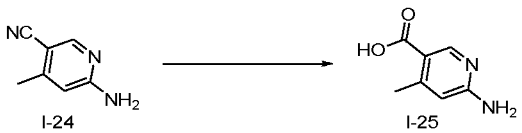

I-24 I-25

Intermediate I-24 (10.2 g, 76.6 mmol) was added to a solution of aqueous sodium hydroxide (90 mL, 10 mol/L) and ethanol (90 mL) at room temperature and the reaction mixture was refluxed for 16 hours. The reaction mixture was cooled to room temperature, the pH was adjusted to neutral with hydrochloric acid (6 mol/L), precipitation was performed to obtain solid, and filtration was performed, while the filter cake was washed with water and dried to give intermediate I-25.
LC-MS (ESI) $[M+H]^+$ 153.3.

Reference Example 26: Preparation of intermediate I-26

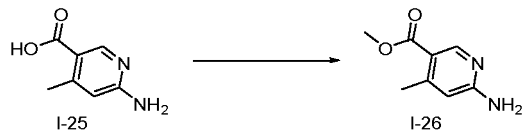

I-25 I-26

Thionyl chloride (15 mL) was added to a solution of intermediate I-25 (7.8g, 51.3 mmol) in methanol (80 mL) at room temperature. The reaction mixture was refluxed for 16 hours. After cooling to room temperature, most of the methanol solvent was removed under reduced pressure, dilution was made with water (100 mL) and PH was adjusted to 13 with an aqueous sodium hydroxide solution (2 mol/L), a solid was precipitated out, filtered with suction, the filter cake was washed with water and dried to give intermediate I-26.
LC-MS (ESI) $[M+H]^+$ 167.1.

Reference Example 27: Preparation of intermediate I-27

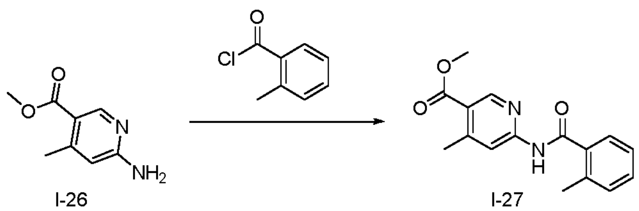

I-26 I-27

Methylbenzoyl chloride (5.14 g, 33.2 mmol) was added to a solution of intermediate I-26 (4.6 g, 27.7 mmol) in pyridine (35 mL) at room temperature, and the reaction mixture was stirred at room temperature for 3 hours. Ice water (70 mL) was added to the reaction solution to precipitate a solid, which was filtered and the filter cake was washed with plenty of water and petroleum ether and dried to afford intermediate I-27.
LC-MS (ESI) $[M+H]^+$ 285.2.

Reference Example 28: Preparation of intermediate I-28

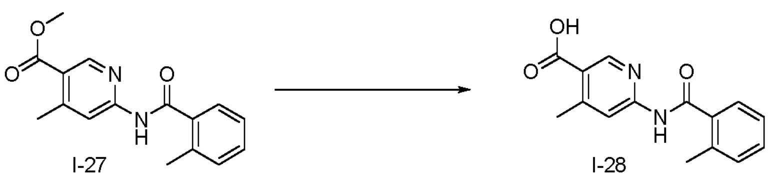

I-27 I-28

5% of aqueous sodium hydroxide solution (48 mL) was added to a solution of intermediate I-27 (4.1 g, 14.4 mmol) in methanol (60 mL) at room temperature, and the reaction mixture was reacted at 70°C for 30 min. The reaction mixture was cooled to room temperature, added with water (50 mL) to dilute, and the pH was adjusted to slightly acidic with hydrochloric acid (3 mol/L) to precipitate a solid, and filtration was performed, the filter cake was washed with copious amounts of water and lyophilize to give intermediate I-28.

LC-MS (ESI) $[M+H]^+$ 271.1.

$^1H$ NMR (400 MHz, DMSO-$d_6$) δ 13.04 (s, 1H), 10.99 (s, 1H), 8.75 (s, 1H), 8.16 (s, 1H), 7.48 (d, *J* = 7.6 Hz, 1H), 7.43 - 7.35 (m, 1H), 7.32 - 7.24 (m, 2H), 2.60 (s, 3H), 2.39 (s, 3H).

Reference Example 29: Preparation of intermediate I-29

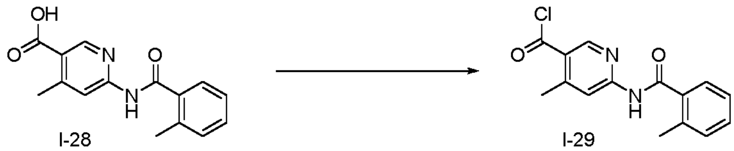

I-28 I-29

Oxalyl chloride (2.07 g, 16.3 mmol) and N,N-dimethylformamide (1 drop) were added to a solution of intermediate I-28 (2.2 g, 8.14 mmol) in dichloromethane (30 mL), and the reaction solution reacted at 0°C for 30 minutes. Concentration of the dry solvent at low temperature gave crude intermediate I-29, which was used in the next reaction without purification.

Reference Example 30: Preparation of intermediate I-30

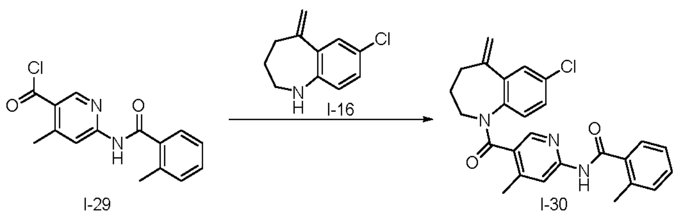


I-29 I-30

Intermediate I-29 (1.75 g) was added to a solution of intermediate I-16(900 mg, 4.65 mmol) in pyridine (30 mL) at room temperature. The reaction mixture was stirred at room temperature for 3 hours. Water (50 mL) was added and extraction was made with ethyl acetate (30 mL×2). The organic phases were combined and washed with water (30 mL×3) and saturated brine (30 mL), dried over anhydrous sodium sulfate, and filtered, and the filtrate was concentrated under reduced pressure to remove the organic solvent to give the crude product. The crude product was purified by silica gel chromatography to afford intermediate I-30.

LC-MS (ESI) $[M+H]^+$ 446.2.

Reference Example 31: Preparation of intermediate I-31

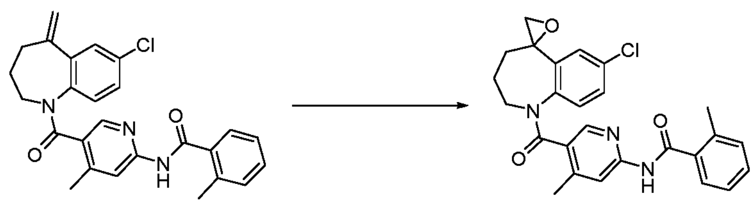

I-30 I-31

N-Bromosuccinimide (110 mg, 0.618 mmol) was added to a solution of intermediate I-30 (230 mg, 0.516 mmol) in tetrahydrofuran/water (15 mL/3 mL) at room temperature and the reaction solution was stirred at room temperature

for 16 hours. Aqueous sodium hydroxide (2 mL, 2.5 N) was added and the reaction solution was stirred at room temperature for 2 hours. The reaction solution was diluted into water (20 mL) and extracted with ethyl acetate (20 mL×2). The organic phases were combined and washed with saturated brine (20 mL), dried over anhydrous sodium sulfate, and filtered, and the filtrate was concentrated under reduced pressure to remove the organic solvent to give the crude product. The crude product was purified by silica gel chromatography to afford intermediate 1-31.

LC-MS (ESI) $[M+H]^+$ 462.0.

$^1H$ NMR (400 MHz, DMSO-$d_6$) δ 10.97 - 10.53 (m, 1H), 8.47 - 7.63 (m, 2H), 7.56 - 7.10 (m, 6H), 7.03 - 6.66 (m, 1H), 4.98 - 4.67 (m, 1H), 3.30 - 2.62 (m, 3H), 2.48 - 2.25 (m, 6H), 2.21 - 1.99 (m, 2H), 1.91 - 1.52 (m, 2H).

Reference Example 32: Preparation of intermediate I-32

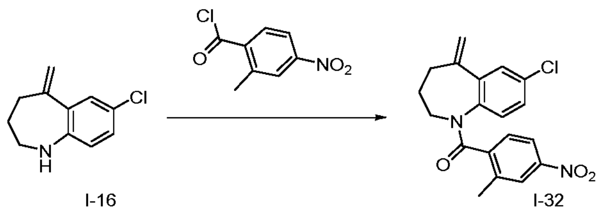

I-16 I-32

2-Methyl-4-nitrobenzoyl chloride (2.06 g, 10.3 mmol) and triethylamine (3.13 g, 30.9 mmol) were added to a solution of intermediate I-16(2.0 g, 10.3 mmol) in dichloromethane (40 mL) at room temperature. The reaction mixture was stirred at room temperature for 1 hour, poured into water (100 mL), and extracted with dichloromethane (50 mL×3), and the organic phases were combined and washed with saturated brine (50 mL), dried over anhydrous sodium sulfate, and filtered and the filtrate was concentrated under reduced pressure to give the crude product. The crude product was purified by silica gel chromatography to afford intermediate I-32.

LC-MS (ESI) $[M+H]^+$ 356.9.

Reference Example 33: Preparation of intermediate I-33

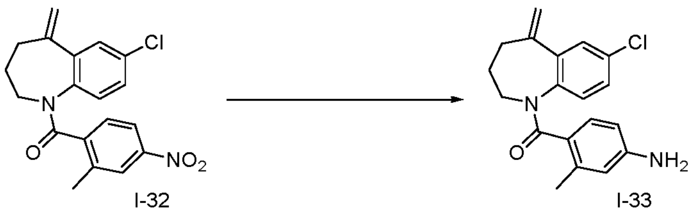

I-32 I-33

Iron powder (282 mg, 5.05 mmol) and ammonium chloride (540 mg, 10.1 mmol) were added to a solution of intermediate I-32 (360 mg, 1.01 mmol) in methanol/water (20 mL/5 mL) at room temperature. The reaction solution was stirred at 60°C for 3 hours under argon. The reaction solution was cooled to room temperature, filtered and the filtrate was concentrated to dryness to give the crude product. The crude product was purified by silica gel chromatography to afford intermediate I-33.

LC-MS (ESI) $[M+H]^+$ 327.0.

Reference Example 34: Preparation of intermediate I-34

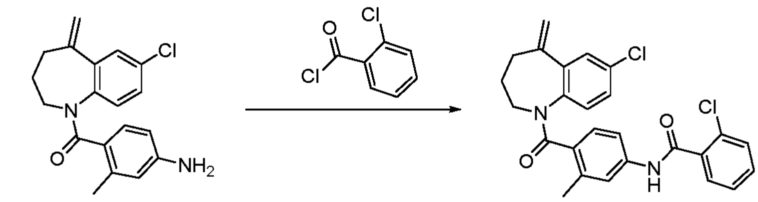

I-33 I-34

Chlorobenzoyl chloride (225 mg, 1.29 mmol) and triethylamine (260 mg, 2.59 mmol) were added to a solution of intermediate I-33 (280 mg, 0.857 mmol) in 1,2-dichloroethane (20 mL) at room temperature. The reaction mixture was stirred at 50°C for 2 hours. The reaction mixture was cooled to room temperature, the reaction solution was diluted with dichloromethane (20 mL), washed with saturated brine (20 mL), dried over anhydrous sodium sulfate, and filtered, while the filtrate was concentrated to dryness to obtain the crude product. The crude product was purified by silica gel chromatography to afford intermediate I-34.

LC-MS (ESI) $[M+H]^+$ 465.2.

Reference Example 35: Preparation of intermediate I-35

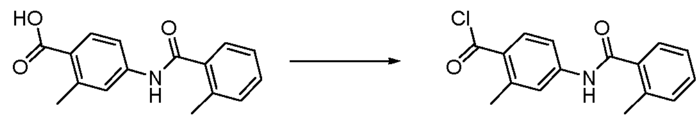

I-9 I-35

Intermediate I-9 (2.00 g, 7.43 mmol) was added to a solution of thionyl chloride (20.0 mL) at room temperature and the reaction mixture was stirred at 40°C for 4 hours. The reaction system was concentrated under reduced pressure to afford crude intermediate I-35, which was used directly in the next step.

Reference Example 36: Preparation of intermediate I-36

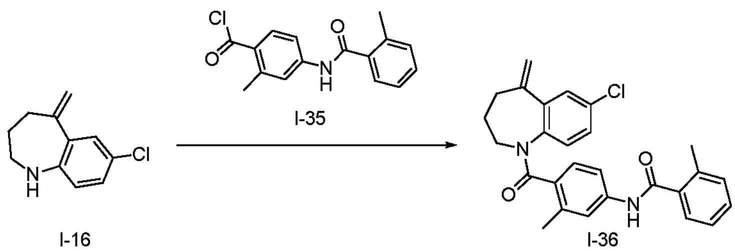

I-35

I-16 I-36

Intermediate I-16(1.42 g, 7.31 mmol) was dissolved in pyridine (30.0 mL) at room temperature, intermediate I-35 (2.00 g) was added and the reaction mixture was stirred at room temperature for 8 hours. The reaction system was poured into water (100 mL), and extracted with ethyl acetate (50 mL×3), and the organic phases were combined and washed with saturated brine (50 mL), dried over anhydrous sodium sulfate, and filtered, while the filtrate was concentrated under reduced pressure to give the crude product. The crude product was purified by silica gel chromatography to afford intermediate I-36.

LC-MS (ESI) $[M+H]^+$ 445.0.

Reference Example 37: Preparation of intermediate I-37

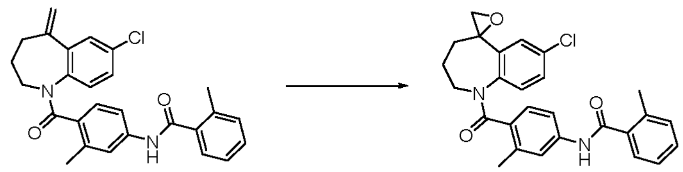

I-36 I-37

Intermediate I-36 (1.00 g, 2.25 mmol) was dissolved in dichloromethane (30.0 mL) at room temperature, m-chloroperoxybenzoic acid (1.95 g, 11.3 mmol) was added and the reaction system was stirred at room temperature for 10 hours. The reaction system was concentrated under reduced pressure at room temperature to afford the crude product, which was purified by C18 reverse phase chromatography to afford intermediate I-37.

$^1$H NMR (400 MHz, DMSO-$d_6$) $\delta$ 10.41 - 10.23 (m, 1H), 7.77 - 7.10 (m, 9H), 6.83 - 6.63 (m, 1H), 5.04 - 3.53 (m, 1H), 3.21 - 2.68 (m, 3H), 2.37 - 2.33 (m, 6H), 2.13 - 1.56 (m, 4H).

Reference Example 38: Preparation of intermediate I-38

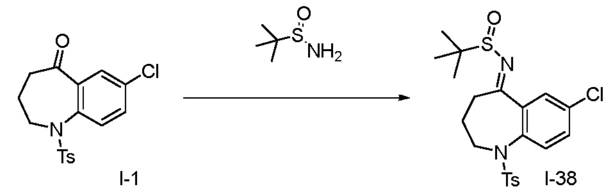

I-1 I-38

Intermediate I-1 (1.00 g, 2.86 mmol) was dissolved in tetrahydrofuran (10 mL) at 25°C, tert-butylsulfinamide (415.74 mg, 3.43 mmol), tetraethyl titanate (1.30 g, 5.72 mmol) were added and stirred at microwave 80°C for 3 hours. It was added with water (10 mL) and filtered, the filter cake was washed with ethyl acetate (20 mL), the filtrate was collected, the filtrate was washed with saturated brine (10 mL) and dried over anhydrous sodium sulfate and filtered, while the filtrate was concentrated to dryness to obtain the crude product. The crude product was purified by silica gel chromatography to afford intermediate I-38.

LC-MS (ESI) $[2M+H]^+$ 453.0.

Reference Example 39: Preparation of intermediate I-39

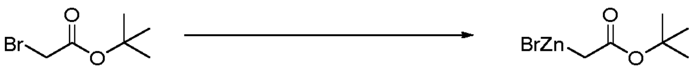

I-39

Zinc dust (2.01 g, 30.76 mmol) was suspended in tetrahydrofuran (100 mL) at 25°C, and nitrogen was replaced three times. 1,2-Dibromoethane (96.31 mg, 0.51 mmol), trimethylsilyl chloride (278.48 mg, 2.56 mmol) were added and stirred at 65°C for 1 hour. tert-Butyl bromoacetate (5.00 g, 25.63 mmol) was added dropwise and after the addition was complete, it was stirred at 50°C for 1 hour. Cooling was made to obtain a solution of intermediate I-39 in tetrahydrofuran (100 mL, 25.63 mmol).

Reference Example 40: Preparation of intermediate I-40

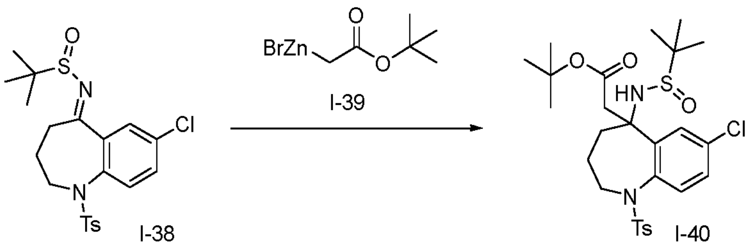

I-38 I-40

To a solution of intermediate I-39 in tetrahydrofuran (100 mL, 25.63 mmol) was added dropwise a solution of intermediate I-38 (850.00 mg, 1.88 mmol) in tetrahydrofuran (20 mL) at 25°C, and the reaction solution was stirred for reaction at 50°C for 16 hours. Water (100 mL) was added and extraction was performed with ethyl acetate (100 mL×3). The organic phase was added and washed with brine (20 mL), dried over anhydrous $Na_2SO_4$, and filtered, and the filtrate was concentrated to give the crude product. The crude product was purified by silica gel chromatography to afford intermediate I-40.

LC-MS (ESI) $[M+H]^+$ 569.2.

Reference Example 41: Preparation of intermediate I-41

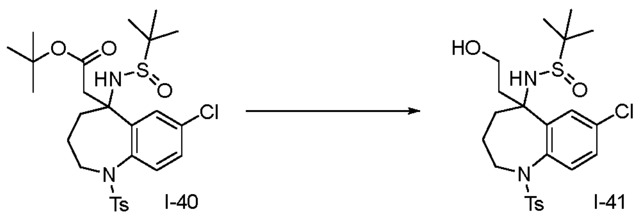
I-40 I-41

Intermediate I-40 (350.00 mg, 0.61 mmol) was dissolved in tetrahydrofuran (10 mL) and a solution of diisobutylaluminum hydride in toluene (2.05 mL, 3.07 mmol, 1.5 M) was added at 25°C and reacted for 16 hours at room temperature. Water (0.12 mL), a 15% aqueous sodium hydroxide solution (0.12 mL), and water (0.3 mL) were added, followed by anhydrous sodium sulfate, and the mixture was stirred at room temperature for 0.5 hours. The filtrate was concentrated and purified by silica gel chromatography to afford intermediate I-41.

LC-MS (ESI) $[M+H]^+$ 499.2.

Reference Example 42: Preparation of intermediate I-42

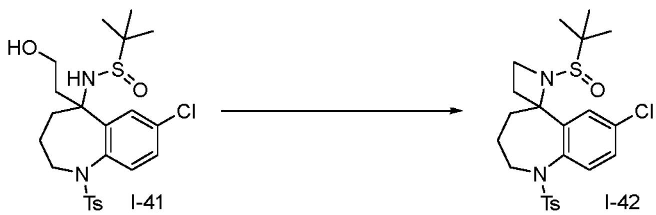
I-41 I-42

Intermediate I-41 (170.00 mg, 0.34 mmol) was dissolved in toluene (10 mL) at 25°C, cyanomethylene tri-n-butylphosphine (98.47 mg, 0.41 mmol) was added and the reaction solution was stirred at 110°C for 3 hours. Cooling was performed to room temperature, water was added (10 mL) and extraction was made with ethyl acetate (10 mL×3). The organic phases were combined and washing was made with brine (10 mL), dried over anhydrous sodium sulfate, and filtered, and the filtrate was concentrated and purified by silica gel chromatography to afford intermediate I-42.

LC-MS (ESI) $[M+H]^+$ 481.2.

Reference Example 43: Preparation of intermediate I-43

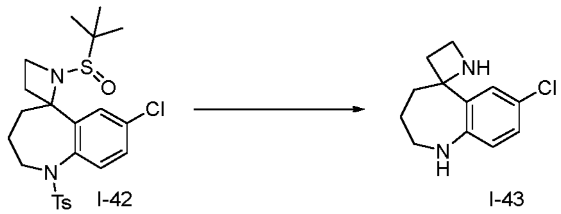

I-42 I-43

Intermediate I-42 (100.00 mg, 0.21 mmol) was dissolved in concentrated hydrochloric acid (5 mL) at 25°C and reacted at 100°C for 16 hours. The solution was directly concentrated to dryness and purified via C18 reverse phase column to obtain intermediate I-43.

LC-MS (ESI) $[M+H]^+$ 223.0.

Reference Example 44: Preparation of intermediate I-44

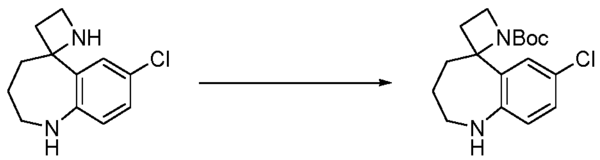

I-43 I-44

Intermediate I-43 (40.00 mg, 0.18 mmol) was dissolved in tetrahydrofuran (5 mL), a 10% aqueous sodium bicarbonate solution (5 mL), and di-tert-butyl dicarbonate (47.04 mg, 0.22 mmol) was added at 25°C and reacted at room temperature for 1 hour. Water (10 mL) was added and extraction was performed with ethyl acetate (10 mL×3). The organic phase was combined and washing was made with brine (10 mL), dried over anhydrous sodium sulfate, and filtered, and the filtrate was concentrated and purified by silica gel chromatography to afford intermediate I-44.

LC-MS (ESI) $[M+H]^+$ 323.2.

Reference Example 45: Preparation of intermediate I-45

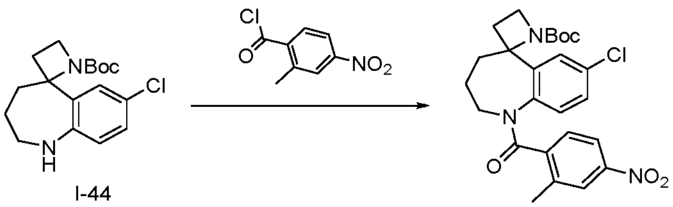

I-44 I-45

Intermediate I-44 (50.00 mg, 0.15 mmol) was dissolved in dichloromethane (10 mL) at 25°C, triethylamine (47.02 mg, 0.46 mmol), 2-methyl-4-nitrobenzoyl chloride (46.37 mg, 0.23 mmol) were added and the reaction solution was stirred at room temperature for 16 hours. Water (10 mL) was added and extraction was performed with dichloromethane (10 mL×3). The organic phases were combined and washing was made with brine (10 mL), dried over anhydrous sodium sulfate, and filtered, and the filtrate was concentrated and purified by silica gel chromatography to afford intermediate I-45.

LC-MS (ESI) $[M-100+H]^+$ 386.2.

Reference Example 46: Preparation of intermediate I-46

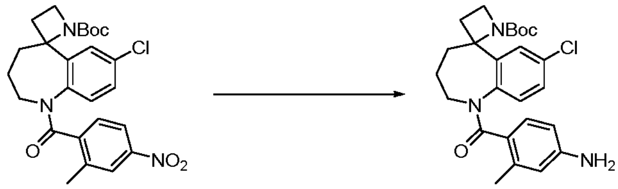

I-45 I-46

Intermediate I-45 (50.00 mg, 0.10 mmol) was dissolved in ethanol (4 mL), and reduced iron powder (28.73 mg, 0.51 mmol) and a saturated aqueous ammonium chloride solution (2 mL) were added at 25°C and the reaction solution was stirred at 80°C for 5 hours. Filtration was performed, the filter cake was washed with ethyl acetate (10 mL), the filtrate was collected and water (10 mL) was added for stratification. The organic phase was collected, concentrated, and purified by silica gel chromatography to afford intermediate I-46.

LC-MS (ESI) $[M-100+H]^+$ 356.2.

Reference Example 47: Preparation of intermediate I-47

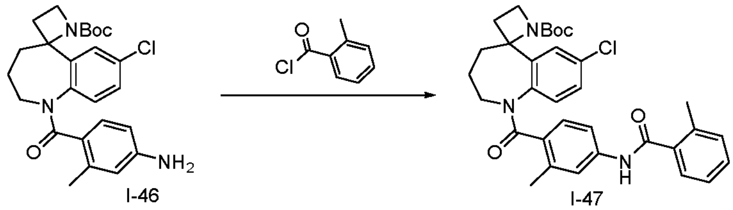

I-46 I-47

Intermediate I-46 (30.00 mg, 0.066 mmol) was dissolved in dichloromethane (5 mL) and triethylamine (19.97 mg, 0.20 mmol) was added followed by 2-methylbenzoyl chloride (12.21 mg, 0.079 mmol) at 25°C and reacted at room temperature for 1 hour. Water (10 mL) was added and extraction was performed with dichloromethane (10 mL×3). The organic phase was combined and washing was made with brine (10 mL), dried over anhydrous sodium sulfate, and filtered, and the filtrate was concentrated to dryness and purified by silica gel chromatography to afford intermediate I-47.

LC-MS (ESI) $[M-100+H]^+$ 474.2.

Reference Example 48: Preparation of intermediate I-48

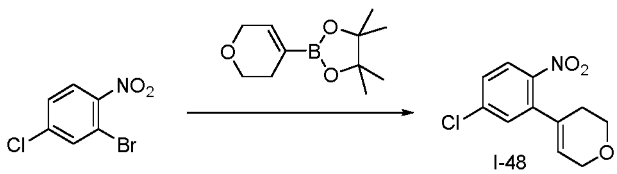

I-48

Cesium carbonate (82.7 g, 254 mmol) and tetrakis triphenylphosphine palladium (2.93 g, 2.54 mmol) were added to a solution of 2-bromo-4-chloro-1-nitrobenzene (20.0 g, 84.6 mmol) and 3,6-dihydro-2H-pyran-4-boronic acid pinacol ester (21.3 g, 101.4 mmol) in dioxane/water (600 mL/200 mL) at room temperature and the reaction mixture was stirred at 100°C for 5 hours under argon. After being cooled to room temperature, most of the solvent was removed under reduced pressure, and extraction was made with ethyl acetate (100 mL×3), and the organic phases were combined and washed with brine (200 mL), dried over anhydrous sodium sulfate, filtered and the filtrate was concentrated under reduced pressure to remove the organic solvent to give the crude product. The crude product was purified by silica gel chromatography to afford intermediate I-48.

LC-MS (ESI) $[M+H]^+$ 240.0.

$^1H$ NMR (400 MHz, $CD_3OD$) δ 7.92 (d, *J* = 8.7 Hz, 1H), 7.52 (dd, *J* = 8.7, 2.3 Hz, 1H), 7.44 (d, *J* = 2.3 Hz, 1H), 5.75 - 5.71 (m, 1H), 4.26 - 4.21 (m, 2H), 3.89 (t, *J* = 5.3 Hz, 2H), 2.36 - 2.31 (m, 2H).

Reference Example 49: Preparation of intermediate I-49

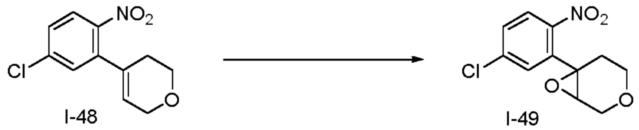

I-48 I-49

m-Chloroperoxybenzoic acid (16.2 g, 93.9 mmol) was added to a solution of intermediate I-48 (15.0 g, 62.6 mmol) in dichloromethane (100 mL) at room temperature. The reaction mixture was stirred at room temperature for 16 hours. The reaction mixture was diluted with dichloromethane (100 mL), washed with a saturated aqueous sodium thiosulfate solution (100 mL× 3), a saturated aqueous sodium carbonate solution (100 mL× 3) and saturated brine (200 mL), dried over anhydrous sodium sulfate, and filtered, and the filtrate was concentrated under reduced pressure to remove the organic solvent to obtain the crude product. The crude product was purified by silica gel chromatography to afford intermediate I-49.

LC-MS (ESI) $[M+H]^+$ 256.0.

$^1$H NMR (400 MHz, DMSO-$d_6$) δ 8.17 (d, *J* = 8.7 Hz, 1H), 7.77 - 7.68 (m, 2H), 4.01 - 3.88 (m, 2H), 3.69 - 3.61 (m, 1H), 3.53 - 3.45 (m, 1H), 3.37 (s, 1H), 2.20 - 2.11 (m, 1H), 2.03 - 1.95 (m, 1H).

Reference Example 50: Preparation of intermediate I-50

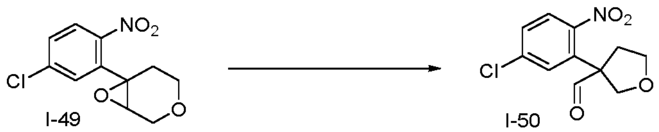

I-49 I-50

Boron trifluoride diethyl etherate (4.37 g, 30.8 mmol) was added to a solution of intermediate I-49 (7.5 g, 29.3 mmol) in dichloromethane (70 mL) under an ice-water bath. The reaction mixture was stirred at 0°C for 30 min. The reaction mixture was washed with saturated brine (50 mL), dried over anhydrous sodium sulfate, and filtered, and the filtrate was concentrated under reduced pressure to remove the organic solvent to give the crude product. The crude product was purified by silica gel chromatography to afford intermediate I-50.

LC-MS (ESI) $[M+H]^+$ 256.0.

$^1$H NMR (400 MHz, DMSO-$d_6$) δ 9.56 (s, 1H), 8.07 (d, *J* = 8.7 Hz, 1H), 7.72 (dd, *J* = 8.7, 2.2 Hz, 1H), 7.65 (d, *J* = 3.6 Hz, 1H), 4.31 (d, *J* = 9.8 Hz, 1H), 4.05 (d, *J* = 9.9 Hz, 1H), 4.00 - 3.86 (m, 2H), 2.69 - 2.60 (m, 1H), 2.40 - 2.31 (m, 1H).

Reference Example 51: Preparation of intermediate I-51

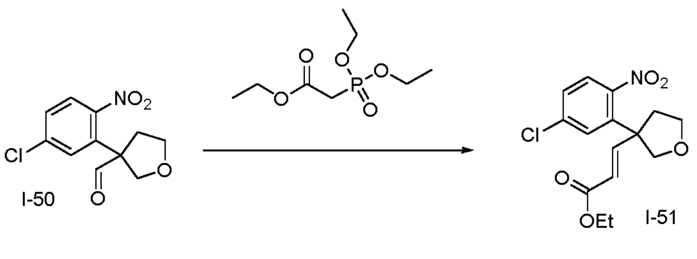

I-50 I-51

Sodium hydride (278 mg, 60% wt, 6.95 mmol) was added to triethyl phosphonoacetate (1.18 g, 5.26 mmol) in tetrahydrofuran (30 mL) under argon in an ice-water bath and stirred for 30 minutes in an ice-water bath, then Intermediate I-50 (890 mg, 3.48 mmol) was added and the reaction mixture was allowed to react at room temperature for 2 hours. The reaction mixture was added water (30 mL) and extracted with ethyl acetate (20 mL×2). The organic phases were combined and washed with saturated brine (50 mL), dried over anhydrous sodium sulfate, and filtered, and the filtrate

was concentrated under reduced pressure to remove the organic solvent to give the crude product. The crude product was purified by silica gel chromatography to afford intermediate I-51.

LC-MS (ESI) $[M+H]^+$ 326.1.

$^1H$ NMR (400 MHz, DMSO-$d_6$) δ 7.91 - 7.84 (m, 1H), 7.70 - 7.63 (m, 2H), 7.08 (d, *J* = 15.9 Hz, 1H), 5.68 (d, *J* = 15.9 Hz, 1H), 4.18 - 4.06 (m, 3H), 3.92 - 3.81 (m, 3H), 2.48 - 2.43 (m, 1H), 2.36 - 2.27 (m, 1H), 1.19 (t, *J* = 7.1 Hz, 3H).

Reference Example 52: Preparation of intermediate I-52

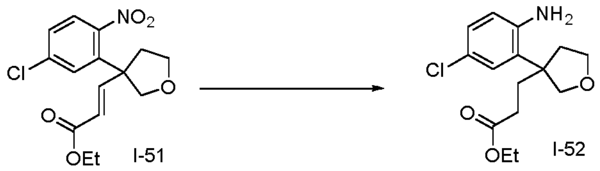

Platinum dioxide (50 mg) was added to a solution of intermediate I-51 (720 mg, 2.21 mmol) in tetrahydrofuran (50 mL) at room temperature. The reaction mixture was hydrogenated with a hydrogen balloon at room temperature for about 6 hours. Filtration and concentration of the filtrate under reduced pressure to remove the organic solvent gave the crude product. The crude product was purified by silica gel chromatography to afford intermediate I-52.

LC-MS (ESI) $[M+H]^+$ 298.1.

Reference Example 53: Preparation of intermediate I-53

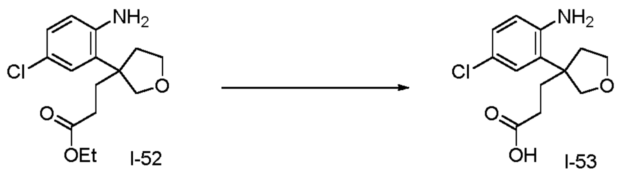

Sodium hydroxide (88.7 mg, 2.22 mmol) was added to a solution of intermediate I-52 (220 mg, 0.739 mmol) in tetrahydrofuran/water (10 mL/3 mL) at room temperature. The reaction solution was stirred at 40°C for 16 hours. After being cooled to room temperature, the reaction solution was adjusted to pH 7 with dilute hydrochloric acid (1 N), and the organic solvent was removed by concentration under reduced pressure to give crude intermediate I-53, which was used in the next step without purification.

LC-MS (ESI) $[M+H]^+$ 270.1.

Reference Example 54: Preparation of intermediate I-54

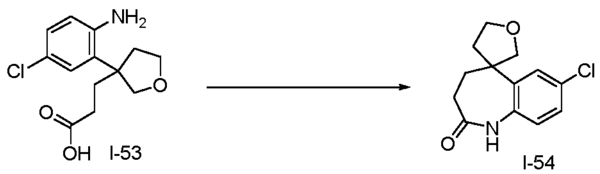

1-(3-Dimethylaminopropyl)-3-ethylcarbodiimide hydrochloride (283 mg, 1.48 mmol) and 4-dimethylamino pyridine (135 mg, 1.11 mmol) were added to a solution of intermediate I-53 (300 mg) in tetrahydrofuran (60 mL) at room temperature, and the reaction solution was allowed to react at room temperature for 16 hours. The reaction mixture was added water (60 mL) and extracted with ethyl acetate (50 mL×3). The organic phases were combined and washed with saturated brine (50 mL), dried over anhydrous sodium sulfate, and filtered, and the filtrate was concentrated under reduced pressure to remove the organic solvent to give the crude product. The crude product was purified by silica gel

chromatography to afford intermediate I-54.

LC-MS (ESI) $[M+H]^+$ 252.1.

$^1H$ NMR (400 MHz, DMSO-$d_6$) δ 9.57 (d, *J* = 30.6 Hz, 1H), 7.40 - 7.29 (m, 2H), 7.04 - 6.97 (m, 1H), 3.90 - 3.78 (m, 3H), 3.73 - 3.65 (m, 1H), 2.31 - 2.05 (m, 6H).

Reference Example 55: Preparation of intermediate I-55

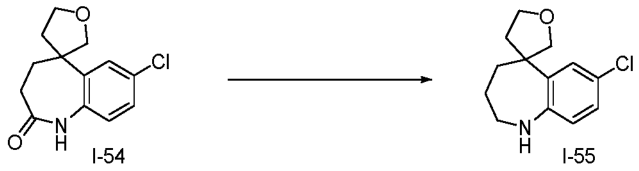

I-54 I-55

A borane-tetrahydrofuran solution (2.09 mL, 1 M) was added to a solution of intermediate I-54 (105 mg, 0.417 mmol) in tetrahydrofuran (20 mL) at room temperature, and the reaction solution was reacted at 40°C for 2 hours. The reaction solution was cooled to room temperature, diluted with water (20 mL), and extracted with ethyl acetate (20 mL×2). The organic phases were combined and washed with saturated brine (20 mL), dried over anhydrous sodium sulfate, and filtered, and the filtrate was concentrated under reduced pressure to remove the organic solvent to give the crude product. The crude product was purified by silica gel chromatography to afford intermediate I-55.

LC-MS (ESI) $[M+H]^+$ 238.1.

$^1H$ NMR (400 MHz, $CDCl_3$) δ 7.21 (d, *J* = 2.3 Hz, 1H), 7.00 (dd, *J* = 8.3, 2.4 Hz, 1H), 6.65 (d, *J* = 8.3 Hz, 1H), 4.10 (d, *J* = 8.8 Hz, 1H), 3.97 - 3.89 (m, 2H), 3.81 - 3.74 (m, 1H), 3.14 - 3.05 (m, 1H), 3.00 - 2.93 (m, 1H), 2.40 - 2.32 (m, 1H), 2.18 - 2.09 (m, 1H), 1.89 - 1.83 (m, 2H), 1.75 - 1.69 (m, 2H).

Reference Example 56: Preparation of intermediate I-56

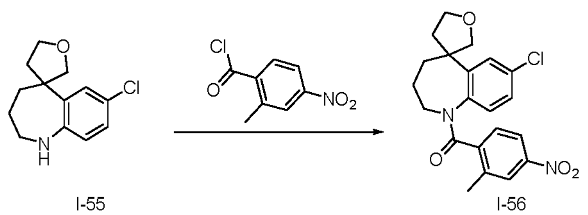

I-55 I-56

2-Methyl-4-nitrobenzoyl chloride (42.4 mg, 0.212 mmol) was added to a solution of intermediate I-55 (42 mg, 0.177 mmol) and pyridine (42.0 mg, 0.531 mmol) in tetrahydrofuran (5 mL) at room temperature. The reaction solution was reacted at 50°C for 5 hours. The reaction solution was cooled to room temperature, diluted with water (15 mL), and extracted with ethyl acetate (10 mL×2). The organic phases were combined and washed with saturated brine (20 mL), dried over anhydrous sodium sulfate, and filtered, and the filtrate was concentrated under reduced pressure to give the crude product. The crude product was purified by silica gel chromatography to afford intermediate I-56.

LC-MS (ESI) $[M+H]^+$ 401.0.

Reference Example 57: Preparation of intermediate I-57

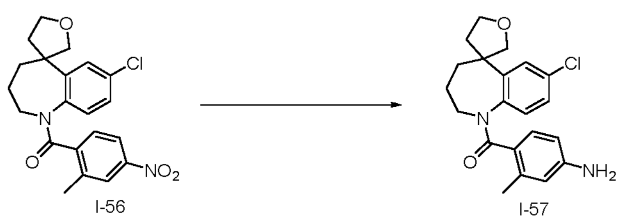

I-56 I-57

Iron powder (31.3 mg, 0.560 mmol) and ammonium chloride (59.9 mg, 1.12 mmol) were added to a solution of intermediate I-56 (45 mg, 0.112 mmol) in methanol/water (10 mL/3 mL) at room temperature. The reaction solution was kept for reaction under argon at 50°C for 2 hours. The reaction solution was cooled to room temperature and filtered, and the filtrate was concentrated to remove most of the solvent, diluted with water (5 mL), and extracted with ethyl acetate (10 mL×3). The organic phases were combined and washed with saturated brine (20 mL), dried over anhydrous sodium sulfate, and filtered, and the filtrate was concentrated under reduced pressure to give the crude product. The crude product was purified by silica gel chromatography to afford intermediate I-57.

LC-MS (ESI) $[M+H]^+$ 371.0.

Reference Example 58: Preparation of intermediate I-58

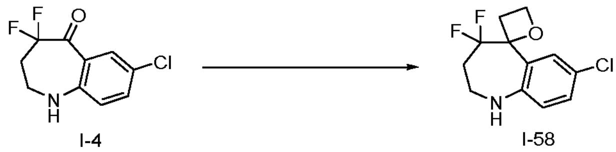

Trimethylsulfoxonium iodide (370 mg, 1.68 mmol) and potassium tert-butoxide (189 mg, 1.68 mmol) were added to dimethylsulfoxide (6.00 mL) at room temperature. After stirring under argon for 30 minutes, intermediate I-4 (130 mg, 0.561 mmol) was added at room temperature and the reaction mixture was stirred at room temperature for 3 hours. Water (20 mL) was added and extraction was made with ethyl acetate (10 mL×3). The organic phases were combined and washed with brine (50 mL), dried over anhydrous sodium sulfate, and filtered, and concentration was performed under reduced pressure to give the crude product. The crude product was purified by silica gel chromatography to afford intermediate I-58.

LCMS (ESI) $[M+H]^+$ 260.0.

$^1$HNMR (400 MHz, DMSO-$d_6$) δ 7.57 (d, *J* = 2.6 Hz, 1H), 7.17 (dd, *J* = 8.4, 2.6 Hz, 1H), 6.86 (d, *J* = 8.4 Hz, 1H), 5.65 (d, *J* = 4.9 Hz, 1H), 4.57 - 4.40 (m, 2H), 3.22 - 3.05 (m, 2H), 2.73 (t, *J* = 12.4 Hz, 1H), 2.41 - 2.32 (m, 1H), 2.20 - 2.04 (m, 1H).

Reference Example 59: Preparation of intermediate I-59

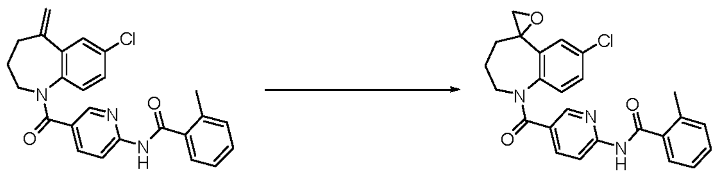

N-Bromosuccinimide (395 mg, 2.22 mmol) was added to a solution of intermediate I-21 (800 mg, 1.85 mmol) in tetrahydrofuran/water (15 mL/3 mL) at room temperature and the reaction solution was stirred at room temperature for 24 hours. Then, a 10% aqueous sodium hydroxide solution (3.70 mL, 9.25 mmol) was added. The reaction solution was stirred at room temperature for an additional hour. The reaction solution was extracted with ethyl acetate (15 mL×3). The organic phases were combined and washed with saturated brine (30 mL), dried over anhydrous sodium sulfate, and filtered, and the filtrate was concentrated under reduced pressure to remove the organic solvent to give the crude product. The crude was purified by C18 reverse phase column to afford intermediate I-59.

LC-MS (ESI) $[M+H]^+$ 448.1.

$^1$H NMR (400 MHz, DMSO-$d_6$) δ 10.87 (m, 2H), 8.20 - 6.90 (m, 10H), 4.94 - 4.81 (m, 1H), 3.13 - 2.78 (m, 3H), 2.35 (s, 3H), 2.15 - 1.93 (m, 2H), 1.66 (m, 2H).

Reference Example 60: Preparation of intermediate 1-60

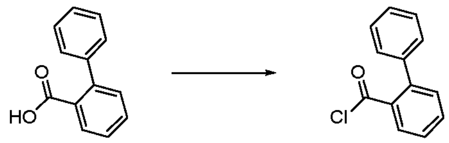

I-60

2-Phenylbenzoic acid (14.0 g, 70.6 mmol) was dissolved in dichloromethane (200 mL) at room temperature, thionyl chloride (16.8 g, 141 mmol) was added to the system and the reaction mixture was stirred at room temperature for 3 hours. The reaction solution was concentrated under reduced pressure to afford crude intermediate I-60, which was used directly in the next step.

Reference Example 61: Preparation of intermediate I-61

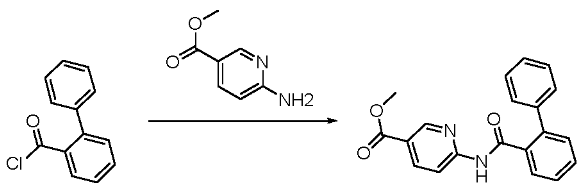

I-60 I-61

Intermediate I-60 (10.0 g) was dissolved in pyridine (80.0 mL) at room temperature, methyl 6-aminonicotinate (7.03 g, 46.2 mmol) was added and the reaction mixture was stirred at room temperature for 3 hours. The reaction system was poured into water (500 mL), and extracted with ethyl acetate (100 mL×3), and the organic phases were combined and washed with saturated brine (100 mL), dried over anhydrous sodium sulfate, and filtered, while the filtrate was concentrated under reduced pressure to give the crude product. The crude product was purified by C18 reverse phase column to afford intermediate I-61.

LC-MS (ESI) $[M+H]^+$ 333.0.

Reference Example 62: Preparation of intermediate I-62

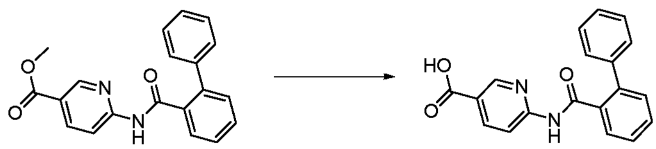

I-61 I-62

Intermediate I-61 (10.0 g, 30.1 mmol) was dissolved in methanol/water (100 mL/20 mL) at room temperature, sodium hydroxide (3.61 g, 90.3 mmol) was added to the system and the reaction mixture was stirred at room temperature for 3 hours. After adjusting pH=6-7 with dilute hydrochloric acid (1.0 mol/L), a large amount of solid precipitated out. The solid was filtered, collected, and dried under reduced pressure to give intermediate I-62.

LC-MS (ESI) $[M+H]^+$ 319.0.

Reference Example 63: Preparation of intermediate I-63

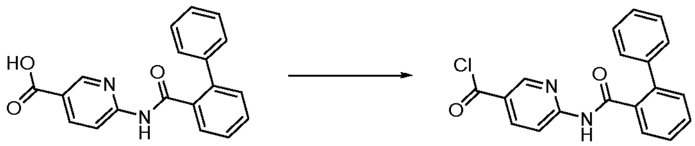

I-62 I-63

Intermediate I-62 (2.00 g, 6.28 mmol) was dissolved in dichloromethane (30.0 mL) at room temperature, thionyl chloride (2.24 g, 18.8 mmol) was added and the reaction mixture was stirred at 50°C for 3 hours. The reaction solution was concentrated under reduced pressure to afford crude intermediate I-63, which was used directly in the next step.

Reference Example 64: Preparation of intermediate I-64

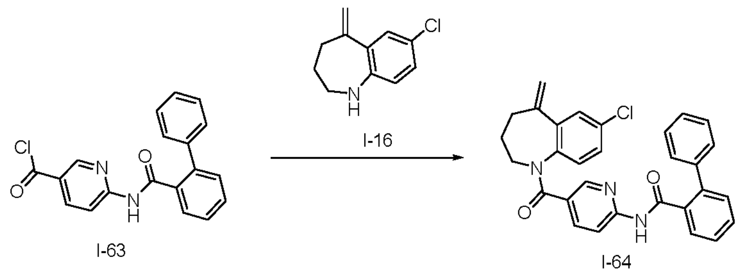

I-16

I-63 I-64

Intermediate I-16 (633 mg, 3.27 mmol) was dissolved in pyridine (10.0 mL) at room temperature, intermediate I-63 (1.10 g) was added and the reaction mixture was stirred at room temperature for 3 hours. The system was poured into water (50.0 mL), and extracted with ethyl acetate (20 mL×3), and the organic phases were combined and washed with saturated brine (20 mL), dried over anhydrous sodium sulfate, and filtered, while the filtrate was concentrated under reduced pressure to give the crude product. The crude product was purified by silica gel chromatography to afford intermediate I-64.

LC-MS (ESI) $[M+H]^+$ 494.2.

Reference Example 65: Preparation of intermediate I-65

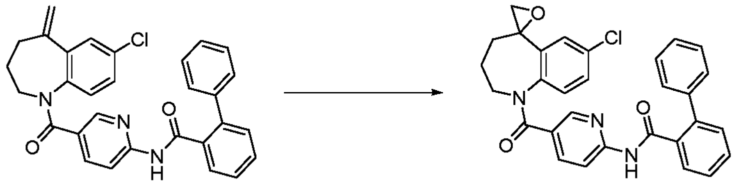

I-64 I-65

Intermediate I-64 (500 mg, 1.01 mmol) was dissolved in tetrahydrofuran/water (25 mL/5 mL) at room temperature, N-bromosuccinimide (360 mg, 2.02 mmol) was added and the reaction mixture was stirred at room temperature for 24 hours. The reaction system was added with a 25% aqueous sodium hydroxide solution (1.00 mL) and the system was stirred at room temperature for 1 hour. The reaction system was poured into water (20 mL), and extracted with ethyl acetate (10 mL×3), and the organic phases were combined and washed with saturated brine (15 mL), dried over anhydrous sodium sulfate and filtered, while the filtrate was concentrated under reduced pressure to give the crude product. The crude product was purified by silica gel chromatography to afford intermediate I-65.

LC-MS (ESI) $[M+H]^+$ 510.1.

Reference Example 66: Preparation of intermediate I-66

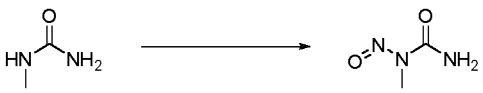

I-66

Under an ice-water bath, N-methylurea (5.0 g, 67.5 mmol) was dissolved in water (40 mL), and then sodium nitrite (5.12 g, 74.2 mmol) was added thereto. Concentrated hydrochloric acid (8.9 mL) was slowly added dropwise with stirring in an ice-water bath for about 30 minutes and reacted for 30 minutes in an ice-water bath. The precipitate was filtered, and the filter cake was washed with water and dried by an oil pump to afford intermediate I-66, which was used in the next reaction without further purification.

Reference Example 67: Preparation of intermediate I-67

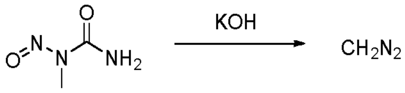


I-66 I-67

Under an ice-water bath, a 40% aqueous potassium hydroxide solution (40 mL) and diethyl ether (54 mL) were added to a smooth reaction flask, and intermediate I-66 (4.2 g, 40.7 mmol) was added in portions to the above system. The reaction flask was gently shaken several times, and after 30 minutes of reaction, the upper ether solution was transferred to another smooth flask containing solid potassium hydroxide for drying. Intermediate I-67 was obtained as a solution in diethyl ether (0.75 M) and used directly in the next step.

Reference Example 68: Preparation of intermediate I-68

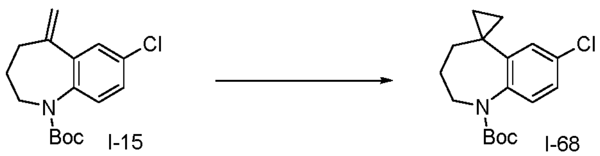

I-15 I-68

A solution of intermediate I-67 in diethyl ether (40 mL, 0.75 M) was slowly added to a solution of intermediate I-15 (300 mg, 1.02 mmol) and palladium acetate (50 mg) in diethyl ether (10 mL) under an ice-water bath and stirred for 1 h under the ice-water bath. The reaction solution was quenched with acetic acid (1 mL), diluted with water (30 mL), and extracted with ether (20 mL×3). The organic phases were combined and washed with brine (50 mL), dried over anhydrous sodium sulfate, and filtered. The filtrate was concentrated under reduced pressure to remove the organic solvent to give the crude product. The crude product was purified by silica gel chromatography to afford intermediate I-68.

LC-MS (ESI) $[M+H-56]^+$ 252.1.

Reference Example 69: Preparation of intermediate I-69

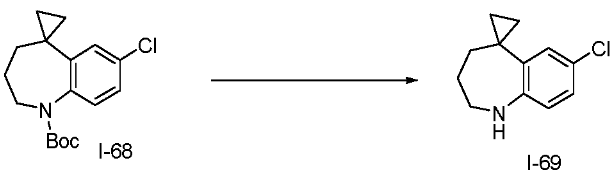

I-68 I-69

Trifluoroacetic acid (1 mL) was added to a solution of intermediate I-68 (90 mg, 0.292 mmol) in dichloromethane

(5 mL) at room temperature. The reaction solution was stirred at room temperature for 5 hours. The reaction solution was concentrated to dryness under reduced pressure to give crude intermediate I-69, which is used directly in the next step.

LC-MS (ESI) $[M+H]^+$ 208.1.

Reference Example 70: Preparation of intermediate I-70

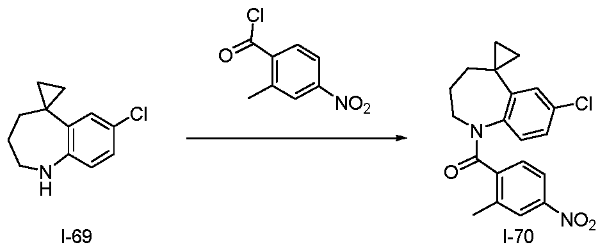

I-69 I-70

2-Methyl-4-nitrobenzoyl chloride (115 mg, 0.576 mmol) was added to a solution of intermediate I-69 (60 mg) and pyridine (68.6 mg, 0.867 mmol) in 1,2-dichloroethane (10 mL) at room temperature and the reaction solution was stirred at 50°C for 16 hours. The reaction solution was cooled to room temperature, diluted with dichloromethane (20 mL), washed with saturated brine (20 mL), dried over anhydrous sodium sulfate, and filtered. The filtrate was concentrated under reduced pressure to remove the organic solvent to give the crude product. The crude product was purified by silica gel chromatography to afford intermediate I-70.

LC-MS (ESI) $[M+H]^+$ 371.1.

Reference Example 71: Preparation of intermediate I-71

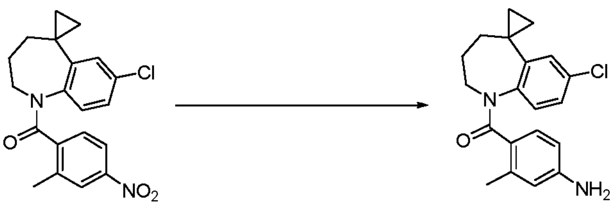

I-70 I-71

Iron powder (30.2 mg, 0.541 mmol) and ammonium chloride (57.8 mg, 1.08 mmol) were added to a solution of intermediate I-70 (40 mg, 0.108 mmol) in methanol/water (10 mL/3 mL) at room temperature, and the reaction solution was stirred at 50°C for 3 hours under argon. The reaction solution was cooled to room temperature, filtered and the filtrate was concentrated to dryness to give the crude product. The crude product was purified by silica gel chromatography to afford intermediate I-71.

LC-MS (ESI) $[M+H]^+$ 341.1.

Reference Example 72: Preparation of intermediate I-72

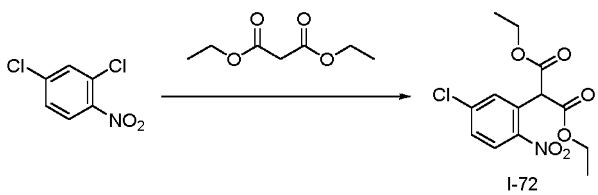

I-72

Diethyl malonate (10.0 g, 62.5 mmol) was dissolved in dimethyl sulfoxide (150 mL) at room temperature. Sodium hydride (2.92 g, 60% wt, 72.9 mmol) was added slowly. After the reaction mixture was stirred at room temperature for 30 minutes, 2,4-dichloronitrobenzene (10.0 g, 52.1 mmol) was added to the reaction solution. After the addition was completed, the reaction solution was heated to 80°C and stirred for 5 hours. Cooling was performed to room temperature, water was added (300 mL) and extraction was made with ethyl acetate (300 mL×3). The organic phases were combined and washed with saturated brine (300 mL), dried over anhydrous sodium sulfate, and filtered, and the filtrate was concentrated under reduced pressure to remove the organic solvent to give the crude product. The crude product was purified by silica gel chromatography to afford intermediate I-72.
LC-MS (ESI) $[M+H]^+$316.0.

Reference Example 73: Preparation of intermediate I-73

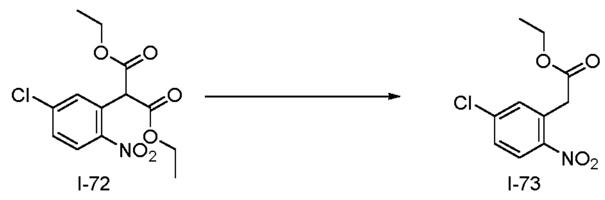

I-72 I-73

Intermediate I-72 (10.0 g, 31.68 mmol) was dissolved in dimethyl sulfoxide (100 mL) and water (1 mL) at room temperature, lithium chloride (6.65 g, 158.4 mmol) was added and after the addition was complete, heated to 100°C and stirred for 5 hours. The reaction solution was cooled to room temperature, diluted with water (200 mL), and extracted with ethyl acetate (200 mL×3). The organic phases were combined and washed with saturated brine (200 mL), dried over anhydrous sodium sulfate, and filtered, and the filtrate was concentrated under reduced pressure to remove the organic solvent to give the crude product. The crude product was purified by silica gel chromatography to afford intermediate I-73.
$^1H$ NMR (400 MHz, $CDCl_3$) δ 8.08 (d, *J* = 8.8 Hz, 1H), 7.44 (dd, *J* = 8.8, 2.3 Hz, 1H), 7.35 (d, *J* = 2.2 Hz, 1H), 4.17 (q, *J* = 7.1 Hz, 2H), 3.99 (s, 2H), 1.25 (t, *J* = 7.1 Hz, 3H).

Reference Example 74: Preparation of intermediate I-74

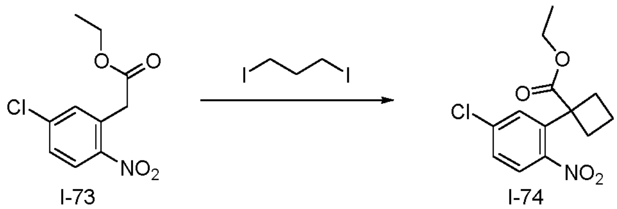

I-73 I-74

Intermediate I-73 (3.5 g, 14.4 mmol) was dissolved in N,N-dimethylformamide (100 mL) and added slowly with a solution of sodium hydride (860 mg, 60% wt, 21.5 mmol) in N,N-dimethylformamide (10 mL) at 0°C and the reaction mixture was stirred for 30 minutes. 1,3-Diiodopropane (5.1 g, 17.2 mmol) was added to the reaction solution, and after the addition was completed, the reaction solution was stirred at room temperature overnight. Water (200 mL) was added for dilution and extraction was performed with ethyl acetate (200 mL×3). The organic phases were combined and washed with saturated brine (200 mL), dried over anhydrous sodium sulfate, and filtered, and the filtrate was concentrated under reduced pressure to remove the organic solvent to give the crude product. The crude product was purified by silica gel chromatography to afford intermediate I-74.
$^1H$ NMR (400 MHz, $CDCl_3$) δ 7.88 (d, *J* = 8.6 Hz, 1H), 7.44 (d, *J* = 2.2 Hz, 1H), 7.37 (dd, *J* = 8.6, 2.2 Hz, 1H), 4.20 (q, *J* = 7.1 Hz, 2H), 2.85 - 2.76 (m, 2H), 2.46 - 2.31 (m, 3H), 1.92 - 1.80 (m, 1H), 1.22 (t, *J* = 7.1 Hz, 3H).

Reference Example 75: Preparation of intermediate I-75

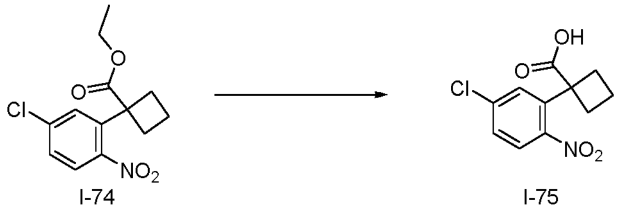

I-74 I-75

Intermediate I-74 (1.3 g, 4.58 mmol) was dissolved in methanol (10 mL) and water (10 mL) at room temperature, and sodium hydroxide (916 mg, 22.9 mmol) was added and heated to 100°C with stirring for 3 hours. After being cooled to room temperature, the reaction solution was concentrated to remove most of methanol, then made acidic with 1 N hydrochloric acid and extracted with ethyl acetate (50 mL×3). The organic phases were combined and washed with brine (50 mL), dried over anhydrous sodium sulfate, and filtered, and the filtrate was concentrated under reduced pressure to remove the organic solvent to afford intermediate I-75.

$^1$H NMR (400 MHz, DMSO-$d_6$) $\delta$ 12.52 (s, 1H), 7.97 (d, *J* = 8.6 Hz, 1H), 7.65 - 7.55 (m, 2H), 2.67 - 2.58 (m, 2H), 2.39 (m, 2H), 2.17 (dt, *J* = 19.4, 8.9 Hz, 1H), 1.85 - 1.72 (m, 1H).

Reference Example 76: Preparation of intermediate I-76

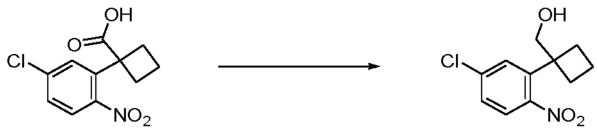

I-75 I-76

A borane-tetrahydrofuran solution (19.5 mL, 1 M) was added to a solution of intermediate I-75 (1.0 g, 3.91 mmol) in tetrahydrofuran (50 mL) at room temperature and the reaction solution was stirred at room temperature overnight. The reaction solution was diluted with water (50 mL) and extracted with ethyl acetate (50 mL×2). The organic phases were combined and washed with saturated brine (20 mL), dried over anhydrous sodium sulfate, and filtered, and the filtrate was concentrated under reduced pressure to remove the organic solvent to give the crude product. The crude was purified by silica gel chromatography to afford intermediate I-76.

Reference Example 77: Preparation of intermediate I-77

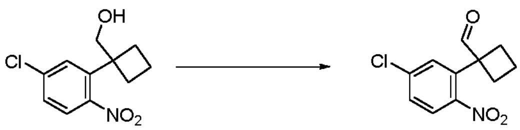

I-76 I-77

Intermediate I-76 (800 mg, 3.31 mmol) was dissolved in dichloromethane (10 mL) and Dess-Martin periodinane (4.21 g, 9.93 mmol) was added at room temperature. After the addition was complete, the reaction solution was stirred at room temperature for 2 hours. The reaction solution was filtered and the filtrate was diluted with water (20 mL) and extracted with dichloromethane (20 mL×3). The organic phases were combined and washed with saturated brine (20 mL), dried over anhydrous sodium sulfate, and filtered, and the filtrate was concentrated under reduced pressure to remove the organic solvent to give the crude product. The crude product was purified by silica gel chromatography to afford intermediate I-77.

Reference Example 78: Preparation of intermediate I-78

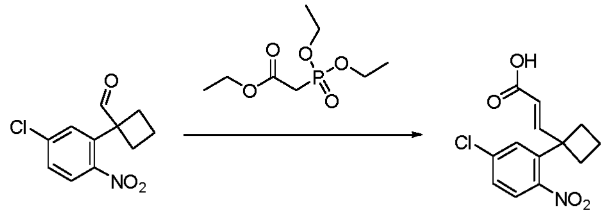

I-77 I-78

Sodium hydride (157 mg, 60% wt, 3.92 mmol) was added to triethyl phosphonoacetate (659 mg, 2.94 mmol) in tetrahydrofuran (30 mL) at 0°C under argon, stirred under an ice-water bath for 30 minutes, then Intermediate I-77 (470 mg, 1.96 mmol) was added. The reaction mixture was allowed to react at room temperature for 2 hours. The reaction solution was concentrated to remove most of tetrahydrofuran, added water (50 mL), and extracted with ethyl acetate (50 mL×3). The aqueous phase was made acidic with 1 N hydrochloric acid and extracted with ethyl acetate (50 mL×3). The organic phases were combined and washed with brine (50 mL), dried over anhydrous sodium sulfate, and filtered, and the filtrate was concentrated under reduced pressure to remove the organic solvent to afford intermediate I-78.

$^1$H NMR (400 MHz, DMSO-$d_6$) $\delta$ 12.22 (s, 1H), 7.88 (d, *J* = 8.7 Hz, 1H), 7.59 (dd, *J* = 8.7, 2.3 Hz, 1H), 7.47 (d, *J* = 2.3 Hz, 1H), 7.30 (d, *J* = 15.7 Hz, 1H), 5.65 (d, *J* = 15.7 Hz, 1H), 2.43 - 2.33 (m, 4H), 2.05 (dd, *J* = 20.5, 10.1 Hz, 1H), 1.79 - 1.70 (m, 1H).

Reference Example 79: Preparation of intermediate I-79

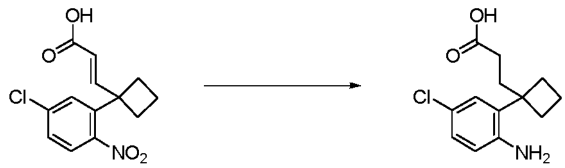

I-78 I-79

Intermediate I-78 (360 mg, 1.28 mmol) was dissolved in tetrahydrofuran (20 mL) and Raney nickel (300 mg) was added at room temperature. After complete addition, the reaction solution was stirred under an atmosphere of hydrogen for 16 hours. The organic solvent was removed by filtration and the filtrate was concentrated under reduced pressure to give crude intermediate I-79. The crude product was used directly in the next reaction.

LC-MS (ESI) $[M+H]^+$ 254.3.

Reference Example 80: Preparation of intermediate I-80

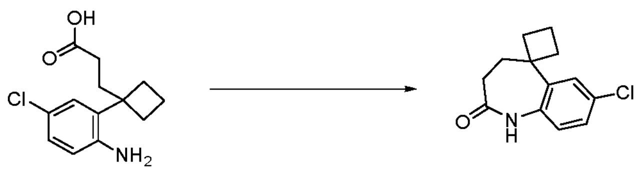

I-79 I-80

1-(3-Dimethylaminopropyl)-3-ethylcarbodiimide hydrochloride (331 mg, 1.73 mmol) and 4-dimethylamino pyridine (211 mg, 1.73 mmol) were added to a solution of intermediate I-79 (220 mg) in tetrahydrofuran (60 mL) at room temperature, and the reaction solution was stirred at room temperature for 16 hours. The reaction mixture was added water (50 mL) and extracted with ethyl acetate (50 mL×3). The organic phases were combined and washed with saturated brine (50 mL), dried over anhydrous sodium sulfate, and filtered, and the filtrate was concentrated under reduced pressure to remove the organic solvent to give the crude product. The crude product was purified by silica gel chromatography to afford intermediate I-80.

LC-MS (ESI) $[M+H]^+$ 236.2.

Reference Example 81: Preparation of intermediate I-81

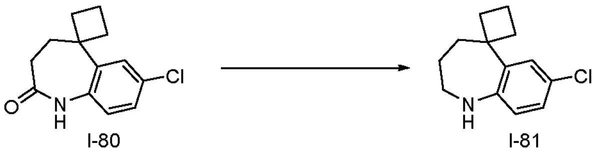

I-80 I-81

A borane-tetrahydrofuran solution (1.9 mL, 1 M) was added to intermediate I-80 (90 mg, 0.382 mmol) in tetrahydrofuran (20 mL) at room temperature. The reaction solution was reacted at 40°C for 2 hours. The reaction solution was cooled to room temperature, diluted with water (20 mL), and extracted with ethyl acetate (20 mL×2). The organic phases were combined and washed with saturated brine (20 mL), dried over anhydrous sodium sulfate, and filtered, and the filtrate was concentrated under reduced pressure to remove the organic solvent to give the crude product. The crude was purified by silica gel chromatography to afford intermediate I-81.

LC-MS (ESI) $[M+H]^+$ 222.2.

Reference Example 82: Preparation of intermediate I-82

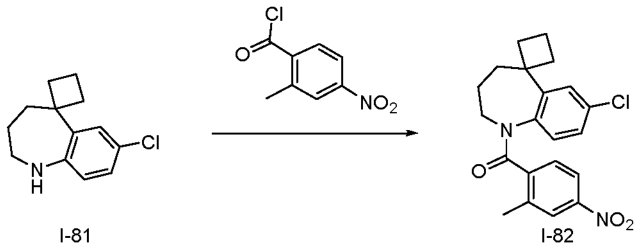

I-81 I-82

2-Methyl-4-nitrobenzoyl chloride (94.8 mg, 0.475 mmol) was added to a solution of intermediate I-81 (70 mg, 0.316 mmol) and pyridine (75.0 mg, 0.948 mmol) in tetrahydrofuran (5 mL) at room temperature. The reaction solution was stirred at 50°C for 5 hours. The reaction solution was cooled to room temperature, diluted with water (15 mL), and extracted with ethyl acetate (10 mL×2). The organic phases were combined and washed with saturated brine (20 mL), dried over anhydrous sodium sulfate, and filtered, and the filtrate was concentrated under reduced pressure to give the crude product. The crude was purified by silica gel chromatography to afford intermediate I-82.

LC-MS (ESI) $[M+H]^+$ 385.2.

Reference Example 83: Preparation of intermediate I-83

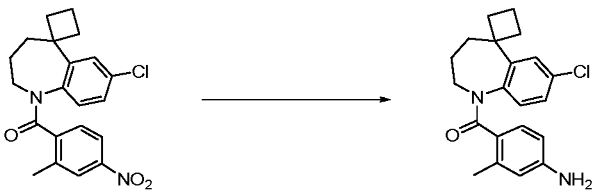

I-82 I-83

Iron powder (39.2 mg, 0.700 mmol) and ammonium chloride (74.9 mg, 1.40 mmol) were added to a solution of intermediate I-82 (54 mg, 0.140 mmol) in methanol/water (10 mL/3 mL) at room temperature. The reaction solution was stirred at 50°C for 2 hours under argon. The reaction solution was cooled to room temperature and filtered, and the filtrate was concentrated to remove most of the solvent, diluted with water (5 mL), and extracted with ethyl acetate (10 mL×3). The organic phases were combined and washed with saturated brine (20 mL), dried over anhydrous sodium sulfate, and filtered, and the filtrate was concentrated under reduced pressure to give the crude product. The crude product was purified by silica gel chromatography to afford intermediate I-83.

LC-MS (ESI) $[M+H]^+$ 355.1.

Reference Example 84: Preparation of intermediate I-84

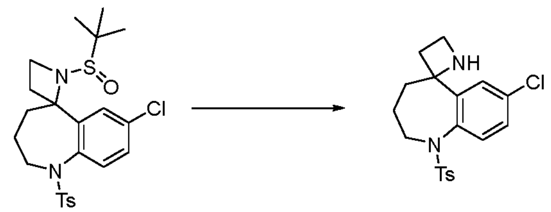

I-42 I-84

Intermediate I-42 (880.00 mg, 1.83 mmol) was dissolved in concentrated hydrochloric acid (5 mL) at 25°C and stirred at 80°C for 16 hours. The solution was directly concentrated to dryness, added with ammonia in methanol (1 mL, 2 M) to basify, and concentrated under reduced pressure to obtain the crude product. The crude product was purified by silica gel chromatography to afford intermediate I-84.

LC-MS (ESI) $[M+H]^+$ 377.2.

Reference Example 85: Preparation of intermediate I-85

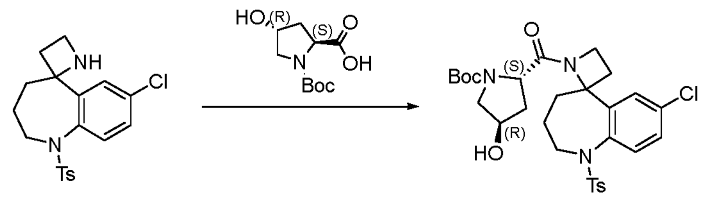

I-84 I-85

Intermediate I-85 (670.00 mg, 1.78 mmol) was dissolved in dichloromethane (10 mL), Boc-L-hydroxyproline (493.30 mg, 2.13 mmol), N,N-diisopropylethyl amine (683.57 mg, 5.33 mmol) and 2-(7-azabenzotriazole)-N,N,N',N'-tetramethyluronium hexafluorophosphate (809.91 mg, 2.13 mmol) were added at 25°C and stirred at room temperature for 1 hour. Water (20 mL) was added and extraction was performed with dichloromethane (20 mL×3). The organic phases were combined and washed with saturated brine (20 mL), dried over anhydrous sodium sulfate, and filtered, and the filtrate was concentrated under reduced pressure to give the crude product. The crude product was purified by silica gel chromatography to afford intermediate I-85.

LC-MS (ESI) $[M+H]^+$ 590.2.

Reference Example 86: Preparation of intermediates I-86A, 86B

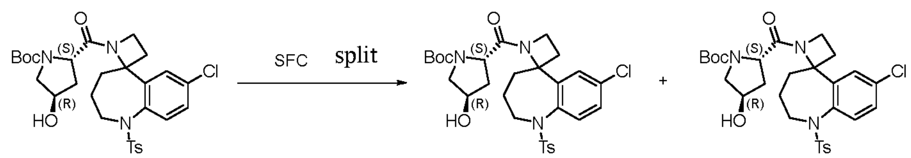


I-85 I-86A I-86B

Intermediate I-85 (1.22 g) was prepared twice on an SFC chiral separation column (ChiralPakAD, 250×30 mm I.D., 10 μm and SS whelk O1, 250×30 mm I.D., 10 μm) to afford chiral intermediate I-86A (Rt=4.726 min) and chiral intermediate I-86B (Rt=5.077 min).

Chiral analytical methods: chromatographic column: Chiralpak AD-3 150×4.6 mm I.D., 3 μM
Mobile phase: A: Supercritical carbon dioxide B: Ethanol (0.05% diethylamine)
Elution gradient: 5% to 40% B in 5 minutes; 40% B in 2.5 min; then 5% B for 2.5 minutes
Flow rate: 2.5 mL/min
Column temperature: 35°C
Automatic back pressure regulator (ABPR): 1500psi
Intermediate I-86A LC-MS (ESI) $[M+H]^+$ 590.4; intermediate I-86B LC-MS (ESI) $[M+H]^+$ 590.2.

Reference Example 87: Preparation of intermediate I-87

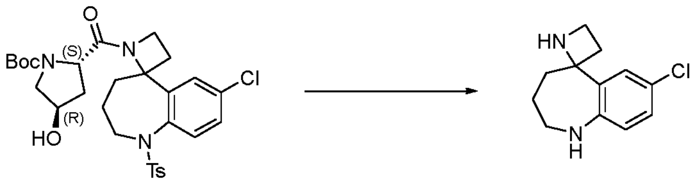

I-86A I-87 (chirality A)

Intermediate I-86A (200.00 mg, 0.34 mmol) was dissolved in concentrated hydrochloric acid (3 mL) at 25°C and stirred at 100°C for 16 hours. Direct concentration to remove the organic solvent afforded the crude chiral intermediate I-87. The crude product was used directly in the next reaction.
LC-MS (ESI) $[M+H]^+$ 223.0.

Reference Example 88: Preparation of intermediate I-88

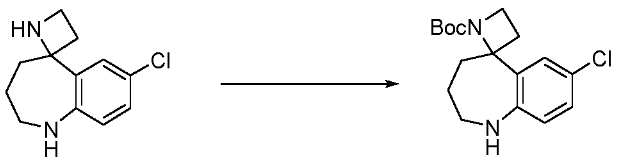

I-87 (chirality A) I-88 (chirality B)

Intermediate I-87 (400.00 mg) was dissolved in tetrahydrofuran (4 mL), a saturated aqueous sodium bicarbonate solution (2 mL), and di-tert-butyl dicarbonate (89.05 mg, 0.41 mmol) were added at 25°C and reacted at room temperature for 1 hour. Water (10 mL) was added and extraction was performed with ethyl acetate (10 mL×3). The organic phases were combined and washed with brine (10 mL), dried over anhydrous sodium sulfate, and filtered, and the filtrate was concentrated under reduced pressure to give the crude product. The crude product was purified by silica gel chromatography to afford intermediate I-88.
LC-MS (ESI) $[M+H]^+$ 323.0.

Reference Example 89: Preparation of intermediate I-89

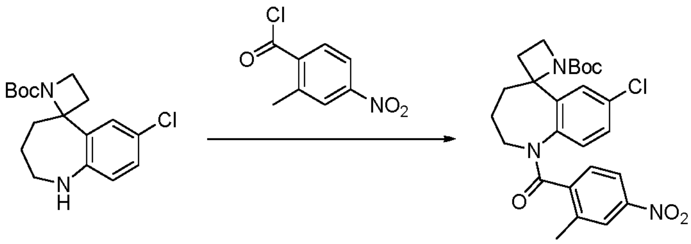

I-88 (chirality A)

I-89 (chirality A)

Intermediate I-88 (60.00 mg, 0.19 mmol) was dissolved in dichloromethane (5 mL), triethylamine (56.42 mg, 0.56 mmol), 2-methyl-4-nitrobenzoyl chloride (55.64 mg, 0.28 mmol) were added at 25°C and stirred at room temperature for 16 hours. Water (10 mL) was added and extraction was performed with dichloromethane (10 mL×3). The organic phases were combined and washed with saturated brine (10 mL), dried over anhydrous sodium sulfate, and filtered, and the filtrate was concentrated under reduced pressure to give the crude product. The crude product was purified by silica gel chromatography to afford intermediate I-89.

LC-MS (ESI) $[M-100+H]^+$ 386.0.

Reference Example 90: Preparation of intermediate I-90

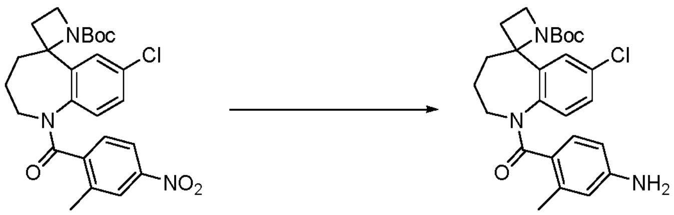

I-89 (chirality A)

I-90 (chirality A)

Intermediate I-89 (60.00 mg, 0.12 mmol) was dissolved in ethanol (8 mL), and reduced iron powder (34.47 mg, 0.62 mmol) and a saturated aqueous ammonium chloride solution (2 mL) were added at 25°C and stirred at 80°C for 5 hours. Filtration was performed, the filter cake was washed with ethyl acetate (20 mL), the filtrate was collected and water (10 mL) was added for stratification. The organic phase was collected, dried over anhydrous sodium sulfate, and filtered, and the filtrate was concentrated under reduced pressure to give the crude product. The crude product was purified by silica gel chromatography to afford intermediate I-90.

LC-MS (ESI) $[M-100+H]^+$ 356.4.

Reference Example 91: Preparation of intermediate I-91

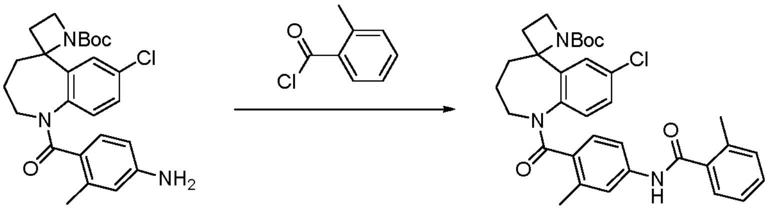

I-90 (chirality A)

I-91 (chirality A)

Intermediate I-90 (50.00 mg, 0.11 mmol) was dissolved in dichloromethane (5 mL), triethylamine (33.29 mg, 0.33 mmol), and o-methylbenzoyl chloride (25.43 mg, 0.16 mmol) were added successively at 25°C and stirred at room temperature for 1 hour. Water (10 mL) was added and extraction was performed with dichloromethane (10 mL×3). The

organic phases were combined and washed with saturated brine (10 mL), dried over anhydrous sodium sulfate, and filtered, and the filtrate was concentrated under reduced pressure to give the crude product. The crude product was purified by silica gel chromatography to afford intermediate I-91.

LC-MS (ESI) $[M-100+H]^+$ 474.0.

Reference Example 92: Preparation of intermediate I-92

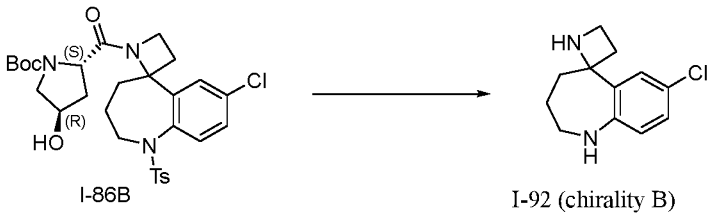

I-86B I-92 (chirality B)

Intermediate I-86B (750.00 mg, 1.27 mmol) was dissolved in concentrated hydrochloric acid (8 mL) at 25°C and stirred at 100°C for 16 hours. Direct concentration to remove the organic solvent afforded the crude chiral intermediate I-92. The crude product was used directly in the next reaction.

LC-MS (ESI) $[M+H]^+$ 223.0.

Reference Example 93: Preparation of intermediate I-93

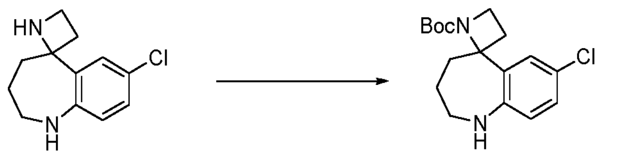

I-92 (chirality B) I-93 (chirality B)

Intermediate I-92 (1.40 g) was dissolved in tetrahydrofuran (20 mL), and a saturated aqueous sodium bicarbonate solution (10 mL) and di-tert-butyl dicarbonate (332.61 mg, 1.52 mmol) were added at 25°C and stirred at room temperature for 1 hour. Water (20 mL) was added and extraction was performed with ethyl acetate (20 mL×3). The organic phases were combined and washed with saturated brine (20 mL), dried over anhydrous sodium sulfate, and filtered, and the filtrate was concentrated under reduced pressure to give the crude product. The crude product was purified by silica gel chromatography to afford intermediate I-93.

LC-MS (ESI) $[M+H]^+$ 323.2.

Reference Example 94: Preparation of intermediate I-94

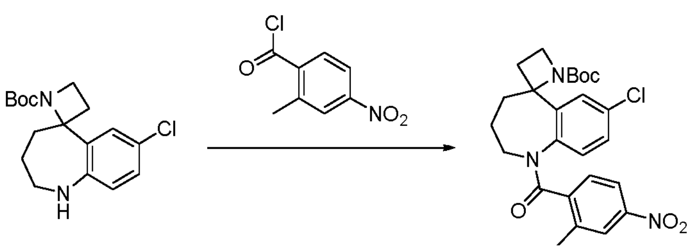

I-93 (chirality B) I-94 (chirality B)

Intermediate I-93 (70.00 mg, 0.22 mmol) was dissolved in dichloromethane (5 mL), and triethylamine (65.77

mg, 0.65 mmol) and 2-methyl-4-nitrobenzoyl chloride (64.92 mg, 0.33 mmol) were added at 25°C and stirred at room temperature for 16 hours. Water (10 mL) was added and extraction was performed with dichloromethane (10 mL×3). The organic phases were combined and washed with saturated brine (10 mL), dried over anhydrous sodium sulfate, and filtered, and the filtrate was concentrated under reduced pressure to give the crude product. The crude product was purified by silica gel chromatography to afford intermediate I-94.

LC-MS (ESI) $[M-100+H]^+$ 386.0.

Reference Example 95: Preparation of intermediate I-95

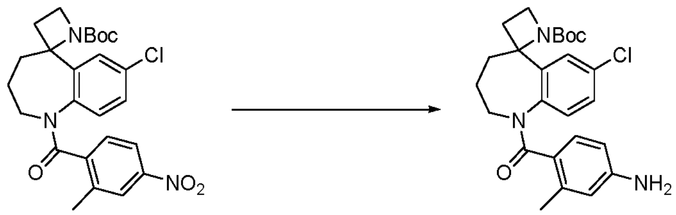

I-94 (chirality B) I-95 (chirality B)

Intermediate I-94 (90.00 mg, 0.19 mmol) was dissolved in ethanol (8 mL), and reduced iron powder (51.71 mg, 0.93 mmol) and a saturated aqueous ammonium chloride solution (2 mL) were added at 25°C and stirred at 80°C for 5 hours. Filtration was performed, the filter cake was washed with ethyl acetate (20 mL), the filtrate was collected and water (20 mL) was added for stratification. The organic phase was collected and concentrated under reduced pressure to give the crude product. The crude product was purified by silica gel chromatography to afford intermediate I-95.

LC-MS (ESI) $[M-100+H]^+$ 356.2.

Reference Example 96: Preparation of intermediate I-96

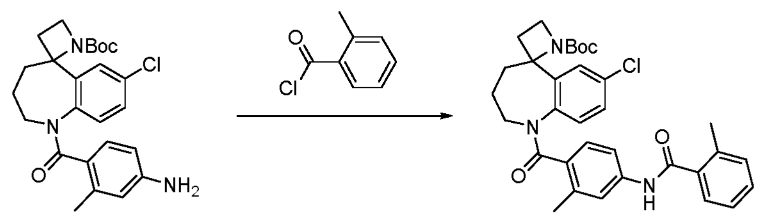

I-95 (chirality B) I-96 (chirality B)

Intermediate I-95 (70.00 mg, 0.15 mmol) was dissolved in dichloromethane (5 mL), and triethylamine (46.60 mg, 0.46 mmol) was added followed by o-methylbenzoyl chloride (35.60 mg, 0.23 mmol) at 25°C and stirred at room temperature for 1 hour. Water (10 mL) was added and extraction was performed with dichloromethane (10 mL×3). The organic phases were combined and washed with saturated brine (10 mL), dried over anhydrous sodium sulfate, and filtered, and the filtrate was concentrated under reduced pressure to give the crude product. The crude product was purified by silica gel chromatography to afford intermediate I-96.

LC-MS (ESI) $[M-100+H]^+$ 474.2.

Reference Example 97: Preparation of intermediate I-97

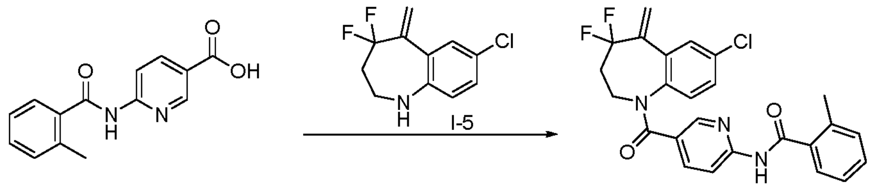

I-19 I-97

Under an ice-water bath, thionyl chloride (311 mg, 2.61 mmol) was added to N,N-dimethylacetamide (15 mL) of intermediate I-19 (335 mg, 1.31 mmol). The reaction mixture was stirred at room temperature for 3 hours, then Intermediate I-5 (150 mg, 0.653 mmol) was added, and the reaction mixture was stirred at room temperature for an additional 16 hours. Water (40 mL) was added and extraction was performed with ethyl acetate (20 mL×2). The organic phases were combined and washed with brine (30 mL), dried over anhydrous sodium sulfate, and filtered, and the filtrate was concentrated under reduced pressure to give the crude product. The crude product was purified by silica gel chromatography to afford intermediate I-97.

LCMS (ESI) $[M+H]^+$468.1.

Reference Example 98: Preparation of intermediate I-98

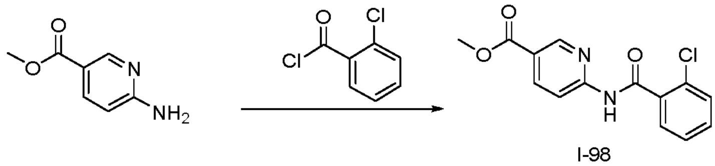

I-98

Chlorobenzoyl chloride (1.27 g, 7.23 mmol) was added to a solution of methyl 6-aminonicotinate (1.00 g, 6.57 mmol) in pyridine (20.0 mL) at room temperature. The reaction mixture was stirred at room temperature for 1 h, poured into water (100 mL), and filtered by suction, and the filter cake was dried to give intermediate I-98.

LC-MS (ESI) $[M+H]^+$ 290.9.

Reference Example 99: Preparation of intermediate I-99

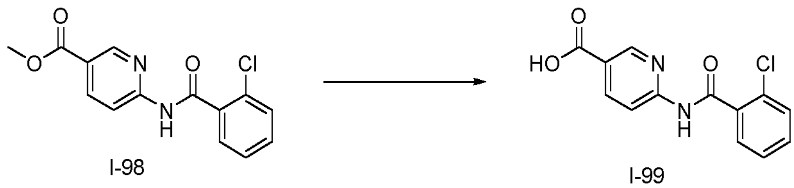

I-98 I-99

Aqueous sodium hydroxide solution (2 mL, 3.5 N) was added to a solution of intermediate I-98 (680 mg, 2.34 mmol) in tetrahydrofuran (10 mL) at room temperature and the reaction solution was stirred at 70°C for 1 hour. The reaction solution was adjusted to pH 5 with dilute hydrochloric acid (1 N), filtered and the filter cake was pumped to dryness to afford intermediate I-99.

LC-MS (ESI) $[M+H]^+$ 277.0.

Reference Example 100: Preparation of intermediate I-100

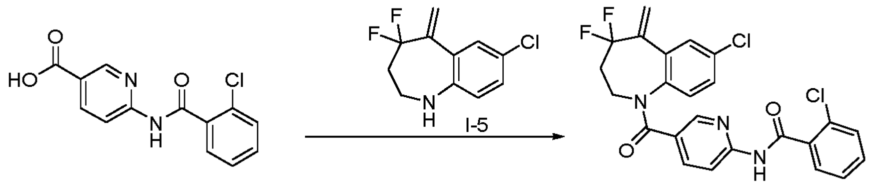

I-99 I-5 I-100

Under an ice-water bath, thionyl chloride (622 mg, 5.23 mmol) was added to N,N-dimethylacetamide (20 mL) of intermediate I-99 (800 mg, 2.89 mmol). The reaction mixture was stirred at room temperature for 3 hours, then Intermediate I-5 (300 mg, 1.31 mmol) was added and the reaction mixture was stirred at room temperature for an additional 16 hours. Water (40 mL) was added and extraction was performed with ethyl acetate (20 mL×2). The organic phases were combined and washed with brine (30 mL), dried over anhydrous sodium sulfate, and filtered, and the filtrate was concentrated under reduced pressure to give the crude product. The crude product was purified by silica gel chromatography to afford intermediate I-100.

LCMS (ESI) $[M+H]^+$487.9.

**Preparation of Examples:**

Example 1: Preparation of compound 1

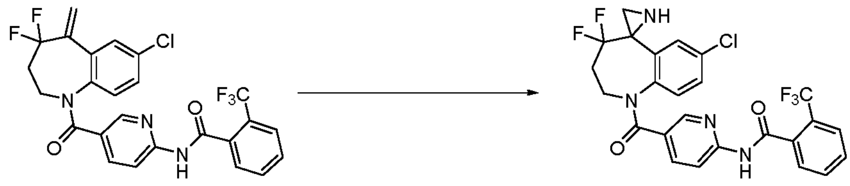

I-8

Compound 1

Intermediate I-8 (150 mg, 0.287 mmol) was added to a solution of aqueous ammonia (8.00 mL) at room temperature, and elemental iodine (728 mg, 2.87 mmol) and α-isotridecyl-ω-hydroxy-poly (oxy-1,2-ethanediyl) (1.00 mL) were added to the reaction system. The reaction mixture was stirred at room temperature for 24 hours, and a saturated aqueous sodium thiosulfate solution (50 mL) was added, and extracted with ethyl acetate (30 mL×3). The organic phases were combined and washed with saturated brine (50 mL), dried over anhydrous sodium sulfate, and filtered, and the filtrate was concentrated under reduced pressure to give the crude product. The crude product was purified by preparative HPLC (ammonia system) to give compound 1.

LC-MS (ESI) $[M+H]^+$ 537.0.

$^1$HNMR (400 MHz, DMSO-$d_6$) δ 11.25 - 11.15 (m, 1H), 8.55 - 8.13 (m, 1H), 8.10 - 7.48 (m, 7H), 7.27 (d, $J$ = 7.4 Hz, 1H), 7.12 - 6.86 (m, 1H), 5.09 - 4.71 (m, 1H), 3.19 - 2.94 (m, 1H), 2.88 - 2.54 (m, 2H), 2.45 - 2.16 (m, 2H).

Example 2: Preparation of compound 2

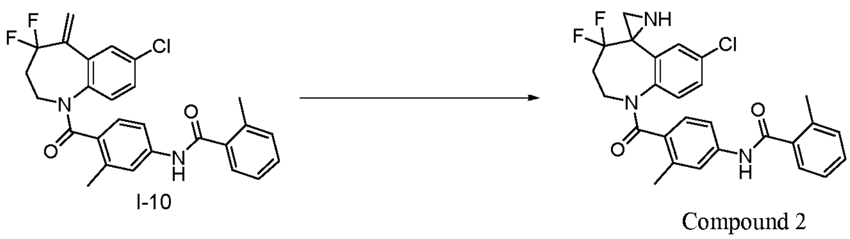

I-10

Compound 2

Intermediate I-10 (150 mg, 0.312 mmol) was added to a solution of aqueous ammonia (8.00 mL) at room

temperature, and elemental iodine (792 mg, 3.12 mmol) and α-isotridecyl-ω-hydroxy-poly (oxy-1,2-ethanediyl) (1.00 mL) were added to the reaction. The reaction mixture was stirred at room temperature for 24 hours. A saturated aqueous sodium thiosulfate solution (50 mL) was added and the mixture was extracted with ethyl acetate (30 mL×3). The organic phases were combined and washed with saturated brine (50 mL), dried over anhydrous sodium sulfate, and filtered, and the filtrate was concentrated under reduced pressure to give the crude product. The crude product was purified by preparative HPLC (ammonia system) to give compound 2.

LC-MS (ESI) $[M+H]^+$ 496.0.

$^1$HNMR (400 MHz, DMSO-$d_6$) δ 10.16 - 10.48 (m, 1H), 7.55 - 7.80 (m, 2H), 7.04 - 7.50 (m, 7H), 6.59 - 6.82 (m, 1H), 4.52 - 4.98 (m, 1H), 3.54 - 3.74 (m, 1H), 3.34 - 3.49 (m, 1H), 2.96 - 3.12 (m, 1H), 2.72 - 2.84 (m, 1H), 2.13 - 2.45 (m, 8H).

Example 3: Preparation of compound 3

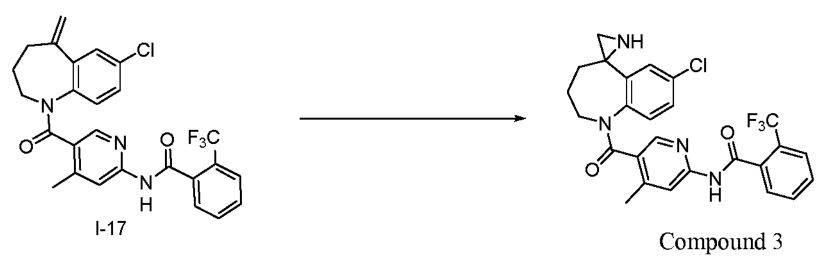

I-17

Compound 3

Intermediate I-17 (40 mg, 0.080 mmol) was added to aqueous ammonia (5 mL) at room temperature, followed by iodine (101.8 mg, 0.800 mmol) and α-isotridecyl-ω-hydroxy-poly (oxy-1,2-ethanediyl) (0.1 mL). After the addition was complete, the reaction mixture was stirred at room temperature overnight. The reaction mixture was diluted with a saturated aqueous sodium sulfite solution (10 mL), and extracted with ethyl acetate (10 mL× 3), and the organic phases were combined and washed with water (10 mL× 3), dried over anhydrous sodium sulfate and filtered, while the filtrate was concentrated under reduced pressure, and the residue was separated by preparative HPLC (aqueous ammonia system) to give compound 3.

LC-MS (ESI) $[M+H]^+$ 515.0.

$^1$H NMR (400 MHz, DMSO-$d_6$) δ 11.28-10.84 (m, 1H), 8.64-6.69 (m, 9H), 5.00-4.67 (m, 1H), 2.78 (dt, *J* = 43.7, 22.6 Hz, 1H), 2.47 (s, 3H), 2.22-1.74 (m, 5H), 1.54 (dd, *J* = 52.0, 31.2 Hz, 2H).

Example 4: Preparation of compound 4

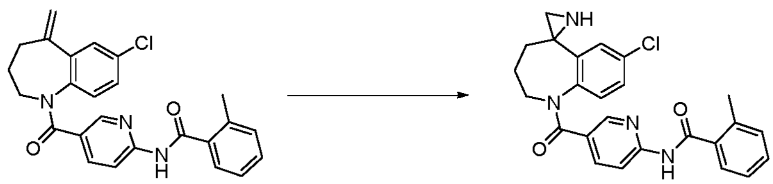

I-21

Compound 4

Intermediate I-21 (100 mg, 0.232 mmol) was dissolved in a solution of aqueous ammonia (5.00 mL) at room temperature, and elemental iodine (177 mg, 0.696 mmol) and α-isotridecyl-ω-hydroxy-poly (oxy-1,2-ethanediyl) (0.300 mL) were added to the reaction. The reaction mixture was stirred at room temperature for 16 hours. Water (20 mL) was added and extraction was made with ethyl acetate (20 mL×3). The organic phases were combined and washed with a saturated aqueous sodium thiosulfate solution (30 mL) and saturated brine (50 mL), dried over anhydrous sodium sulfate and filtered, and the filtrate was concentrated under reduced pressure to give the crude product. The crude product was purified by preparative HPLC (ammonia system) to give compound 4.

LC-MS (ESI) $[M+H]^+$ 447.1.

$^1$H NMR (400 MHz, DMSO-$d_6$) δ 10.86 - 10.76 (m, 1H), 8.48 - 8.01 (m, 2H), 7.61 - 7.09 (m, 7H), 6.85 (dd, *J* = 43.8, 8.3 Hz, 1H), 4.86 (d, *J* = 12.6 Hz, 1H), 2.84 - 2.71 (m, 1H), 2.35 (s, 3H), 2.13 - 1.77 (m, 5H), 1.58 - 1.48 (m, 1H).

Example 5: Preparation of compound 5

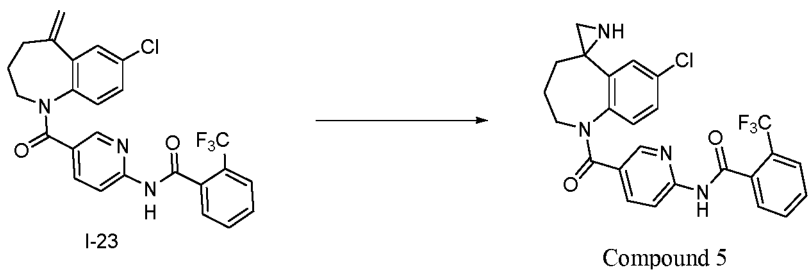

I-23

Compound 5

Intermediate I-23 (100 mg, 0.206 mmol) was added to aqueous ammonia (5 mL) at room temperature, followed by iodine (533 mg, 2.1 mmol) and α-isotridecyl-ω-hydroxy-poly (oxy-1,2-ethanediyl) (0.3 mL). After the addition was complete, the reaction mixture was stirred at room temperature overnight. The reaction mixture was diluted with a saturated aqueous sodium sulfite solution (10 mL), and extracted with ethyl acetate (10 mL× 3), and the organic phases were combined and washed with water (10 mL× 3), dried over anhydrous sodium sulfate and filtered, while the filtrate was concentrated under reduced pressure, and the residue was separated by preparative HPLC (aqueous ammonia system) to give compound 5.

LC-MS (ESI) $[M+H]^+$ 501.0.

$^1H$ NMR (400 MHz, DMSO-$d_6$) δ 11.21 - 11.10 (m, 1H), 8.50 - 7.93 (m, 2H), 7.84 - 7.10 (m, 7H), 6.96 - 6.76 (m, 1H), 4.96 - 4.71 (m, 1H), 2.86 - 2.69 (m, 1H), 2.21 - 1.83 (m, 5H), 1.80 - 1.44 (m, 2H).

Example 6: Preparation of compound 6

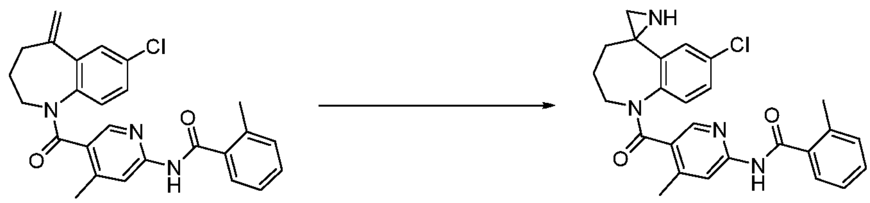

I-30

Compound 6

Intermediate I-30 (150 mg, 0.336 mmol) was dissolved in ammonia (5.00 mL) at room temperature, and elemental iodine (426 mg, 1.68 mmol) and α-isotridecyl-ω-hydroxy-poly (oxy-1,2-ethanediyl) (0.300 mL) were added to the reaction, and the reaction mixture was stirred at room temperature for 16 hours. Water (10 mL) was added and extraction was made with ethyl acetate (10 mL×3). The organic phases were combined and washed with a saturated aqueous sodium thiosulfate solution (20 mL) and saturated brine (20 mL), dried over anhydrous sodium sulfate and filtered, and the filtrate was concentrated under reduced pressure to give the crude product. The crude product was purified by preparative HPLC (ammonia system) to give compound 6.

LC-MS (ESI) $[M+H]^+$ 461.1.

$^1H$ NMR (400 MHz, DMSO-$d_6$) δ 10.97 - 10.44 (m, 1H), 8.59 - 7.03 (m, 8H), 6.75 (dd, $J$ = 41.4, 8.3 Hz, 1H), 4.97 - 4.75 (m, 1H), 2.90 - 2.63 (m, 1H), 2.48 - 2.31 (m, 6H), 2.22 - 1.74 (m, 5H), 1.54 (dd, $J$ = 53.9, 30.7 Hz, 2H).

Example 7: Preparation of compound 7

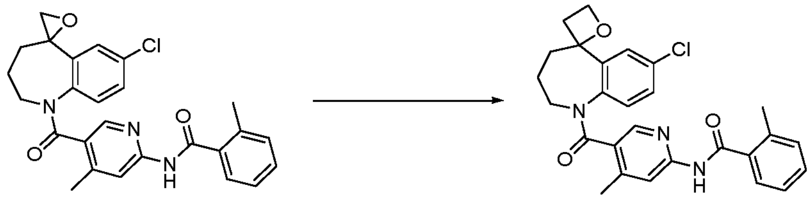

I-31

Compound 7

Potassium tert-butoxide (85.3 mg, 0.760 mmol) and trimethylsulfoxonium iodide (335 mg, 1.52 mmol) were added to dimethyl sulfoxide (1.5 mL) at room temperature and stirred at room temperature for 10 minutes. Intermediate I-31 (70 mg, 0.152 mmol) was then added and the reaction solution was allowed to react at 50°C for 16 hours. The reaction solution was cooled to room temperature, diluted with water (10 mL), and extracted with ethyl acetate (10 mL×3). The organic phases were combined. The organic phases were washed with saturated brine (15 mL), dried over anhydrous sodium sulfate, and filtered, and the filtrate was concentrated under reduced pressure to give the crude product. The crude product was purified by preparative HPLC (ammonium bicarbonate system) to give compound 7.

LC-MS (ESI) $[M+H]^+$ 476.1.

$^1H$ NMR (400 MHz, DMSO-$d_6$) δ 8.43 - 7.64 (m, 1H), 7.53 - 6.91 (m, 7H), 6.88 - 6.51 (m, 1H), 4.94 - 4.43 (m, 1H), 3.39 (d, *J* = 9.5 Hz, 1H), 3.26 (d, *J* = 12.6 Hz, 2H), 3.18 - 2.77 (m, 2H), 2.29 - 1.90 (m, 8H), 1.84 - 1.46 (m, 2H).

Example 8: Preparation of compound 8

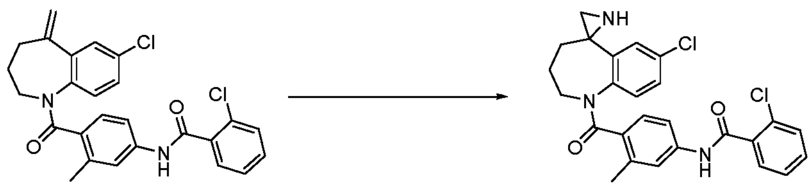

I-34

Compound 8

Intermediate I-34 (150 mg, 0.322 mmol) was dissolved in ammonia (6 mL) and elemental iodine (817 mg, 3.22 mmol) and α-isotridecyl-ω-hydroxy-poly (oxy-1,2-ethanediyl) (0.2 mL) were added to the reaction at room temperature. The reaction mixture was stirred at room temperature for 16 hours. The reaction solution was quenched with saturated sodium sulfite (10 mL), and extracted with ethyl acetate (20 mL×3), and the organic phases were combined and washed with saturated brine (30 mL), dried over anhydrous sodium sulfate, and filtered, and the filtrate was concentrated under reduced pressure to give the crude product. The crude product was isolated and purified by preparative HPLC to give compound 8.

LC-MS (ESI) $[M+H]^+$ 479.9.

$^1H$ NMR (400 MHz, DMSO-$d_6$) δ 10.66 - 10.31 (m, 1H), 7.74 - 6.99 (m, 9H), 6.78 - 6.56 (m, 1H), 4.84 - 3.63 (m, 1H), 3.14 - 2.65 (m, 1H), 2.39 (s, 3H), 2.19 - 1.72 (m, 5H), 1.69 - 1.45 (m, 2H).

Example 9: Preparation of compound 9

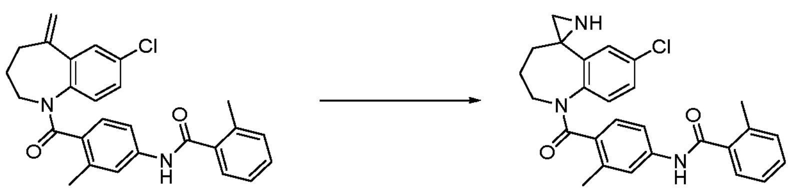

I-36

Compound 9

Compound I-36 (300 mg, 0.674 mmol) was dissolved in ammonia (20.0 mL) at room temperature, and elemental iodine (513 mg, 2.02 mmol) and α-isotridecyl-ω-hydroxy-poly (oxy-1,2-ethanediyl) (30 mg) were added to the reaction system, and the reaction mixture was stirred at room temperature for 8 hours. The reaction system was poured into a saturated sodium bisulfite solution (100 mL), and extracted with ethyl acetate (50 mL×3), and the organic phases were combined and washed with saturated brine (50 mL), dried over anhydrous sodium sulfate, and filtered and the filtrate was concentrated under reduced pressure to give the crude product. The crude product was isolated and purified by preparative HPLC to give compound 9.

LC-MS (ESI) $[M+H]^+$ 460.0.

$^1H$ NMR (400 MHz, DMSO-$d_6$) δ 10.49 - 10.10 (m, 1H), 7.92 - 6.91 (m, 9H), 6.79 - 6.53 (m, 1H), 4.95 - 3.61 (m, 1H), 3.10 - 2.67 (m, 1H), 2.40 - 2.33 (m, 6H), 2.12 - 1.75 (m, 4H), 1.69 - 1.43 (m, 2H).

Example 10: Preparation of compounds 10A, 10B

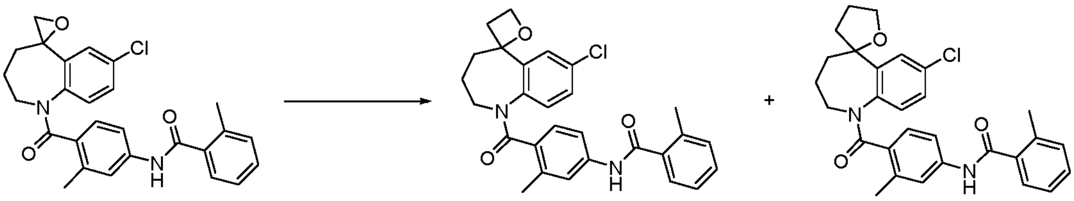

I-37 Compound 10A + Compound 10B

Intermediate I-37 (220 mg, 0.477 mmol) was dissolved in tert-butanol (2.20 mL) at room temperature, and trimethylsulfoxonium iodide (1.05 g, 4.77 mmol) were added followed by potassium tert-butoxide (268 mg, 2.39 mmol), and the reaction mixture was stirred at 55°C for 18 hours. The reaction mixture was cooled to room temperature poured into water (30.0 mL), and extracted with ethyl acetate (10 mL×4), and the organic phases were combined and washed with saturated brine (10 mL), dried over anhydrous sodium sulfate and filtered, while the filtrate was concentrated under reduced pressure to give the crude product. The crude product was isolated and purified by preparative HPLC to afford compound 10A and compound 10B.

Compound 10A:

LC-MS (ESI) $[M+Na]^+$ 497.0.

$^1H$ NMR (400 MHz, DMSO-$d_6$) δ 10.41 - 10.28 (m, 1H), 7.83 - 7.54 (m, 3H), 7.48 - 7.22 (m, 7H), 4.74 - 4.42 (m, 2H), 3.54 (m, 1H), 3.15 - 3.01 (m, 1H), 2.96 - 2.80 (m, 1H), 2.41 - 2.31 (m, 8H), 1.87 - 1.52 (m, 3H).

Compound 10B:

LC-MS (ESI) $[M+H]^+$ 489.1.

$^1H$ NMR (400 MHz, DMSO-$d_6$ + $D_2O$) δ 7.70 - 7.56 (m, 1H), 7.41 (dd, J = 8.3, 2.4 Hz, 1H), 7.33 - 6.87 (m, 8H), 4.62 - 4.33 (m, 2H), 3.41 - 3.30 (m, 2H), 3.28 - 3.20 (m, 1H), 3.09 - 2.96 (m, 1H), 2.88 - 2.72 (m, 1H), 2.30 - 2.06 (m, 8H), 1.82 - 1.36 (m, 3H).

Example 11: Preparation of compound 11

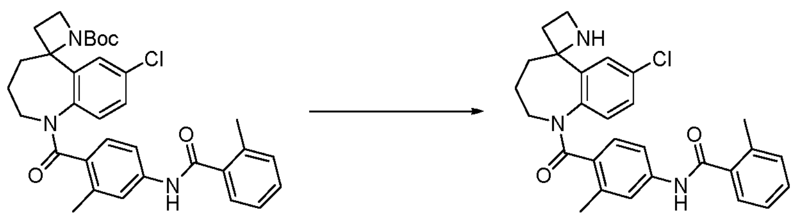

I-47 Compound 11

Intermediate I-47 (40.00 mg, 0.070 mmol) was dissolved in a solution of hydrogen chloride in dioxane (5 mL,

3 M) at 25°C and reacted at room temperature for 1 hour. The product was concentrated to dryness and purified via preparative HPLC (ammonium bicarbonate system) to give compound 11.

LC-MS (ESI) $[M+H]^+$ 474.2.

$^1H$ NMR (400 MHz, Methanol-$d_4$) δ 7.68 - 7.51 (m, 1H), 7.48 - 7.08 (m, 5H), 6.92 (dd, *J* = 8.5, 2.5 Hz, 1H), 6.73 (d, *J* = 8.5 Hz, 1H), 6.60 (d, *J* = 8.4 Hz, 1H), 6.09 (dt, *J* = 64.2, 6.5 Hz, 1H), 4.65 - 4.55 (m, 1H), 3.71 (s, 1H), 3.46 (dt, *J* = 13.0, 5.2 Hz, 1H), 2.89 - 2.78 (m, 2H), 2.78 - 2.64 (m, 1H), 2.62 - 2.51 (m, 1H), 2.51 - 2.40 (m, 1H), 2.30 - 2.28 (m, 6H), 2.24 - 2.06 (m, 1H), 1.97 (m, 1H).

Example 12: Preparation of compound 12

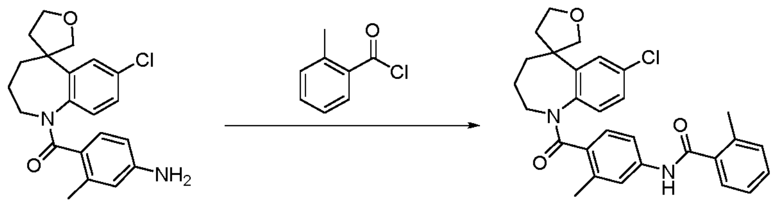

I-57

Compound 12

2-Methylbenzoyl chloride (21.9 mg, 0.142 mmol) and triethylamine (28.7 mg, 0.284 mmol) were added to a solution of intermediate I-57 (35 mg, 0.0944 mmol) in dichloromethane (5 mL) at room temperature, and the reaction solution was allowed to react at room temperature for 3 hours. The reaction solution was diluted with dichloromethane (10 mL), washed with saturated brine (10 mL), dried over anhydrous sodium sulfate, and filtered, and the filtrate was concentrated under reduced pressure to give the crude product. The crude product was purified by preparative HPLC (ammonium bicarbonate system) to give compound 12.

LC-MS (ESI) $[M+H]^+$ 489.1

$^1H$ NMR (400 MHz, DMSO-$d_6$) δ 10.41 - 10.26 (m, 1H), 7.77 - 6.58 (m, 10H), 4.37 - 4.04 (m, 1H), 3.99 - 3.47 (m, 4H), 3.06 - 2.80 (m, 1H), 2.70 - 2.51 (m, 1H), 2.45 - 2.16 (m, 7H), 2.04 - 1.93 (m, 1H), 1.86 - 1.51 (m, 3H).

Example 13: Preparation of compound 13

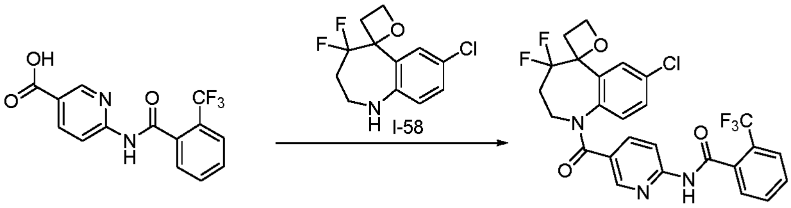


I-7

Compound 13

Intermediate I-7 (54.0 mg, 0.174 mmol) was dissolved in N,N-dimethylacetamide (5.00 mL) at room temperature, cooled to 0°C and thionyl chloride (20.7 mg, 0.174 mmol) was added under argon atmosphere. After the reaction solution was stirred at 0°C for 3 hours, intermediate I-58 (30.0 mg, 0.116 mmol) was added and the reaction mixture was stirred at room temperature for another 16 hours. Water (20 mL) was added and extraction was made with ethyl acetate (10 mL×3). The organic phases were combined and washed with brine (20 mL), dried over anhydrous sodium sulfate, and filtered, and concentration was performed under reduced pressure to give the crude product. The crude product was purified by preparative HPLC (ammonia system) to give compound 13.

LCMS (ESI) $[M+H]^+$552.0.

$^1H$NMR (400 MHz, DMSO-$d_6$) δ 11.29 - 11.19 (m, 1H), 8.41 - 7.97 (m, 2H), 7.90 - 7.63 (m, 6H), 7.45 - 6.92 (m, 2H), 4.85 - 4.49 (m, 3H), 3.54 - 2.63 (m, 4H), 2.38 - 2.15 (m, 1H).

Example 14: Preparation of compound 14

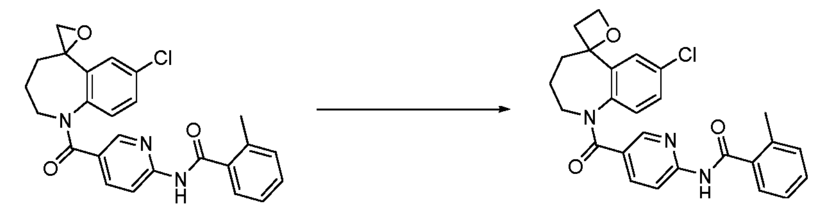

I-59

Compound 14

Potassium tert-butoxide (175 mg, 1.56 mmol) and trimethylsulfoxonium iodide (343 mg, 1.56 mmol) were added to dimethyl sulfoxide (1.00 mL) at room temperature and stirred at room temperature for 5 minutes. Intermediate I-59 (100 mg, 0.223 mmol) was then added and the reaction solution was stirred at 70°C for 20 hours. The reaction solution was cooled to room temperature, diluted with water (10 mL), and extracted with ethyl acetate (5 mL×3). The organic phases were combined. The organic phases ware washed with saturated brine (10 mL), dried over anhydrous sodium sulfate, and filtered, and the filtrate was concentrated under reduced pressure to give the crude product. The crude product was purified by preparative HPLC (ammonia system) to give compound 14.

LC-MS (ESI) $[M+H]^+$ 462.0.

$^1H$ NMR (400 MHz, DMSO-d6) δ 11.09 - 10.74 (m, 1H), 8.56 - 8.00 (m, 2H), 7.87 - 7.17 (m, 7H), 7.06 - 6.78 (m, 1H), 4.80 - 4.33 (m, 3H), 3.11 - 2.92 (m, 1H), 2.89 - 2.54 (m, 2H), 2.47 - 2.15 (m, 4H), 2.12 - 1.55 (m, 3H).

Example 15: Preparation of compound 15

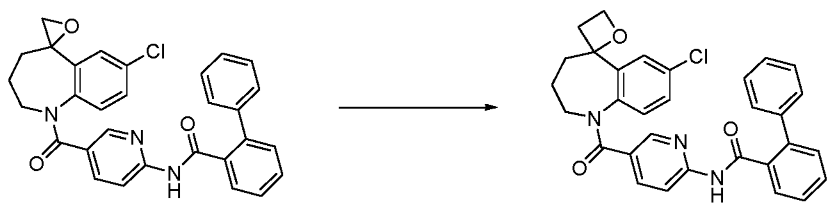

I-65

Compound 15

Intermediate I-65 (200 mg, 0.392 mmol) was dissolved in a solution of dimethylsulfoxide (5.00 mL) at room temperature, trimethylsulfoxonium iodide (863 mg, 3.92 mmol) was added followed by potassium tert-butoxide (220 mg, 1.96 mmol), and the reaction mixture was stirred at 50°C for 24 hours. The reaction mixture was cooled to room temperature poured into water (30 mL), and extracted with ethyl acetate (20 mL×3), and the organic phases were combined and washed with saturated brine (15 mL), dried over anhydrous sodium sulfate and filtered, while the filtrate was concentrated under reduced pressure to give the crude product. The crude product was purified by preparative HPLC (ammonium bicarbonate system) to give compound 15.

LC-MS (ESI) $[M+H]^+$ 524.0.

$^1H$ NMR (400 MHz, DMSO-$d_6$) δ 8.12 - 7.65 (m, 1H), 7.64 - 7.10 (m, 10H), 7.08-6.01 (m, 4H), 4.88 - 4.43 (m, 1H), 3.14 - 2.86 (m, 4H), 2.83 - 2.66 (m, 1H), 2.17 - 1.51 (m, 4H).

Example 16: Preparation of compound 16

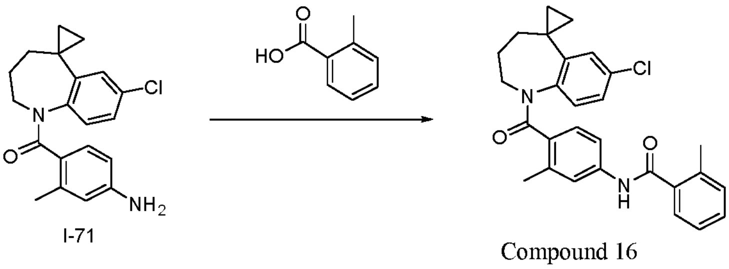

I-71

Compound 16

-(7-azabenzotriazol-1-yl)-N,N,N'-tetramethyluronium hexafluorophosphate (46.8 mg, 0.123 mmol) and N,N-diisopropylethyl amine (23.9 mg, 0.185 mmol) were added to a solution of intermediate I-71 (21 mg, 0.0616 mmol) and o-toluic acid (10.1 mg, 0.0742 mmol) in N,N-dimethylformamide (5 mL) at room temperature, and the reaction solution was stirred at 50°C for 16 hours. Water (10 mL) was added to the reaction solution and extraction was made with ethyl acetate (10 mL×3). The organic phases were combined and washed with brine (20 mL), dried over anhydrous sodium sulfate, and filtered. The filtrate was concentrated to dryness and purified by C18 reverse phase chromatography to give compound 16.

LC-MS (ESI) $[M+H]^+$ 459.2.

$^1H$ NMR (400 MHz, DMSO-$d_6$) δ 10.40 - 10.24 (m, 1H), 7.77 - 7.45 (m, 2H), 7.40 - 7.02 (m, 7H), 6.76 - 6.66 (m, 1H), 4.88 - 2.68 (m, 1H), 2.37 (m, 6H), 2.00 - 1.87 (m, 1H), 1.82 - 1.52 (m, 2H), 1.38 - 1.14 (m, 2H), 1.02 - 0.85 (m, 1H), 0.82 - 0.68 (m, 2H).

Example 17: Preparation of compound 17

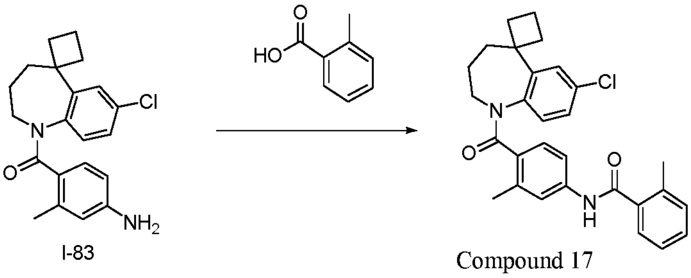

I-83

Compound 17

2-Methylbenzoic acid (19.3 mg, 0.142 mmol), N,N-diisopropylethyl amine (43.7 mg, 0.339 mmol), O-(7-azabenzotriazol-1-yl)-N,N,N'-tetramethyluronium hexafluorophosphate (85.9 mg, 0.226 mmol) were added to a solution of intermediate I-83 (40 mg, 0.113 mmol) in N,N-dimethylformamide (3 mL) at room temperature and the reaction solution was allowed to react at room temperature for 16 hours. The reaction solution was purified by preparative HPLC (formic acid system) to give compound 17.

LC-MS (ESI) $[M+H]^+$ 473.3.

$^1H$ NMR (400 MHz, DMSO-$d_6$) δ 10.40 - 10.26 (m, 1H), 7.75 - 7.17 (m, 9H), 6.86 - 6.55 (m, 1H), 4.52 - 3.50 (m, 1H), 2.98 - 2.83 (m, 1H), 2.77 - 2.64 (m, 1H), 2.41 - 2.23 (m, 7H), 2.22 - 1.35 (m, 8H).

Example 18: Preparation of compound 18

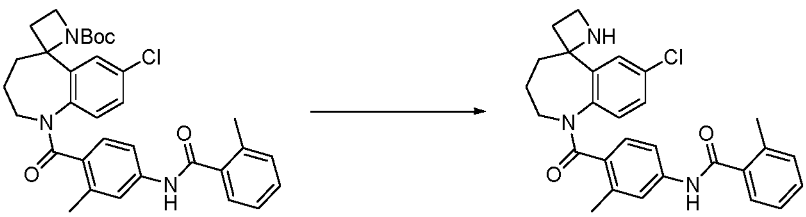

I-91 (chirality A)

Compound 18 (chirality A)

Intermediate I-91 (50.00 mg, 0.087 mmol) was dissolved in a solution of hydrogen chloride in dioxane (5 mL, 3 M) at 25°C and stirred at room temperature for 1 hour. The product was concentrated to dryness and purified via preparative HPLC (ammonium bicarbonate system) to give a chiral compound 18.

LC-MS (ESI) $[M+H]^+$ 474.2.

$^1H$ NMR (400 MHz, DMSO-$d_6$) δ 10.31 (s, 1H), 7.58 - 7.21 (m, 6H), 7.08 (dd, *J* = 8.3, 2.5 Hz, 1H), 6.83 (d, *J* = 8.3 Hz, 1H), 6.69 (d, *J* = 8.4 Hz, 1H), 6.32 - 5.90 (m, 1H), 4.68 - 4.49 (m, 1H), 3.77 - 3.64 (m, 1H), 3.59 (s, 1H), 3.49 - 3.40 (m, 2H), 2.82 - 2.69 (m, 2H), 2.44 (d, *J* = 6.2 Hz, 2H), 2.39 (s, 1H), 2.34 (s, 3H), 2.29 (s, 3H), 2.22 - 2.06 (m, 1H).

Example 19: Preparation of compound 19

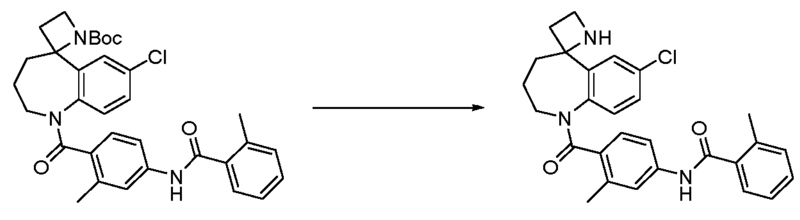

I-96 (chirality B) Compound 19 (chirality B)

Intermediate I-96 (70.00 mg, 0.12 mmol) was dissolved in a solution of hydrogen chloride in dioxane (5 mL, 3 M) at 25°C and stirred at room temperature for 1 hour. The product was concentrated to dryness and purified via preparative HPLC (ammonium bicarbonate system) to give a chiral compound 19.

LC-MS (ESI) $[M+H]^+$ 474.2.

$^1H$ NMR (400 MHz, DMSO-$d_6$) δ 10.34 (s, 1H), 7.57 - 7.23 (m, 6H), 7.11 (dd, *J* = 8.5, 2.4 Hz, 1H), 6.85 (d, *J* = 8.5 Hz, 1H), 6.66 (d, *J* = 8.4 Hz, 1H), 6.27 - 5.98 (m, 1H), 4.63 - 4.48 (m, 1H), 3.85 - 3.57 (m, 1H), 3.51 - 3.34 (m, 1H), 2.99 - 2.78 (m, 3H), 2.64 (q, *J* = 8.6, 7.7 Hz, 1H), 2.48 (d, *J* = 18.7 Hz, 2H), 2.39 (s, 1H), 2.34 - 2.28 (m, 6H), 2.23 - 1.90 (m, 1H).

Example 20: Preparation of compound 20

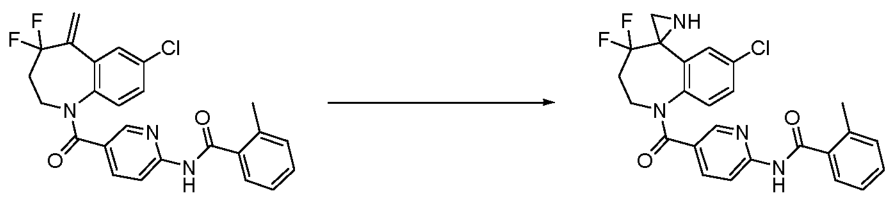

I-97 Compound 20

α-Isotridecyl-ω-hydroxy-poly (oxy-1,2-ethanediyl) (1.00 mL) and elemental iodine (1.19 g, 4.69 mmol) were added to an aqueous ammonia solution (20 mL) of intermediate I-97 (220 mg, 0.470 mmol) at room temperature. The reaction mixture was stirred at room temperature for 16 hours (LCMS and TLC showed about 10% product formation with most of the starting material remaining). A saturated aqueous sodium sulfite solution (40 mL) was added and extracted with ethyl acetate (30 mL× 2). The organic phases were combined and washed with saturated brine (30 mL), dried over anhydrous sodium sulfate, and filtered, and the filtrate was concentrated under reduced pressure to give the crude product, which was purified by silica gel chromatography to recover the starting material and product mixture (210 mg), and then re-charged according to the above procedure, which was repeated twice. A saturated aqueous sodium sulfite solution (30 mL) was added to the reaction solution, which was extracted with ethyl acetate (30 mL× 2). The organic phases were combined and washed with saturated brine (30 mL), dried over anhydrous sodium sulfate, and filtered, and the filtrate was concentrated under reduced pressure to give the crude product. The crude product was purified by silica gel chromatography followed by C18 reverse phase chromatography to afford compound 20.

LC-MS (ESI) $[M+H]^+$ 483.1.

$^1H$ NMR (400 MHz, DMSO-$d_6$) δ 10.90 - 10.80 (m, 1H), 8.48 - 8.07 (m, 1H), 8.08 - 7.20 (m, 8H), 7.09 - 6.82

(m, 1H), 5.03 - 4.76 (m, 1H), 3.19 - 2.92 (m, 1H), 2.84 - 2.54 (m, 2H), 2.42 - 2.13 (m, 5H).

Example 21: Preparation of compound 21

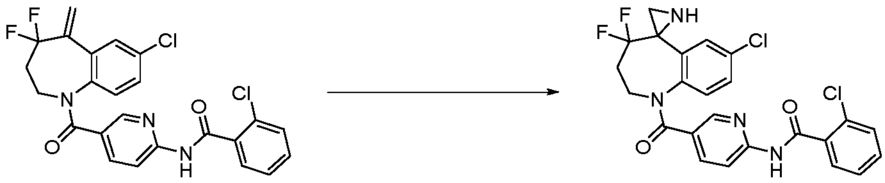

I-100

Compound 21

Alpha-isotridecyl-omega-hydroxy-poly (oxy-1,2-ethylene) (2.00 mL) and elemental iodine (1.56 g, 6.15 mmol) were added to I-100 (300 mg, 0.614 mmol) in aqueous ammonia (20 mL) at room temperature. The reaction mixture was stirred at room temperature for 16 hours (LCMS and TLC showed about 10% product formation with most of the starting material remaining). A saturated aqueous sodium sulfite solution (40 mL) was added and extracted with ethyl acetate (30 mL× 2). The organic phases were combined and washed with saturated brine (30 mL), dried over anhydrous sodium sulfate, and filtered, and the filtrate was concentrated under reduced pressure to give the crude product, which was purified by silica gel chromatography to recover the starting material and product mixture (295 mg), and then re-charged according to the above procedure. A saturated aqueous sodium sulfite solution (30 mL) was added to the reaction solution, which was extracted with ethyl acetate (30 mL× 2). The organic phases were combined and washed with saturated brine (30 mL), dried over anhydrous sodium sulfate, and filtered, and the filtrate was concentrated under reduced pressure to give the crude product. The crude product was purified by silica gel chromatography followed by C18 reverse phase chromatography to afford compound 21.

LC-MS (ESI) $[M+H]^+$ 503.0.

$^1H$ NMR (400 MHz, DMSO-$d_6$) δ 11.16 - 11.06 (m, 1H), 8.56 - 6.84 (m, 10H), 5.08 - 4.57 (m, 1H), 3.18 - 2.94 (m, 1H), 2.89 - 2.53 (m, 2H), 2.46 - 2.16 (m, 2H).

Experimental Example 1: $IC_{50}$ assay for inhibition of vasopressin-induced vasopressin receptor V2R activation by compounds

(1) Cell

Hela cell line stably expressing the human vasopressin receptor V2R (HeLa-V2R): it was constructed by Shanghai Jikai Gene Chemical Technology Co. Ltd. using lentivirus infection method and stably expressed human V2R as verified by qPCR.

(2) Reagents

DMEM cell culture medium: brand: Gibco, code: 11995065; fetal bovine serum: brand: Jitai, article No: FND500; 0.25% Trypsin: brand: Cibco, code: 25200072; Puromycin Dihydrochloride: brand: Gibco, code: A1113803; cAMP-GS HIRANGE KIT: brand: Cisbio, code: 62AM6PEC; IBMX: brand: Sigma, code: i5879; vasopressin AVP: Ji'er Biochemical (Shanghai) Co. Ltd.

(3) Test methods

Hela-V2R cells were cultured in a DMEM medium supplemented with 10% fetal bovine serum at 37°C with 5% $CO_2$ and continuously selected for V2R-expressing cells by adding 2μg/mL puromycin. On the day of the experiment, cells were trypsinized, washed twice with stimulation buffer from the cAMP-GS HIRANGE kit, resuspended, and counted to $1.6\times10^6$ cells/ml, and IBMX was added to a final concentration of 0.5 mM. 5 μL of cell suspension/well was transferred to a 384-well plate and 2.5 μL of test compound or DMSO (minimum Min, maximum Max control) was added at various concentrations (10 μM in a 3-fold dilution, 10 concentration gradients) to the corresponding wells. After 30 minutes of incubation at room temperature, 2.5 μL vasopressin AVP solution was added to a final concentration of 2.25 nM to the test compound wells and 2.5 μL stimulation buffer to the minimum wells and incubated for 60 minutes at 25 C. Simultaneously cAMP standard sample (3-fold dilution from 5.6 μM, 10 concentration points) was prepared, and 10 μL of

cAMP standard sample was transferred to corresponding well of a 384-well plate. The cAMP-d2 fluorescence and anti-cAMP antibody probes provided in the kit were diluted 20-fold with lysis buffer in the cAMP-GS HIRANGE kit, 5 μL of each was taken and added into each well of the 384-well plate in turn, mixed well with simple centrifugation performed, and incubated at 25°C for 2 hours and then detect. Sample detection: fluorescence intensity was measured at 615 nm and 665 nm using the HTRF method in the Envision plate reader. Two duplicate wells were made for each sample to be tested, and 32 duplicate wells were made for Min and Max respectively.

(4) Data processing

The fluorescence intensity ratio $FI_{665/615}$ at the wavelengths of 665 nm and 615 nm was calculated for each well sample. The standard curve was obtained by fitting the "log(inhibitor) vs response - variable slope (four parameters)" model in Prism 8.0 software with the logarithm of standard concentration as X and $FI_{665/615}$X1000 as Y value. With the test well $FI_{665/615}$X1000 as a Y value, the cAMP concentration corresponding to each sample was calculated according to the above standard curve in Prism 8.0 software.

The %Inhibition (inhibition percentage) was calculated as follows:

$$\%\ inhibition = (\overline{Cmax} - Ccmpd)/(\overline{Cmax} - \overline{Cmin}) \times 100$$

where $\overline{Cmax}$ is the average calculated cAMP concentration in all maximum wells; $\overline{Cmm}$ is the average calculated cAMP concentration in all maximum wells; Ccmpd is the calculated cAMP concentration of the test compound.

$IC_{50}$s were calculated by non-linear regression using the "log(inhibitor) vs response - variable slope (four parameters)" model in Prism 8.0 software with % Inhibition (inhibition percentage) as Y value and log value of compound concentration as X, where Y=Bottom + (Top-Bottom)/(1+10^((Log$IC_{50}$-X)*Hill Slope)).

The results are shown in Table 1:

Table 1: Evaluation of compounds on inhibition of the cAMP increase in human cervical cancer cells (Human V2R Hela-Stable cell line OE2)

| Compound No. | $IC_{50}$ (nM) | Compound No. | $IC_{50}$ (nM) |
|---|---|---|---|
| 1 | 10.88 | 2 | 9.35 |
| 4 | 3.14 | 5 | 4.67 |
| 8 | 5.01 | 9 | 4.88 |
| 16 | 11.23 | 17 | 10.43 |
| 20 | 5.54 | 21 | 4.51 |

Experimental Example 2: In vivo pharmacokinetic experiments with compounds of the disclosure

This study evaluated in vivo pharmacokinetics in mice by intravenous and oral administration.

Experimental methods and conditions: male CD1 mice, aged 6-8 weeks, had free access to drinking water and were given a single intravenous injection of test compound 1 mg/Kg (vehicle 5% DMSO/10% Solutol/85% Saline), 5 min, 15 min, 30 min, 1 hr, 2 hr, 4 hr, 8 hr, 24 hr or oral gavage administration of 10 mg/kg (vehicle 5% DMSO/10% Solutol/85% Saline) after administration. Orbital blood was collected at 15 min, 30 min, 1 hr, 2 hr, 4 hr, 6 hr, 8 hr, and 24 hr after administration. No less than 50 μL of each sample was collected. Heparin sodium was used for anticoagulation. After collection, the sample was placed on ice, and the plasma was separated and tested within 1 hour. Plasma concentrations were determined by liquid-phase tandem mass spectrometry (LC/MS/MS). Pharmacokinetic parameters were calculated using Phoenix WinNonlin software. The results are shown in Tables 2 and 3 with Tofapriptan as control 1.

Table 2: Pharmacokinetics of oral administration (10 mg/kg)

| Compounds | $T_{1/2}$ (hr) | $C_{max}$ (ng/mL) | $AUC_{0-inf}$ (ng*hr/mL) | F (%) |
|---|---|---|---|---|
| Compound 1 | 0.60 | 1255 | 4058 | 56 |
| Control 1 | 1.58 | 1307 | 1613 | 44 |

Table 3: Pharmacokinetics of intravenous administration (1 mg/kg)

| Compound | $T_{1/2}$ (hr) | $AUC_{0\text{-}inf}$ (ng*hr/mL) | Cl (ml/min/kg) |
|---|---|---|---|
| Compound 1 | 0.92 | 719 | 23.2 |
| Control 1 | 0.53 | 367 | 45.5 |

The experimental data show that the in vivo pharmacokinetic results of intravenous and oral administration of the compounds of the present disclosure in mice show lower in vivo metabolic clearance Cl and higher in vivo exposure $AUC_{0\text{-}inf}$.

Experimental Example 3: compound proliferation inhibition assay on LLC-PK1 cells

(1) Cell

Porcine kidney epithelial cells LLC-PK1: purchased from ATCC, Cat#CL-101

(2) Reagents:

Medium 199, Gibco (Cat#11150059)
Fetal Bovine Serum (FBS), Australia, Jitai (Cat# FND500)
Trypsin-EDTA (0.25%), phenol red, Gibco(Cat# 25200072)
PBS, pH 7.4, Gibco (Cat# 10010031)
DMSO (Dimethyl Sulfoxide), Sigma (Cat # D8418)
Poly-D-lysine, Gibco(Cat# A3890401)
Vasopressin AVP: Ji'er Biochemical Co. Ltd. Customization
Verapamil hydrochloride, MCE(Cat# HY-A0064)
AlamarBlue™ HS Cell Viability Reagent, Invitrogen (Cat# A50100)

(3) Test method:

The pathogenesis of polycystic kidney disease is associated with low intracellular calcium concentration and cAMP-dependent hyperproliferation of renal collecting duct epithelial cells. Referring to the research paper published in The Journal of Biological Chemistry by Tamio Yamaguchi et al. 2004, we optimized and carried out the renal epithelial cell LLC-PK1 proliferation assay to evaluate the ability of compounds to inhibit vasopressin-induced cell proliferation after decreasing intracellular calcium ion concentration.

LLC-PK1 cells were cultured in M199 medium supplemented with 10% fetal bovine serum at 37°C with 5% $CO_2$. On the first day of the experiment, 100 μL of 0.01% Poly-D-lysine coat 96-well plate into each well, allow to stand at room temperature for 10 min, suctioned and air dried at room temperature for 1 hr, and washed once using 200 μL of 1XPBS for preparation. LLC-PK1 cells were trypsinized, resuspended in serum-free M199 after centrifugation, counted, diluted to a cell suspension of $1\times10^5$/mL in serum-free M199 culture medium, and FBS was added to a final concentration of 1%. 200 μL cell suspension/well was transferred to a 96-well plate. After the cells were cultured for 24 hr, the supernatant of the solution was aspirated, washed once with 200 μL PBS, and then 160 μL of M199 culture solution containing 0.05% FBS and 20 μL of 10X Verapamil (final concentration: 5 μM) were added successively to continue the culture for 24 hrs. On day three, 10 μL of different concentrations of test compound (3 μM from final concentration, 3-fold dilution, 8 concentration gradients) or DMSO (minimum Min, maximum Max control) were added to the corresponding wells. 10 μL vasopressin AVP solution was added to a final concentration of 10 nM to test compound wells and 10 μL serum-free M199 media to minimum wells and continue incubation for 48 hr. On the fifth day, the culture solution was carefully suctioned away, the cells were washed once with 200 μL PBS, 90 μL of M199 serum-free culture solution was carefully added, then 10 μL Alamarblue reagent was added, and the mixture was centrifuged at 300 rpm for 1 min, incubated at 37°C for 2h, and then detected. SpectraMax instrument was used for sample detection, with excitation light at 560 nm and emission light at 595 nm. 3 replicate wells were made for each sample to be tested, and 6 replicate wells for Min and Max respectively.

(4) Data processing

The compound concentration is taken as the X value, and the mean fluorescence intensity of each experimental well sample minus the background well fluorescence intensity is the Y value, representing the number of viable cells in the well at the time of assay. Bars were plotted using Grouped-Summary data-Separated bar graph in GraphPad Prism 8.0 software to reflect the dose-effect relationship of different compounds on AVP-induced cell proliferation. Qualitative evaluation of the inhibitory effect of the compound on proliferation using tolvaptan as a positive control: "+++" means that the overall performance was superior to that of tolvaptan, "++" means that the overall performance was similar to that of tolvaptan, "+" means that the overall performance was weaker than that of tolvaptan, and "-" means that there is no proliferation inhibition.

Data quality control: S/B, i.e., Max well average/Min well average, is calculated and ≥ 2 is considered QC pass.

The experimental results showed that the inhibitory effect of compound 1 on the proliferation of LLC-PK1 cells was stronger than that of control 1 (tofapriptan), and there was no pro-proliferation effect under conditions, as shown in Figure 1.

## Claims

1. A compound of Formula (X), an optical isomer thereof, and a pharmaceutically acceptable salt thereof,

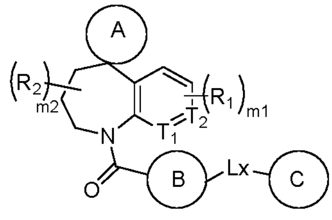

(X)

,

wherein

ring A is selected from heterocycloalkyl and cycloalkyl, and the heterocycloalkyl and cycloalkyl are optionally substituted with 1, 2, 3, or 4 $R_A$ groups;
ring B is selected from aryl, heteroaryl, heterocycloalkyl, and cycloalkyl, and the aryl, heteroaryl, heterocycloalkyl, or cycloalkyl is optionally substituted with 1, 2, or 3 $R_3$ groups;
ring C is selected from aryl, heteroaryl, heterocycloalkyl, and cycloalkyl, and the aryl, heteroaryl, heterocycloalkyl, or cycloalkyl is optionally substituted with 1, 2, or 3 $R_4$ groups;
$T_1$ and $T_2$ are each independently selected from N and CH;
$R_1$, $R_2$, $R_3$, and $R_4$ are each independently selected from H, F, Cl, Br, I, CN, OH, $NH_2$, alkyl, heteroalkyl, aryl, heteroaryl, heterocycloalkyl, and cycloalkyl, and the alkyl, heteroalkyl, aryl, heteroaryl, heterocycloalkyl, or cycloalkyl is optionally substituted with 1, 2, 3, or 4 R groups;
R and $R_A$ are each independently selected from H, F, Cl, Br, I, CN, OH, $NH_2$, alkyl, heteroalkyl, aryl, heteroaryl, heterocycloalkyl, and cycloalkyl, and the alkyl, heteroalkyl, aryl, heteroaryl, heterocycloalkyl, or cycloalkyl is optionally substituted with 1, 2, 3, or 4 R' groups;
R' is selected from H, F, Cl, Br, I, CN, OH, $NH_2$, alkyl, and heteroalkyl;
m1 and m2 are each independently selected from 1, 2, 3, or 4;
Lx is selected from -NH(C=O)-, -alkyl-NH(C=O)-, -NH(C=O)-alkyl-, alkyl, alkenyl, and alkynyl, and the -alkyl-NH(C=O)-, -NH(C=O)-alkyl-, alkyl, alkenyl, or alkynyl is optionally substituted with 1, 2, 3, or 4 R groups; and
when ring A is selected from heterocycloalkyl, the compound of Formula (I) is not selected from

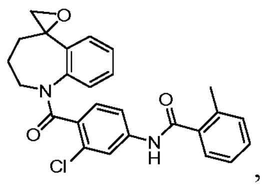

,

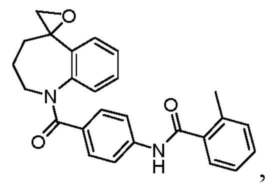

, and

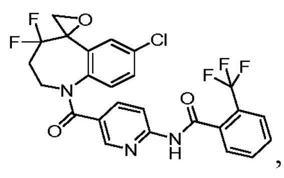

,

the heterocycloalkyl or heteroaryl comprises 1, 2, 3 or 4 heteroatoms or heteroatomic groups independently selected from -O-, -NH-, -N=, -S-, -C(=O)-, -C(=O)O-, -S(=O)-, - $S(=O)_2$-, and N.

**2.** A compound of Formula (I), an optical isomer thereof, and a pharmaceutically acceptable salt thereof,

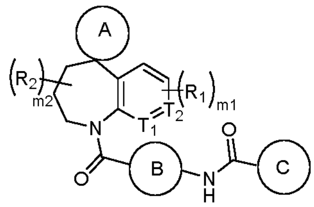

(I) ,

wherein

ring A is selected from 3- to 6-membered heterocycloalkyl and $C_{3-6}$ cycloalkyl, and the 3-to 6-membered heterocycloalkyl and $C_{3-6}$ cycloalkyl are optionally substituted with 1 or 2 $R_A$ groups;
ring B is selected from phenyl and 5- to 6-membered heteroaryl, and the phenyl or 5- to 6-membered heteroaryl is optionally substituted with 1, 2 or 3 $R_3$ groups;
ring C is selected from phenyl and 5- to 6-membered heteroaryl, and the phenyl or 5- to 6-membered heteroaryl is optionally substituted with 1, 2 or 3 $R_4$ groups;
$T_1$ and $T_2$ are each independently selected from N and CH;
$R_1$ is each independently selected from H, F, Cl, Br, I, CN, OH, $NH_2$, and $C_{1-6}$ alkyl, and the $C_{1-6}$ alkyl is optionally substituted with 1, 2, or 3 R groups;
$R_2$ is each independently selected from H, F, Cl, Br, I, CN, OH, $NH_2$, $C_{1-6}$ alkyl, and $C_{3-6}$ cycloalkyl, and the $C_{1-6}$ alkyl or $C_{3-6}$ cycloalkyl is optionally substituted with 1, 2 or 3 R groups;
$R_3$ is each independently selected from H, F, Cl, Br, I, CN, OH, $NH_2$, and $C_{1-6}$ alkyl, and the $C_{1-6}$ alkyl is optionally substituted with 1, 2, or 3 R groups;
$R_4$ is each independently selected from H, F, Cl, Br, I, CN, OH, $NH_2$, $C_{1-6}$ alkyl, $C_{1-6}$ alkoxy, $C_{1-6}$ alkylamino, $C_{3-6}$ cycloalkyl, phenyl, and 5- to 6-membered heteroaryl, and the $C_{1-6}$ alkyl, $C_{1-6}$ alkoxy, $C_{1-6}$ alkylamino, $C_{3-6}$ cycloalkyl, phenyl, or 5- to 6-membered heteroaryl is optionally substituted with 1, 2, or 3 groups;
R and $R_A$ are each independently selected from H, F, Cl, Br, I, CN, OH, $NH_2$, $C_{1-6}$ alkyl, $C_{1-6}$ alkoxy, and $C_{1-6}$ alkylamino, and the $C_{1-6}$ alkyl, $C_{1-6}$ alkoxy, and $C_{1-6}$ alkylamino is optionally substituted with 1, 2 or 3 R' groups;
R' is selected from H, F, Cl, Br, I, CN, OH, $NH_2$, and $C_{1-6}$ alkyl;
m1 and m2 are each independently selected from 1, 2, or 3; and
when ring A is selected from 3- to 6-membered heterocycloalkyl, the compound of Formula (I) is not selected from

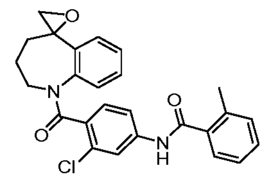

,

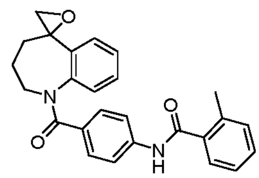

and

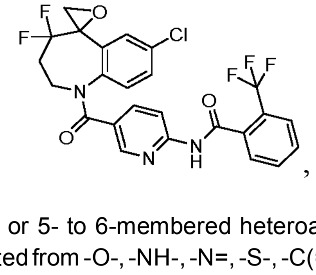

,

the 3- to 6-membered heterocycloalkyl or 5- to 6-membered heteroaryl comprises 1, 2, or 3 heteroatoms or heteroatom groups independently selected from -O-, -NH-, -N=, -S-, -C(=O)-, -C(=O)O-, -S(=O)-, -$S(=O)_2$-, and N.

**3.** A compound of Formula (II), an optical isomer thereof, and a pharmaceutically acceptable salt thereof,

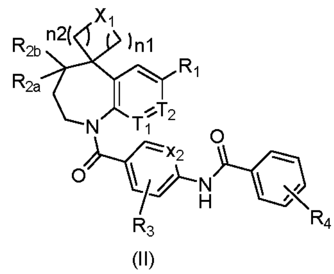

(II) ,

wherein $X_1$ is selected from $C(R_A)_2$, NH, and O;

$X_2$ is selected from CH and N;

$T_1$ and $T_2$ are each independently selected from N and CH;

$R_1$ is each independently selected from H, F, Cl, Br, I, CN, OH, $NH_2$, and $C_{1-6}$ alkyl, and the $C_{1-6}$ alkyl is optionally substituted with 1, 2, or 3 R groups;

$R_{2a}$ and $R_{2b}$ are each independently selected from H, F, Cl, Br, I, CN, OH, $NH_2$, $C_{1-6}$ alkyl, and $C_{3-6}$ cycloalkyl, and the $C_{1-6}$ alkyl or $C_{3-6}$ cycloalkyl is optionally substituted with 1, 2 or 3 R groups;

$R_3$ is selected from H, F, Cl, Br, I, CN, OH, $NH_2$, and $C_{1-6}$ alkyl, and the $C_{1-6}$ alkyl is optionally substituted with 1, 2, or 3 R groups;

$R_4$ is each independently selected from H, F, Cl, Br, I, CN, OH, $NH_2$, $C_{1-6}$ alkyl, $C_{1-6}$ alkoxy, $C_{1-6}$ alkylamino, $C_{3-6}$ cycloalkyl, phenyl, and 5- to 6-membered heteroaryl, and the $C_{1-6}$ alkyl, $C_{1-6}$ alkoxy, $C_{1-6}$ alkylamino, $C_{3-6}$ cycloalkyl, phenyl, or 5- to 6-membered heteroaryl is optionally substituted with 1, 2 or 3 R groups;

R and $R_A$ are each independently selected from H, F, Cl, Br, I, CN, OH, $NH_2$, $C_{1-6}$ alkyl, $C_{1-6}$ alkoxy, and $C_{1-6}$ alkylamino, and the $C_{1-6}$ alkyl, $C_{1-6}$ alkoxy, and $C_{1-6}$ alkylamino are optionally substituted with 1, 2 or 3 R' groups;

R' is selected from H, F, Cl, Br, I, CN, OH, $NH_2$, and $C_{1-6}$ alkyl;

n1 is selected from 0, 1, or 2;

n2 is selected from 1, 2, or 3; and

when $X_1$ is selected from O, the compound of Formula (II) is not selected from

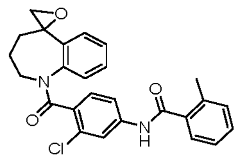
,
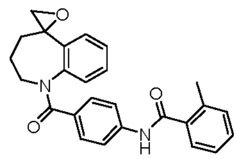
and
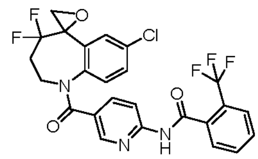
.

4. A compound of Formula (III), an optical isomer thereof, and a pharmaceutically acceptable salt thereof,

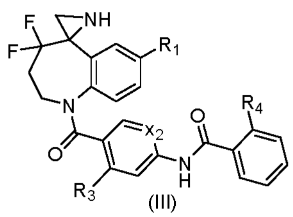

(III) ,

wherein

$R_1$ is each independently selected from H, F, Cl, Br, I, CN, OH, $NH_2$, and $C_{1-6}$ alkyl, and the $C_{1-6}$ alkyl is optionally substituted with 1, 2, or 3 R groups;

$R_3$ is selected from H, F, Cl, Br, I, CN, OH, $NH_2$, and $C_{1-6}$ alkyl, and the $C_{1-6}$ alkyl is optionally substituted with 1, 2, or 3 R groups;

$R_4$ is each independently selected from H, F, Cl, Br, I, CN, OH, $NH_2$, $C_{1-6}$ alkyl, $C_{1-6}$ alkoxy, $C_{1-6}$ alkylamino, $C_{3-6}$ cycloalkyl, phenyl, and 5- to 6-membered heteroaryl, and the $C_{1-6}$ alkyl, $C_{1-6}$ alkoxy, $C_{1-6}$ alkylamino, $C_{3-6}$ cycloalkyl, phenyl, or 5- to 6-membered heteroaryl is optionally substituted with 1, 2 or 3 R groups;

R is selected from H, F, Cl, Br, I, CN, OH, $NH_2$, $C_{1-6}$ alkyl, $C_{1-6}$ alkoxy, and $C_{1-6}$ alkylamino, and the $C_{1-6}$ alkyl, $C_{1-6}$ alkoxy, and $C_{1-6}$ alkylamino are optionally substituted with 1, 2 or 3 R' groups;

R' is selected from H, F, Cl, Br, I, CN, OH, $NH_2$, and $C_{1-6}$ alkyl;
$X_2$ is selected from CH and N.

**5.** The compound, the optical isomer thereof, and the pharmaceutically acceptable salt thereof according to any one of claims 1 to 4, wherein R and $R_A$ are each independently selected from H, F, Cl, Br, I, CN, OH, $NH_2$, $CH_3$, $CF_3$,

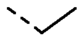, 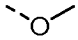, 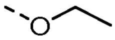, 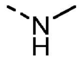, and 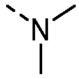.

**6.** The compound, the optical isomer thereof, and the pharmaceutically acceptable salt thereof according to any one of claims 1 to 3, wherein $R_A$ is selected from H, OH, and $NH_2$.

**7.** The compound, the optical isomer thereof, and the pharmaceutically acceptable salt thereof according to any one of claims 1 to 4, wherein $R_4$ is selected from H, F, Cl, Br, I, CN, OH, $NH_2$, $CH_3$, $CF_3$,

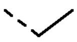, 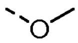, 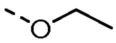, 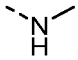, 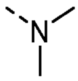,

cyclopropyl, cyclobutyl, cyclopentyl, phenyl, pyridyl, pyrimidinyl, thienyl, and thiazolyl.

**8.** The compound, the optical isomer thereof, and the pharmaceutically acceptable salt thereof according to claim 1 or 2, wherein ring A is selected from cyclopropyl, cyclobutyl, cyclopentyl, aziridinyl, oxiranyl, azetidinyl, oxetanyl, pyrrolidinyl, and tetrahydrofuranyl, and the cyclopropyl, cyclobutyl, cyclopentyl, aziridinyl, oxiranyl, azetidinyl, oxetanyl, pyrrolidinyl, or tetrahydrofuranyl is optionally substituted with 1 or 2 $R_A$ groups.

**9.** The compound, the optical isomer thereof, and the pharmaceutically acceptable salt thereof according to claim 8, wherein ring A is selected from

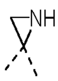, 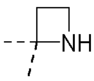, 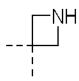, 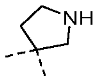,

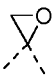, 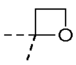, 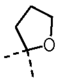, 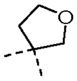, 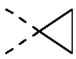, 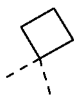, 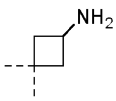, and 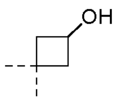.

**10.** The compound, the optical isomer thereof, and the pharmaceutically acceptable salt thereof according to claim 3, wherein the structural moiety

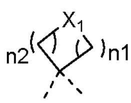

is selected from

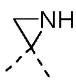,

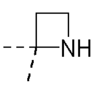, 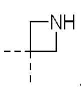, 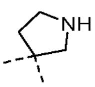, 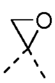, 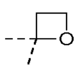, 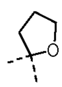, 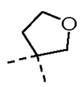, 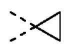, 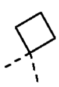, 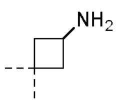, and 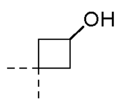.

**11.** The compound, the optical isomer thereof, and the pharmaceutically acceptable salt thereof according to claim 1 or 2, wherein ring B is selected from phenyl and pyridyl, and the phenyl or pyridyl is optionally substituted with 1, 2 or 3 $R_3$ groups.

**12.** The compound, the optical isomer thereof, and the pharmaceutically acceptable salt thereof according to claim 1 or 2, wherein ring C is selected from

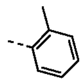,
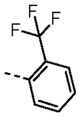,
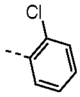,

and

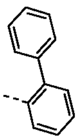.

**13.** A compound of the following formula, an optical isomer thereof, and a pharmaceutically acceptable salt thereof, selected from

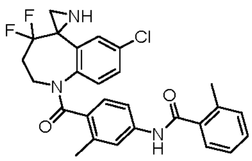
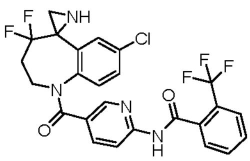
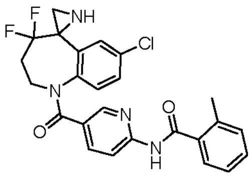
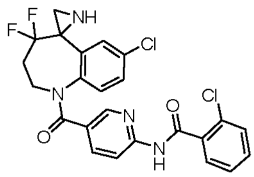
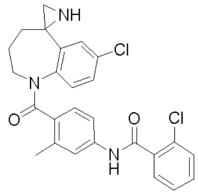
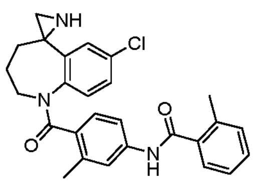
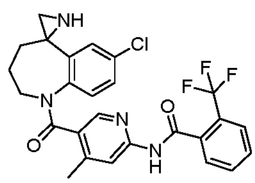
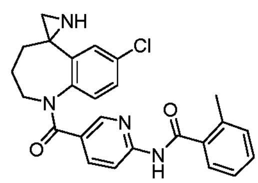
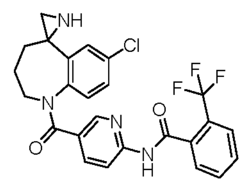
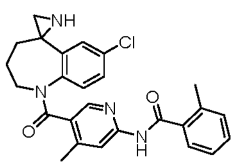
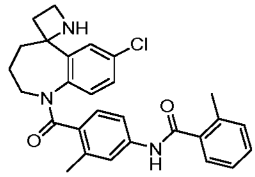
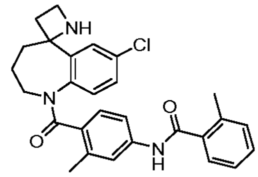
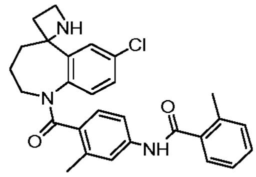
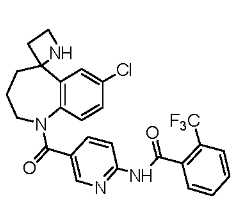
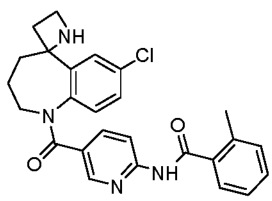
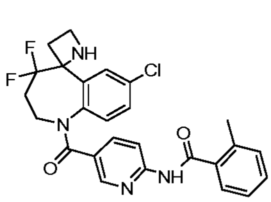
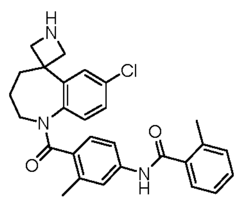

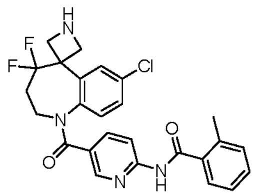
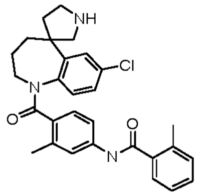
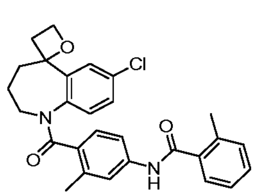
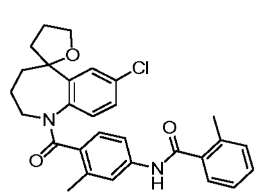
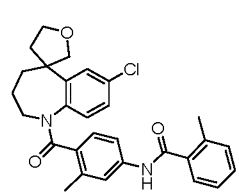

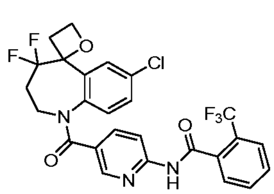
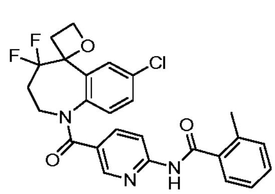
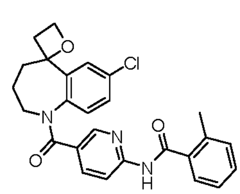
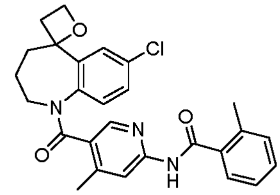

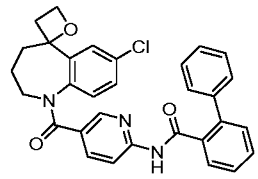
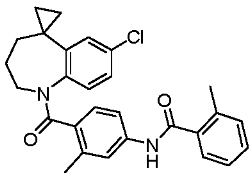
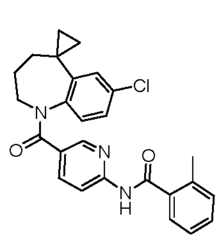
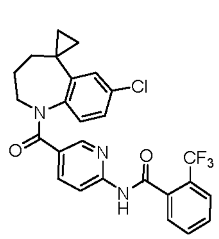
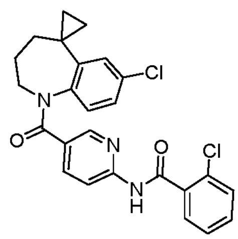

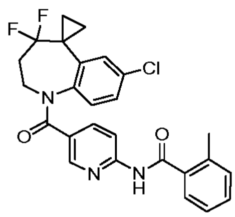
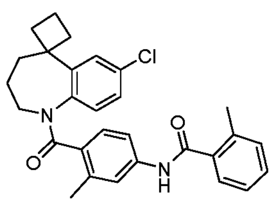
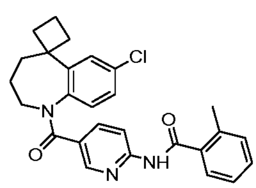
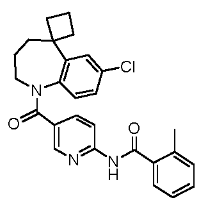
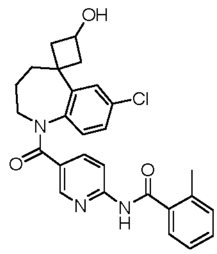

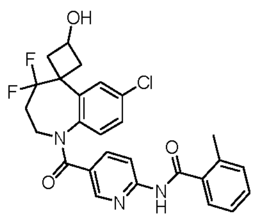
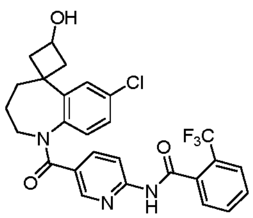
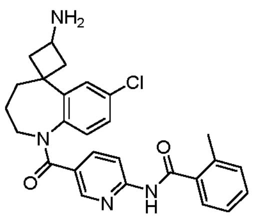
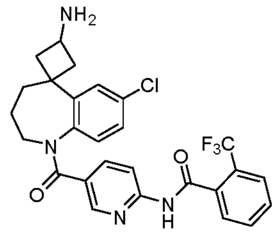

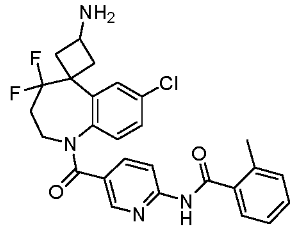.

**14.** A use of the compound, the optical isomer thereof, and the pharmaceutically acceptable salt thereof according to any one of claims 1 to 13 in the manufacture of a medicament for the prevention or treatment of a disease associated with arginine vasopressin V1a receptor, arginine vasopressin V1b receptor, arginine vasopressin V2 receptor, sympathetic nervous system, or renin angiotensin aldosterone system.

**15.** The use according to claim 14, the disease associated with arginine vasopressin V1a receptor, arginine vasopressin V1b receptor, arginine vasopressin V2 receptor, sympathetic nervous system, or renin angiotensin aldosterone system comprises: hypertension, Raynaud's syndrome, dysmenorrhea, premature labor, corticotropin releasing hormone secretion disorder, adrenal hyperplasia, depression, chronic congestive heart failure, cirrhosis, syndrome of inappropriate antidiuretic hormone secretion, hyponatremia due to chronic heart failure/cirrhosis/inappropriate antidiuretic hormone secretion, or polycystic kidney disease.

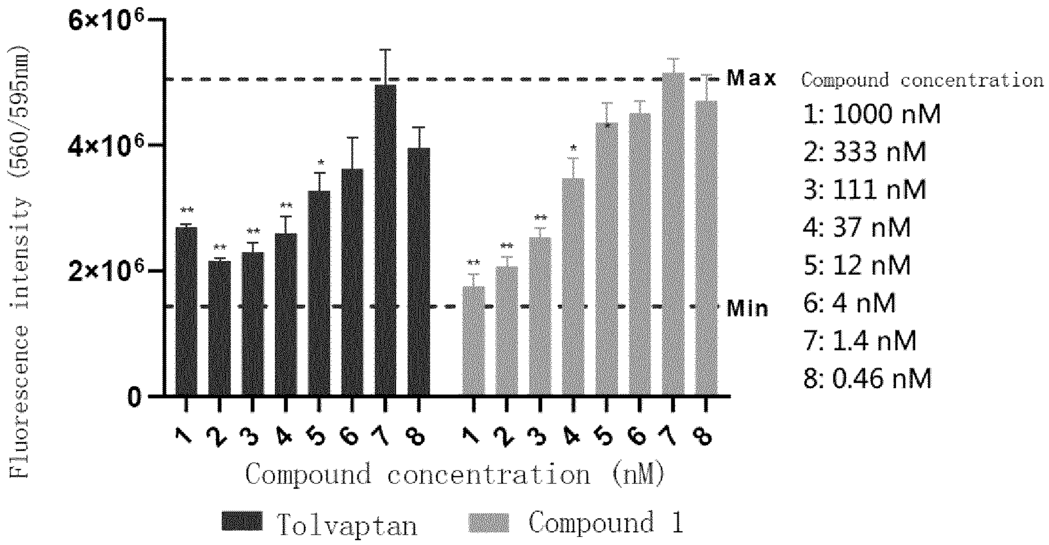
6×10^6
4×10^6
2×10^6
0
Fluorescence intensity (560/595nm)
Max
Min
Compound concentration
1: 1000 nM
2: 333 nM
3: 111 nM
4: 37 nM
5: 12 nM
6: 4 nM
7: 1.4 nM
8: 0.46 nM
1 2 3 4 5 6 7 8 1 2 3 4 5 6 7 8
Compound concentration (nM)
Tolvaptan
Compound 1

FIG. 1

**INTERNATIONAL SEARCH REPORT**

International application No.
**PCT/CN2021/133158**

**A. CLASSIFICATION OF SUBJECT MATTER**

C07D 223/16(2006.01)i; C07D 405/02(2006.01)i; C07D 487/10(2006.01)i; A61K 31/55(2006.01)i; A61P 9/12(2006.01)i; A61P 9/04(2006.01)i; A61P 5/38(2006.01)i; A61P 25/24(2006.01)i; A61P 1/16(2006.01)i; A61P 15/06(2006.01)i; A61P 13/12(2006.01)i

According to International Patent Classification (IPC) or to both national classification and IPC

**B. FIELDS SEARCHED**

Minimum documentation searched (classification system followed by classification symbols)

C07D223/-; C07D405/-; C07D487/-; A61K31/-; A61P9/-; A61P5/-; A61P25/-; A61P1/-; A61P15/-; A61P13/-

Documentation searched other than minimum documentation to the extent that such documents are included in the fields searched

Electronic data base consulted during the international search (name of data base and, where practicable, search terms used)

WPI, EPODOC, CNPAT, CNKI, STN(REGISTRY); 苯并氮杂#, 苯并[b]氮杂#, 螺, 精氨酸加压素, 加压素受体, 升压素受体, 血管紧张受体, 血管加压素, 抗利尿激素, 拮抗剂, 肾素-血管紧张素-醛固酮系统, vasopressin antagonist, arginine vasopressin, AVP, tetrahydrobenzazepine, benzazepine, benzazepinylcarbonylpyridine, benzanilide, spiro, spirobenzazepine, structural formula search

**C. DOCUMENTS CONSIDERED TO BE RELEVANT**

| Category* | Citation of document, with indication, where appropriate, of the relevant passages | Relevant to claim No. |
|---|---|---|
| Y | CN 1449386 A (ORTHO-MCNEIL PHARMACEUTICAL, INC.) 15 October 2003 (2003-10-15)<br>description page 2 line 14- page 80 line 1 | 1-15 |
| Y | CN 101541806 A (JANSSEN PHARMACEUTICA NV.) 23 September 2009 (2009-09-23)<br>description page 2 paragraph 3- page 62 paragraph 4 | 1-15 |
| Y | CN 102796077 A (TIANJIN INSTITUTE OF PHARMACEUTICAL RESEARCH) 28 November 2012 (2012-11-28)<br>description paragraphs [0010]-[0196] | 1-15 |
| A | CN 1127508 A (YAMANOUCHI PHARMACEUTICAL CO., LTD.) 24 July 1996 (1996-07-24)<br>description, page 2 line 19 - page 5, line 20, references 1-27, embodiments 1-113 | 1-15 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 February 2022** | **22 February 2022** |
| Name and mailing address of the ISA/CN<br>**China National Intellectual Property Administration (ISA/CN)**<br>**No. 6, Xitucheng Road, Jimenqiao, Haidian District, Beijing 100088, China** | Authorized officer |
| Facsimile No. **(86-10)62019451** | Telephone No. |

Form PCT/ISA/210 (second sheet) (January 2015)

**INTERNATIONAL SEARCH REPORT**

| International application No. |
|---|
| **PCT/CN2021/133158** |

**C. DOCUMENTS CONSIDERED TO BE RELEVANT**

| Category* | Citation of document, with indication, where appropriate, of the relevant passages | Relevant to claim No. |
|---|---|---|
| A | XIANG, M. A. et al. "Synthesis and evaluation of nonpeptide substituted spirobenzazepines as potent vasopressin antagonists" *Bioorg. Med. Chem. Lett.,* Vol. 14, No. 12, 14 June 2004 (2004-06-14), pp. 3143-3146 | 1-15 |

Form PCT/ISA/210 (second sheet) (January 2015)

**INTERNATIONAL SEARCH REPORT**
**Information on patent family members**

International application No.
**PCT/CN2021/133158**

| Patent document cited in search report | Publication date (day/month/year) | Patent family member(s) | Publication date (day/month/year) |
|---|---|---|---|
| CN 1449386 A | 15 October 2003 | NZ 523450 A | 26 November 2004 |
| | | US 2006111567 A1 | 25 May 2006 |
| | | US 7238687 B2 | 03 July 2007 |
| | | ZA 200300972 B | 04 May 2004 |
| | | NO 20030028 D0 | 03 January 2003 |
| | | NO 20030028 L | 03 March 2003 |
| | | NO 324499 B1 | 29 October 2007 |
| | | IL 153710 A | 30 December 2010 |
| | | EP 1307430 A1 | 07 May 2003 |
| | | EP 1307430 B1 | 28 September 2005 |
| | | ES 2250432 T3 | 16 April 2006 |
| | | WO 0202531 A1 | 10 January 2002 |
| | | HU 0301590 A2 | 29 September 2003 |
| | | HU 0301590 A3 | 28 January 2005 |
| | | TW I316057 B | 21 October 2009 |
| | | BR 0112372 A | 22 July 2003 |
| | | DE 60113696 D1 | 09 February 2006 |
| | | DE 60113696 T2 | 13 July 2006 |
| | | CN 1449386 B | 04 July 2012 |
| | | US 2007117790 A1 | 24 May 2007 |
| | | US 7365062 B2 | 29 April 2008 |
| | | IL 153710 D0 | 06 July 2003 |
| | | MY 135101 A | 29 February 2008 |
| | | AT 305454 T | 15 October 2005 |
| | | AU 2001271780 B2 | 14 September 2006 |
| | | MX PA03000135 A | 17 February 2005 |
| | | CA 2413945 A1 | 10 January 2002 |
| | | CA 2413945 C | 17 May 2011 |
| | | AU 7178001 A | 14 January 2002 |
| | | US 2007135409 A1 | 14 June 2007 |
| | | US 7691844 B2 | 06 April 2010 |
| | | DK 1307430 T3 | 30 January 2006 |
| | | US 2003045517 A1 | 06 March 2003 |
| | | US 7001898 B2 | 21 February 2006 |
| | | JP 2004502677 A | 29 January 2004 |
| | | JP 4916639 B2 | 18 April 2012 |
| CN 101541806 A | 23 September 2009 | WO 2008036755 A1 | 27 March 2008 |
| | | EP 2078022 A1 | 15 July 2009 |
| | | EP 2078022 B1 | 09 November 2011 |
| | | SI 2078022 T1 | 31 January 2012 |
| | | CA 2665849 A1 | 27 March 2008 |
| | | AU 2007299818 A1 | 27 March 2008 |
| | | AU 2007299818 B2 | 02 August 2012 |
| | | CY 1112270 T1 | 09 December 2015 |
| | | DK 2078022 T3 | 13 February 2012 |
| | | PL 2078022 T3 | 30 April 2012 |
| | | JP 2010504351 A | 12 February 2010 |
| | | AT 532786 T | 15 November 2011 |
| | | ES 2374582 T3 | 20 February 2012 |
| | | PT 2078022 E | 11 January 2012 |

Form PCT/ISA/210 (patent family annex) (January 2015)

**INTERNATIONAL SEARCH REPORT**
**Information on patent family members**

International application No.
**PCT/CN2021/133158**

| Patent document cited in search report | Publication date (day/month/year) | Patent family member(s) | Publication date (day/month/year) |
|---|---|---|---|
| | | US 2008076753 A1 | 27 March 2008 |
| | | US 7825110 B2 | 02 November 2010 |
| CN 102796077 A | 28 November 2012 | CN 102796077 B | 18 November 2015 |
| CN 1127508 A | 24 July 1996 | HU 9600102 D0 | 28 March 1996 |
| | | HU T74582 A | 28 January 1997 |
| | | DE 69432632 D1 | 12 June 2003 |
| | | DE 69432632 T2 | 19 February 2004 |
| | | WO 9503305 A1 | 02 February 1995 |
| | | NO 960231 D0 | 19 January 1996 |
| | | NO 960231 L | 21 March 1996 |
| | | NO 306303 B1 | 18 October 1999 |
| | | RU 2129123 C1 | 20 April 1999 |
| | | PL 312654 A1 | 29 April 1996 |
| | | PL 177738 B1 | 31 January 2000 |
| | | KR 960703917 A | 31 August 1996 |
| | | KR 100304331 B1 | 22 November 2001 |
| | | US 5723606 A | 03 March 1998 |
| | | US 5856564 A | 05 January 1999 |
| | | ES 2198418 T3 | 01 February 2004 |
| | | NZ 268671 A | 22 September 1997 |
| | | CN 1040210 C | 14 October 1998 |
| | | DE 69434032 D1 | 28 October 2004 |
| | | DE 69434032 T2 | 22 September 2005 |
| | | EP 0709386 A1 | 01 May 1996 |
| | | EP 0709386 A4 | 25 June 1997 |
| | | EP 0709386 B1 | 07 May 2003 |
| | | AT 277003 T | 15 October 2004 |
| | | AU 1998071957 A1 | 24 December 1998 |
| | | AU 2000071957 A1 | 07 June 2001 |
| | | EP 1097920 A1 | 09 May 2001 |
| | | EP 1097920 B1 | 22 September 2004 |
| | | ES 2228405 T3 | 16 April 2005 |
| | | UA 64690 C2 | 15 March 2004 |
| | | AU 3990697 A | 18 December 1997 |
| | | AU 7195794 A | 20 February 1995 |
| | | AU 683483 B2 | 13 November 1997 |
| | | FI 960260 A0 | 19 January 1996 |
| | | FI 960260 A | 19 January 1996 |
| | | FI 113178 B | 15 March 2004 |
| | | AT 239726 T | 15 May 2003 |
| | | TW 279163 B | 21 June 1996 |
| | | CA 2167673 A1 | 02 February 1995 |
| | | CA 2167673 C | 21 September 2004 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202011353057
- CN 202111322711